(19) Europäisches Patentamt / European Patent Office / Office européen des brevets

(11) EP 3 462 349 A1

(12) **EUROPEAN PATENT APPLICATION**

(43) Date of publication:
**03.04.2019 Bulletin 2019/14**

(21) Application number: **17194288.1**

(22) Date of filing: **02.10.2017**

(51) Int Cl.:
***G06F 19/12*** *(2011.01)* ***C12Q 1/6844*** *(2018.01)*
***C12Q 1/6886*** *(2018.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
- **VAN DE STOLPE, Anja**
  **5656 AE Eindhoven (NL)**
- **HOLTZER, Laurent**
  **5656 AE Eindhoven (NL)**
- **VERHAEGH, Wilhelmus Franciscus Johannes**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

# (54) ASSESSMENT OF NOTCH CELLULAR SIGNALING PATHWAY ACTIVITY USING MATHEMATICAL MODELLING OF TARGET GENE EXPRESSION

(57) The present invention relates to a computer-implemented method for inferring activity of a Notch cellular signaling pathway in a subject based on expression levels of three or more target genes of the Notch cellular signaling pathway measured in a sample of the subject. The present invention further relates to an apparatus, to a non-transitory storage medium, and to a computer program for inferring activity of a Notch cellular signaling pathway in a subject. The present invention further relates to a kit for measuring expression levels of three or more target genes of the Notch cellular signaling pathway in a sample of a subject, to a kit for inferring activity of a Notch cellular signaling pathway in a subject, and to the use of such kits in performing the method.

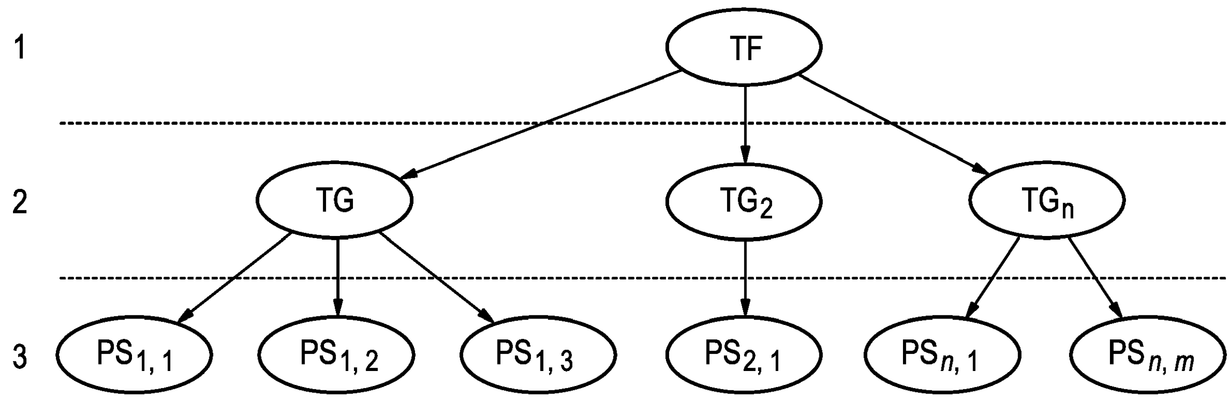


FIG. 2

EP 3 462 349 A1

SEQUENCE LISTING

<110> Koninklijke Philips N. V.

<120> Assessment of Notch cellular signaling pathway activity using mathematical modelling of target gene expression

<130> 2016PF01362

<160> 26

<170> PatentIn version 3.5

<210> 1
<211> 4900
<212> DNA
<213> Homo sapiens

<400> 1
taaagtcatc aaaacaacgt tatatcctgt gtgaaatgct gcagtcagga tgccttgtgg 60

tttgagtgcc ttgatcatgt gccctaaggg gatggtggcg gtggtggtgg ccgtggatga 120

cggagactct caggccttgg caggtgcgtc tttcagttcc cctcacactt cgggttcctc 180

ggggaggagg ggctggaacc ctagcccatc gtcaggacaa agatgctcag gctgctcttg 240

gctctcaact tattcccttc aattcaagta acaggaaaca agattttggt gaagcagtcg 300

cccatgcttg tagcgtacga caatgcggtc aaccttagct gcaagtattc ctacaatctc 360

ttctcaaggg agttccgggc atcccttcac aaaggactgg atagtgctgt ggaagtctgt 420

gttgtatatg ggaattactc ccagcagctt caggtttact caaaaacggg gttcaactgt 480

gatgggaaat tgggcaatga atcagtgaca ttctacctcc agaatttgta tgttaaccaa 540

acagatattt acttctgcaa aattgaagtt atgtatcctc ctccttacct agacaatgag 600

aagagcaatg gaaccattat ccatgtgaaa gggaaacacc tttgtccaag tcccctattt 660

cccggacctt ctaagccctt ttgggtgctg gtggtggttg gtggagtcct ggcttgctat 720

agcttgctag taacagtggc ctttattatt ttctgggtga ggagtaagag gagcaggctc 780

ctgcacagtg actacatgaa catgactccc cgccgccccg ggcccacccg caagcattac 840

cagccctatg ccccaccacg cgacttcgca gcctatcgct cctgacacgg acgcctatcc 900

agaagccagc cggctggcag cccccatctg ctcaatatca ctgctctgga taggaaatga 960

ccgccatctc cagccggcca cctcaggccc ctgttgggcc accaatgcca atttttctcg 1020

agtgactaga ccaaatatca agatcatttt gagactctga aatgaagtaa aagagatttc 1080

ctgtgacagg ccaagtctta cagtgccatg gcccacattc caacttacca tgtacttagt 1140

gacttgactg agaagttagg gtagaaaaca aaaagggagt ggattctggg agcctcttcc 1200

ctttctcact cacctgcaca tctcagtcaa gcaaagtgtg gtatccacag acattttagt 1260

tgcagaagaa aggctaggaa atcattcctt ttggttaaat gggtgtttaa tcttttggtt 1320

agtgggttaa acggggtaag ttagagtagg gggagggata ggaagacata tttaaaaacc 1380
attaaaacac tgtctcccac tcatgaaatg agccacgtag ttcctattta atgctgtttt 1440
cctttagttt agaaatacat agacattgtc ttttatgaat tctgatcata tttagtcatt 1500
ttgaccaaat gagggatttg gtcaaatgag ggattccctc aaagcaatat caggtaaacc 1560
aagttgcttt cctcactccc tgtcatgaga cttcagtgtt aatgttcaca atatactttc 1620
gaaagaataa aatagttctc ctacatgaag aaagaatatg tcaggaaata aggtcacttt 1680
atgtcaaaat tatttgagta ctatgggacc tggcgcagtg gctcatgctt gtaatcccag 1740
cactttggga ggccgaggtg ggcagatcac ttgagatcag gaccagcctg gtcaagatgg 1800
tgaaactccg tctgtactaa aaatacaaaa tttagcttgg cctggtggca ggcacctgta 1860
atcccagctg cccaagaggc tgaggcatga gaatcgcttg aacctggcag gcggaggttg 1920
cagtgagccg agatagtgcc acagctctcc agcctgggcg acagagtgag actccatctc 1980
aaacaacaac aacaacaaca acaacaacaa caaaccacaa aattatttga gtactgtgaa 2040
ggattatttg tctaacagtt cattccaatc agaccaggta ggagctttcc tgtttcatat 2100
gtttcagggt tgcacagttg gtctctttaa tgtcggtgtg gagatccaaa gtgggttgtg 2160
gaaagagcgt ccataggaga agtgagaata ctgtgaaaaa gggatgttag cattcattag 2220
agtatgagga tgagtcccaa gaaggttctt tggaaggagg acgaatagaa tggagtaatg 2280
aaattcttgc catgtgctga ggagatagcc agcattaggt gacaatcttc cagaagtggt 2340
caggcagaag gtgccctggt gagagctcct ttacagggac tttatgtggt ttagggctca 2400
gagctccaaa actctgggct cagctgctcc tgtaccttgg aggtccattc acatgggaaa 2460
gtattttgga atgtgtcttt tgaagagagc atcagagttc ttaagggact gggtaaggcc 2520
tgaccctgaa atgaccatgg atatttttct acctacagtt tgagtcaact agaatatgcc 2580
tggggacctt gaagaatggc ccttcagtgg ccctcaccat ttgttcatgc ttcagttaat 2640
tcaggtgttg aaggagctta ggttttagag gcacgtagac ttggttcaag tctcgttagt 2700
agttgaatag cctcaggcaa gtcactgccc acctaagatg atggttcttc aactataaaa 2760
tggagataat ggttacaaat gtctcttcct atagtataat ctccataagg gcatggccca 2820
agtctgtctt tgactctgcc tatccctgac atttagtagc atgcccgaca tacaatgtta 2880
gctattggta ttattgccat atagataaat tatgtataaa aattaaactg ggcaatagcc 2940
taagaagggg ggaatattgt aacacaaatt taaacccact acgcagggat gaggtgctat 3000
aatatgagga ccttttaact tccatcattt tcctgtttct tgaaatagtt tatcttgtaa 3060
tgaaatataa ggcacctccc acttttatgt atagaaagag gtcttttaat tttttttaa 3120
tgtgagaagg aagggaggag taggaatctt gagattccag atcgaaaata ctgtactttg 3180
gttgattttt aagtgggctt ccattccatg gatttaatca gtcccaagaa gatcaaactc 3240

agcagtactt gggtgctgaa gaactgttgg atttaccctg gcacgtgtgc cacttgccag 3300
cttcttgggc acacagagtt cttcaatcca agttatcaga ttgtatttga aaatgacaga 3360
gctggagagt tttttgaaat ggcagtggca aataaataaa tacttttttt taaatggaaa 3420
gacttgatct atggtaataa atgattttgt tttctgactg gaaaaatagg cctactaaag 3480
atgaatcaca cttgagatgt ttcttactca ctctgcacag aaacaaagaa gaaatgttat 3540
acagggaagt ccgttttcac tattagtatg aaccaagaaa tggttcaaaa acagtggtag 3600
gagcaatgct ttcatagttt cagatatggt agttatgaag aaaacaatgt catttgctgc 3660
tattattgta agagtcttat aattaatggt actcctataa tttttgattg tgagctcacc 3720
tatttgggtt aagcatgcca atttaaagag accaagtgta tgtacattat gttctacata 3780
ttcagtgata aaattactaa actactatat gtctgcttta aatttgtact ttaatattgt 3840
cttttggtat taagaaagat atgctttcag aatagatatg cttcgctttg gcaaggaatt 3900
tggatagaac ttgctattta aaagaggtgt ggggtaaatc cttgtataaa tctccagttt 3960
agcctttttt gaaaaagcta gactttcaaa tactaatttc acttcaagca gggtacgttt 4020
ctggtttgtt tgcttgactt cagtcacaat ttcttatcag accaatggct gacctctttg 4080
agatgtcagg ctaggcttac ctatgtgttc tgtgtcatgt gaatgctgag aagtttgaca 4140
gagatccaac ttcagccttg accccatcag tccctcgggt taactaactg agccaccggt 4200
cctcatggct attttaatga gggtattgat ggttaaatgc atgtctgatc ccttatccca 4260
gccatttgca ctgccagctg ggaactatac cagacctgga tactgatccc aaagtgttaa 4320
attcaactac atgctggaga ttagagatgg tgccaataaa ggacccagaa ccaggatctt 4380
gattgctata gacttattaa taatccaggt caaagagagt gacacacact ctctcaagac 4440
ctggggtgag ggagtctgtg ttatctgcaa ggccatttga ggctcagaaa gtctctcttt 4500
cctatagata tatgcatact ttctgacata taggaatgta tcaggaatac tcaaccatca 4560
caggcatgtt cctacctcag ggcctttaca tgtcctgttt actctgtcta gaatgtcctt 4620
ctgtagatga cctggcttgc ctcgtcaccc ttcaggtcct tgctcaagtg tcatcttctc 4680
ccctagttaa actaccccac accctgtctg ctttccttgc ttatttttct ccatagcatt 4740
ttaccatctc ttacattaga catttttctt atttatttgt agtttataag cttcatgagg 4800
caagtaactt tgctttgttt cttgctgtat ctccagtgcc cagagcagtg cctggtatat 4860
aataaatatt tattgactga gtgaaaaaaa aaaaaaaaaa 4900

<210> 2
<211> 5748
<212> DNA
<213> Homo sapiens

<400> 2

```
gagaagaaag ccagtgcgtc tctgggcgca ggggccagtg gggctcggag gcacaggcac      60
cccgcgacac tccaggttcc ccgacccacg tccctggcag ccccgattat ttacagcctc     120
agcagagcac ggggcggggg cagaggggcc cgcccgggag ggctgctact tcttaaaacc     180
tctgcgggct gcttagtcac agcccccctt gcttgggtgt gtccttcgct cgctccctcc     240
ctccgtctta ggtcactgtt ttcaacctcg aataaaaact gcagccaact tccgaggcag     300
cctcattgcc cagcggaccc cagcctctgc caggttcggt ccgccatcct cgtcccgtcc     360
tccgccggcc cctgccccgc gcccagggat cctccagctc ctttcgcccg cgccctccgt     420
tcgctccgga caccatggac aagttttggt ggcacgcagc ctggggactc tgcctcgtgc     480
cgctgagcct ggcgcagatc gatttgaata taacctgccg ctttgcaggt gtattccacg     540
tggagaaaaa tggtcgctac agcatctctc ggacggaggc cgctgacctc tgcaaggctt     600
tcaatagcac cttgcccaca atggcccaga tggagaaagc tctgagcatc ggatttgaga     660
cctgcaggta tgggttcata gaagggcacg tggtgattcc ccggatccac cccaactcca     720
tctgtgcagc aaacaacaca ggggtgtaca tcctcacatc caacacctcc cagtatgaca     780
catattgctt caatgcttca gctccacctg aagaagattg tacatcagtc acagacctgc     840
ccaatgcctt tgatggacca attaccataa ctattgttaa ccgtgatggc acccgctatg     900
tccagaaagg agaatacaga acgaatcctg aagacatcta ccccagcaac cctactgatg     960
atgacgtgag cagcggctcc tccagtgaaa ggagcagcac ttcaggaggt tacatctttt    1020
acaccttttc tactgtacac cccatcccag acgaagacag tccctggatc accgacagca    1080
cagacagaat ccctgctacc actttgatga gcactagtgc tacagcaact gagacagcaa    1140
ccaagaggca agaaacctgg gattggtttt catggttgtt tctaccatca gagtcaaaga    1200
atcatcttca cacaacaaca caaatggctg gtacgtcttc aaataccatc tcagcaggct    1260
gggagccaaa tgaagaaaat gaagatgaaa gagacagaca cctcagtttt tctggatcag    1320
gcattgatga tgatgaagat tttatctcca gcaccatttc aaccacacca cgggcttttg    1380
accacacaaa acagaaccag gactggaccc agtggaaccc aagccattca aatccggaag    1440
tgctacttca gacaaccaca aggatgactg atgtagacag aaatggcacc actgcttatg    1500
aaggaaactg gaacccagaa gcacaccctc ccctcattca ccatgagcat catgaggaag    1560
aagagacccc acattctaca agcacaatcc aggcaactcc tagtagtaca acggaagaaa    1620
cagctaccca gaaggaacag tggtttggca acagatggca tgagggatat cgccaaacac    1680
ccaaagaaga ctcccattcg acaacaggga cagctgcagc ctcagctcat accagccatc    1740
caatgcaagg aaggacaaca ccaagcccag aggacagttc ctggactgat ttcttcaacc    1800
caatctcaca ccccatggga cgaggtcatc aagcaggaag aaggatggat atggactcca    1860
```

gtcatagtat aacgcttcag cctactgcaa atccaaacac aggtttggtg gaagatttgg 1920
acaggacagg acctctttca atgacaacgc agcagagtaa ttctcagagc ttctctacat 1980
cacatgaagg cttggaagaa gataaagacc atccaacaac ttctactctg acatcaagca 2040
ataggaatga tgtcacaggt ggaagaagag acccaaatca ttctgaaggc tcaactactt 2100
tactggaagg ttatacctct cattacccac acacgaagga aagcaggacc ttcatcccag 2160
tgacctcagc taagactggg tcctttggag ttactgcagt tactgttgga gattccaact 2220
ctaatgtcaa tcgttcctta tcaggagacc aagacacatt ccaccccagt gggggtccc 2280
ataccactca tggatctgaa tcagatggac actcacatgg gagtcaagaa ggtggagcaa 2340
acacaacctc tggtcctata aggacacccc aaattccaga atggctgatc atcttggcat 2400
ccctcttggc cttggctttg attcttgcag tttgcattgc agtcaacagt cgaagaaggt 2460
gtgggcagaa gaaaaagcta gtgatcaaca gtggcaatgg agctgtggag gacagaaagc 2520
caagtggact caacggagag gccagcaagt ctcaggaaat ggtgcatttg gtgaacaagg 2580
agtcgtcaga aactccagac cagtttatga cagctgatga gacaaggaac ctgcagaatg 2640
tggacatgaa gattggggtg taacacctac accattatct tggaaagaaa caaccgttgg 2700
aaacataacc attacaggga gctgggacac ttaacagatg caatgtgcta ctgattgttt 2760
cattgcgaat ctttttagc ataaaatttt ctactctttt tgttttttgt gttttgttct 2820
ttaaagtcag gtccaatttg taaaaacagc attgctttct gaaattaggg cccaattaat 2880
aatcagcaag aatttgatcg ttccagttcc cacttggagg cctttcatcc ctcgggtgtg 2940
ctatggatgg cttctaacaa aaactacaca tatgtattcc tgatcgccaa cctttccccc 3000
accagctaag gacatttccc agggttaata gggcctggtc cctgggagga aatttgaatg 3060
ggtccatttt gcccttccat agcctaatcc ctgggcattg ctttccactg aggttggggg 3120
ttggggtgta ctagttacac atcttcaaca gaccccctct agaaattttt cagatgcttc 3180
tgggagacac ccaaagggtg aagctattta tctgtagtaa actatttatc tgtgtttttg 3240
aaatattaaa ccctggatca gtcctttgat cagtataatt ttttaaagtt actttgtcag 3300
aggcacaaaa gggtttaaac tgattcataa taaatatctg tacttcttcg atcttcacct 3360
tttgtgctgt gattcttcag tttctaaacc agcactgtct gggtccctac aatgtatcag 3420
gaagagctga gaatggtaag gagactcttc taagtcttca tctcagagac cctgagttcc 3480
cactcagacc cactcagcca aatctcatgg aagaccaagg agggcagcac tgtttttgtt 3540
ttttgttttt tgtttttttt ttttgacact gtccaaaggt tttccatcct gtcctggaat 3600
cagagttgga agctgaggag cttcagcctc ttttatggtt taatggccac ctgttctctc 3660
ctgtgaaagg ctttgcaaag tcacattaag tttgcatgac ctgttatccc tggggcccta 3720
tttcatagag gctggcccta ttagtgattt ccaaaaacaa tatggaagtg ccttttgatg 3780

tcttacaata agagaagaag ccaatggaaa tgaaagagat tggcaaaggg gaaggatgat 3840

gccatgtaga tcctgtttga catttttatg gctgtatttg taaacttaaa cacaccagtg 3900

tctgttcttg atgcagttgc tatttaggat gagttaagtg cctggggagt ccctcaaaag 3960

gttaaaggga ttcccatcat tggaatctta tcaccagata ggcaagttta tgaccaaaca 4020

agagagtact ggctttatcc tctaacctca tattttctcc cacttggcaa gtcctttgtg 4080

gcatttattc atcagtcagg gtgtccgatt ggtcctagaa cttccaaagg ctgcttgtca 4140

tagaagccat tgcatctata aagcaacggc tcctgttaaa tggtatctcc tttctgaggc 4200

tcctactaaa agtcatttgt tacctaaact tatgtgctta acaggcaatg cttctcagac 4260

cacaaagcag aaagaagaag aaaagctcct gactaaatca gggctgggct tagacagagt 4320

tgatctgtag aatatcttta aaggagagat gtcaactttc tgcactattc ccagcctctg 4380

ctcctccctg tctaccctct cccctccctc tctccctcca cttcacccca caatcttgaa 4440

aaacttcctt tctcttctgt gaacatcatt ggccagatcc attttcagtg gtctggattt 4500

ctttttattt tcttttcaac ttgaaagaaa ctggacatta ggccactatg tgttgttact 4560

gccactagtg ttcaagtgcc tcttgttttc ccagagattt cctgggtctg ccagaggccc 4620

agacaggctc actcaagctc tttaactgaa aagcaacaag ccactccagg acaaggttca 4680

aaatggttac aacagcctct acctgtcgcc ccagggagaa aggggtagtg atacaagtct 4740

catagccaga gatggttttc cactccttct agatattccc aaaaagaggc tgagacagga 4800

ggttattttc aattttattt tggaattaaa tacttttttc cctttattac tgttgtagtc 4860

cctcacttgg atatacctct gttttcacga tagaaataag ggaggtctag agcttctatt 4920

ccttggccat tgtcaacgga gagctggcca agtcttcaca aacccttgca acattgcctg 4980

aagtttatgg aataagatgt attctcactc ccttgatctc aagggcgtaa ctctggaagc 5040

acagcttgac tacacgtcat ttttaccaat gattttcagg tgacctgggc taagtcattt 5100

aaactgggtc tttataaaag taaaaggcca acatttaatt attttgcaaa gcaacctaag 5160

agctaaagat gtaatttttc ttgcaattgt aaatcttttg tgtctcctga agacttccct 5220

taaaattagc tctgagtgaa aaatcaaaag agacaaaaga catcttcgaa tccatatttc 5280

aagcctggta gaattggctt ttctagcaga acctttccaa aagttttata ttgagattca 5340

taacaacacc aagaattgat tttgtagcca acattcattc aatactgtta tatcagagga 5400

gtaggagaga ggaaacattt gacttatctg gaaaagcaaa atgtacttaa gaataagaat 5460

aacatggtcc attcaccttt atgttataga tatgtctttg tgtaaatcat ttgttttgag 5520

ttttcaaaga atagcccatt gttcattctt gtgctgtaca atgaccactg ttattgttac 5580

tttgactttt cagagcacac ccttcctctg gtttttgtat atttattgat ggatcaataa 5640

taatgaggaa agcatgatat gtatattgct gagttgaaag cacttattgg aaaatattaa 5700

aaggctaaca ttaaaagact aaaggaaaca gaaaaaaaaa aaaaaaaa 5748

<210> 3
<211> 2993
<212> DNA
<213> Homo sapiens

<400> 3
agcaaaccaa tcgcaagcct cgttgagtgg aaggggtggg atcttccccg gaagtgttgg 60

ttaaagcccc tccaatcagc ggctcggtgc ggcaagtttg aatttcgtgg aggctcgggt 120

tgtgagggtt cctgcttcgg agtcggcggt ggtcgtccag accgagtgtt ctttactttt 180

tgtttggttg aggtttcacg ctagaaggtg gctcaggatg tcttcatcac attttgccag 240

tcgacacagg aaggatataa gtactgaaat gattagaact aaaattgctc ataggaaatc 300

actgtctcag aaagaaaata gacataagga atacgaacga aatagacact ttggtttgaa 360

agatgtaaac attccaacct tggaaggtag aattcttgtt gaattagatg agacatctca 420

agggcttgtt ccagaaaaga ccaatgttaa gccaagggca atgaaaacta ttctaggtga 480

tcaacgaaaa cagatgctcc aaaaatacaa agaagaaaag caacttcaaa aattgaaaga 540

gcagagagag aaagctaaac gaggaatatt taaagtgggt cgttatagac ctgatatgcc 600

ttgttttctt ttatcaaacc agaatgctgt gaaagctgag ccaaaaaagg ctattccatc 660

ttctgtacgg attacaaggt caaaggccaa agaccaaatg gagcagacta agattgataa 720

cgagagtgat gttcgagcaa tccgacctgg tccaagacaa acttctgaaa agaaagtgtc 780

agacaaagag aaaaaagttg tgcagcctgt aatgcccacg tcgttgagaa tgactcgatc 840

agctactcaa gcagcaaagc aggttcccag aacagtctca tctaccacag caagaaagcc 900

agtcacaaga gctgctaatg aaaacgaacc agaaggaaag gtgccaagta aaggaagacc 960

tgccaaaaat gtagaaacaa aacccgacaa gggtatttct tgtaaagtcg atagtgaaga 1020

aaatactttg aattcacaaa ctaatgcaac aagtggaatg aatccagatg gagtcttatc 1080

aaaaatggaa aacttacctg agataaatac tgcaaaaata aaagggaaga attcctttgc 1140

acctaaggat tttatgtttc agccactgga tggtctgaag acctatcaag taacacctat 1200

gactcccaga agtgccaatg ctttttttgac acccagttac acctggactc ctttaaaaac 1260

agaagttgat gagtctcaag caacaaaaga aattttggca caaaaatgta aaacttactc 1320

taccaagaca atacagcaag attcaaataa attgccatgt cctttgggtc ctctaactgt 1380

ttggcatgaa gaacatgttt taaataaaaa tgaagctact actaaaaatt taaatggcct 1440

tccaataaaa gaagtcccat cacttgaaag aaatgaaggt cgaattgctc agccccacca 1500

tggtgtgcca tatttcagaa atatcctcca gtcagaaact gagaaattaa cttcacattg 1560

cttcgagtgg gacaggaaac ttgaattgga cattccagat gatgctaaag atcttattcg 1620

cacagcagtt ggtcaaacaa gactccttat gaaggaaagg tttaaacagt ttgaaggact 1680

ggttgatgat tgtgaatata aacgaggtat aaaggagact acctgtacag atctggatgg 1740

attttgggat atggttagtt ttcagataga agatgtaatc cacaaattca acaatctgat 1800

caaacttgag gaatctgggt ggcaagtcaa taataatatg aatcataata tgaacaaaaa 1860

tgtctttagg aaaaaagttg tctcaggtat agcaagtaaa ccaaaacagg atgatgctgg 1920

aagaattgca gcgagaaatc gcctagctgc cataaaaaat gcaatgagag agagaattag 1980

gcaggaagaa tgtgctgaaa cagcagtttc tgtgatacca aaggaagttg ataaaatagt 2040

gttcgatgct ggatttttca gagttgaaag tcctgttaaa ttattctcag gactttctgt 2100

ctcttctgaa ggcccttctc aaagacttgg aacacctaag tctgtcaaca aagctgtatc 2160

tcagagtaga aatgagatgg gcattccaca acaaactaca tcaccagaaa atgccggtcc 2220

tcagaatacg aaaagtgaac atgtgaagaa gactttgttt ttgagtattc ctgaaagcag 2280

gagcagcata gaagatgctc agtgtcctgg attaccagat ttaattgaag aaaatcatgt 2340

tgtaaataag acagacttga aggtggattg tttatccagt gagagaatga gtttgcctct 2400

tcttgctggt ggagtagcag atgatattaa tactaacaaa aaagaaggaa tttcagatgt 2460

tgtggaagga atggaactga attcttcaat tacatcacag gatgttttga tgagtagccc 2520

tgaaaaaaat acagcttcac aaaatagcat cttagaagaa ggggaaacta aaatttctca 2580

gtcagaacta tttgataata aaagtctcac tactgaatgc caccttcttg attcaccagg 2640

tctaaactgc agtaatccat ttactcagct ggagaggaga catcaagaac atgccagaca 2700

catttctttt ggtggtaacc tgattacttt ttcacctcta caaccaggag aattttgaat 2760

ttaaaaataa atccaaacat tttccttcat attatcaatg cttatatatt ccttagacta 2820

ttgaaatttt ggagaaaatg tatttgtgtt cacttctata gcatataatg ttttaatatt 2880

ctgtgttcat caaagtgtat tttagatata ctctttctca agggaagtgg ggatattttg 2940

tacattttca acacagaata aaaaatgtac tgtgccttgc ctctcttgtt taa 2993

<210> 4
<211> 3317
<212> DNA
<213> Homo sapiens

<400> 4
cgaacagggg cggctgcctc actccctacc tgagccagcc gaggggggcca aggactttag 60

agctgtttcc tccggcataa gagagacact tgctttccag ggcagcaccc tttatcggag 120

aaggctctac agggaagggg tctttgcagc ctggatggcc atcccacatt cctttaacgg 180

aggtctctag gcctcagaga gaacccagag ttagaaagga ggccagacgg tccttgctgt 240

cccccctgggg agagaggaag ttgccgcctg ctgccaggcc caggaggagc tgggcctgca 300

atagtggggg acctggcccc tgaggcagtg gcggccatgt cacggccagg ccacggtggg 360

ctgatgcctg tgaatggtct gggcttccca ccgcagaacg tggcccgggt ggtggtgtgg 420

gagtggctga atgagcacag ccgctggcgg ccctacacgg ccaccgtgtg ccaccacatt 480

gagaacgtgc tgaaggagga cgctcgcggt tccgtggtcc tggggcaggt ggacgcccag 540

cttgtgccct acatcatcga cctgcagtcc atgcaccagt ttcgccagga cacaggcacc 600

atgcggcccg tgcggcgcaa cttctacgac ccgtcgtcgg cgccgggcaa gggcatcgtg 660

tgggagtggg agaacgacgg cggcgcatgg acggcctacg atatggacat ctgcatcacc 720

atccagaacg cctacgagaa gcagcacccg tggctcgacc tctcatcgct aggcttctgc 780

tacctcatct acttcaacag catgtcgcag atgaaccgcc agacgcgccg gcgccgccgc 840

ctgcgccgcc gcctggacct cgcctacccg ctcaccgtgg gctccatccc taagtcgcag 900

tcgtggcccg tgggcgccag ctcgggccag ccctgctcct gccagcagtg cctgctggtc 960

aacagcacgc gcgccgcctc caacgccatc ctggcctcgc agcgccgcaa ggcgccccc 1020

gcgcccccgc tgccgccgcc gccgccacct ggagggcctc caggcgcgct tgccgtgcgc 1080

cccagcgcca ccttcacagg cgccgcgctc tgggcagcgc ccgccgccgg ccccgccgag 1140

cccgcgccgc ctcccggggc gcccccacgg agcccgggcg cccccggcgg agcgcgcacc 1200

ccggggcaga acaacctcaa ccggcccggg ccccagcgca ccaccagcgt gagcgcgcgc 1260

gcctccatcc cgcggggggt ccccgcactc ccggtgaaga acttgaatgg tactgggccg 1320

gtccatccgg ccctggcagg gatgaccggg atactgctgt gcgcggccgg gctgcccgtg 1380

tgcctgacgc gggcccccaa gcccatcctg cacccgccgc ccgtgagcaa gagcgacgtg 1440

aagcccgtgc ctggcgtgcc cggggtgtgc cgcaagacca agaagaagca ccttaaaaag 1500

agtaagaatc ccgaggatgt ggttcgaaga tacatgcaga aggtgaaaaa cccacctgat 1560

gaggactgca ccatctgcat ggagcgactg gtcacagcat caggctacga gggcgtgctt 1620

cggcacaagg gcgtgcggcc tgagctcgtg ggccgcctgg gccgctgtgg ccacatgtac 1680

cacctgctgt gcctcgtggc catgtactcc aatggcaaca aggatggcag cctgcagtgc 1740

cccacctgca aggccatcta cggggagaag acgggtacgc agccgcctgg gaagatggag 1800

ttccacctca tcccccactc gctgcccggc ttccctgata cccagaccat ccgcatcgtc 1860

tatgacatcc ccacaggcat ccagggccct gagcacccca accccgggaa gaagttcacc 1920

gcaagaggat tccctcgcca ctgctatcta cccaacaacg agaaaggccg gaaggtgctg 1980

cggctgctca tcacggcctg ggagagaaga ctcatcttca ctatcggcac gtccaacacc 2040

acgggcgagt cggacaccgt ggtgtggaac gagatccacc acaagaccga gtttggatcc 2100

aacctcacgg gccacggcta cccggacgct agctacctag acaacgtgct ggctgagctc 2160

acagcccagg gcgtatccga ggctgcagcc aaggcttgag gcccaaggct gcccaccttc 2220

cctcctgctt tgcccctggt ccggcaaatg cctccttcgc caggtgtgtc ctggtagccc 2280

aggttcaggg ctggggagga gcctgcggaa ggggccgcag ccattcaggg gacctgcctg 2340

gtggcagctg ggatgaagag agatggcatg tcaggctggc cccgaatcat agctccctga 2400

gagggccaag cagagagtac tggaaacctc cctaccaaaa agacagagac ccgccccctc 2460

acacacaaac acacatgtcc tgttgaactc atgcacgcac acccacgtgc ctgtacttgc 2520

ccccaggctg gaagagaaga gacagaaaga ccccatgacc cccccatgtg gatccccatc 2580

tgtgtctcag ttgcatctgt acagccttgt ctgcaaactg gaggatgcgg ggcaagccct 2640

taggggcctg ccagggctcg gggggcaaag agggactcgg gaaactcagt gtaccccaga 2700

tgcctcaccc attccgtgtc atcacccatg tctgccaccc actgattggg caattgtggg 2760

cccatggggt ggaagccccc agatgactga gcagttctac aaaagaatgg ccagcacgag 2820

cggggactag agggtcctga ttttgtgtct gtgcctcttc atctctctgg actctgatct 2880

ccttctccct tcccatctcc aggccttctg tctgtcccag ataaaggcgc tgttctccca 2940

tcctccctac cccatcctct ccaccaaatc gctcccaatt ttgagagcca aaggctggcg 3000

cttctgactt caggagcgaa aggaggaggc ctagtttggg ccgatgtatt ttaaagcaga 3060

gtggacagca gagagtcaat ttccctttcg ttgggagtgg gcagtggggt ggctaattgt 3120

cttcggccaa ccaggggcct gttgcccagg caactcacca gctccgcctc tgctgattgg 3180

ctgccacggt gggagtcagc caagatttaa agggatgcca gcgattgctc ttttcaaaac 3240

ctaccagtcc cactgtgggt ggagaaataa atggtctttc tcctcctcaa aaaaaaaaaa 3300

aaaaaaaaaa aaaaaaa 3317

<210> 5
<211> 4234
<212> DNA
<213> Homo sapiens

<400> 5
cgtgagcggc gcagcaagat cccagctcgg accccggacg gcgcgcgccc ccgaagcccc 60

ggatcccagt cgggcccgca gctgaccgcc agattactgt gcatcccgaa tcacgaccac 120

ctgcaccctc ctgccccggc ccgcccccca agtcctcagg cacccagctc cccggcgccc 180

cggatcctcc tggaccggtc cgtccagatt cccgcgggac cgacctgtcc gcatccccag 240

gaccgccggg ctcggtgcac cgcctcggtc ccggagccgc ccgcctggat tgcattccct 300

cctctcctgg atctcctggg acccgacgcg agcctgcccc ggagcccgcc gagcgcaccc 360

tctctcgggt gcctgcagcc ccgccggcgc ggcccggccc ggcgcggccc ggctcggctc 420

ctagagctgc cacggccatg gccagagccc gcccgccgcc gccgccgtcg ccgccgccgg 480

```
ggcttctgcc gctgctccct ccgctgctgc tgctgccgct gctgctgctg cccgccggct      540
gccgggcgct ggaagagacc ctcatggaca caaaatgggt aacatctgag ttggcgtgga      600
catctcatcc agaaagtggg tgggaagagg tgagtggcta cgatgaggcc atgaatccca      660
tccgcacata ccaggtgtgt aatgtgcgcg agtcaagcca gaacaactgg cttcgcacgg      720
ggttcatctg gcggcgggat gtgcagcggg tctacgtgga gctcaagttc actgtgcgtg      780
actgcaacag catccccaac atccccggct cctgcaagga gaccttcaac ctcttctact      840
acgaggctga cagcgatgtg gcctcagcct cctccccctt ctggatggag aacccctacg      900
tgaaagtgga caccattgca cccgatgaga gcttctcgcg gctggatgcc ggccgtgtca      960
acaccaaggt gcgcagcttt gggccacttt ccaaggctgg cttctacctg gccttccagg     1020
accagggcgc ctgcatgtcg ctcatctccg tgcgcgcctt ctacaagaag tgtgcatcca     1080
ccaccgcagg cttcgcactc ttccccgaga ccctcactgg ggcggagccc acctcgctgg     1140
tcattgctcc tggcacctgc atccctaacg ccgtggaggt gtcggtgcca ctcaagctct     1200
actgcaacgg cgatggggag tggatggtgc ctgtgggtgc ctgcacctgt gccaccggcc     1260
atgagccagc tgccaaggag tcccagtgcc gcccctgtcc ccctgggagc tacaaggcga     1320
agcagggaga ggggccctgc ctcccatgtc cccccaacag ccgtaccacc tccccagccg     1380
ccagcatctg cacctgccac aataacttct accgtgcaga ctcggactct gcggacagtg     1440
cctgtaccac cgtgccatct ccaccccgag gtgtgatctc caatgtgaat gaaacctcac     1500
tgatcctcga gtggagtgag ccccgggacc tgggtggccg ggatgacctc ctgtacaatg     1560
tcatctgcaa gaagtgccat ggggctggag ggcctcagc ctgctcacgc tgtgatgaca     1620
acgtggagtt tgtgcctcgg cagctgggcc tgacggagcg ccgggtccac atcagccatc     1680
tgctggccca cacgcgctac acctttgagg tgcaggcggt caacggtgtc tcgggcaaga     1740
gccctctgcc gcctcgttat gcggccgtga atatcaccac aaaccaggct gccccgtctg     1800
aagtgcccac actacgcctg cacagcagct caggcagcag cctcacccta tcctgggcac     1860
ccccagagcg gcccaacgga gtcatcctgg actacgagat gaagtacttt gagaagagcg     1920
agggcatcgc ctccacagtg accagccaga tgaactccgt gcagctggac gggcttcggc     1980
ctgacgcccg ctatgtggtc caggtccgtg cccgcacagt agctggctat gggcagtaca     2040
gccgccctgc cgagtttgag accacaagtg agagaggctc tggggcccag cagctccagg     2100
agcagcttcc cctcatcgtg ggctccgcta cagctgggct tgtcttcgtg gtggctgtcg     2160
tggtcatcgc tatcgtctgc ctcaggaagc agcgacacgg ctctgattcg gagtacacgg     2220
agaagctgca gcagtacatt gctcctggaa tgaaggttta tattgaccct tttacctacg     2280
aggaccctaa tgaggctgtt cgggagtttg ccaaggagat cgacgtgtcc tgcgtcaaga     2340
```

tcgaggaggt gatcggagct ggggaatttg gggaagtgtg ccgtggtcga ctgaaacagc 2400
ctggccgccg agaggtgttt gtggccatca agacgctgaa ggtgggctac accgagaggc 2460
agcggcggga cttcctaagc gaggcctcca tcatgggtca gtttgatcac cccaatataa 2520
tccggctcga gggcgtggtc accaaaagtc ggccagttat gatcctcact gagttcatgg 2580
aaaactgcgc cctggactcc ttcctccggc tcaacgatgg gcagttcacg gtcatccagc 2640
tggtgggcat gttgcggggc attgctgccg gcatgaagta cctgtccgag atgaactatg 2700
tgcaccgcga cctggctgct cgcaacatcc ttgtcaacag caacctggtc tgcaaagtct 2760
cagactttgg cctctcccgc ttcctggagg atgacccctc cgatcctacc tacaccagtt 2820
ccctgggcgg gaagatcccc atccgctgga ctgccccaga ggccatagcc tatcggaagt 2880
tcacttctgc tagtgatgtc tggagctacg gaattgtcat gtgggaggtc atgagctatg 2940
gagagcgacc ctactgggac atgagcaacc aggatgtcat caatgccgtg gagcaggatt 3000
accggctgcc accacccatg gactgtccca cagcactgca ccagctcatg ctggactgct 3060
gggtgcggga ccggaacctc aggcccaaat tctcccagat tgtcaatacc ctggacaagc 3120
tcatccgcaa tgctgccagc ctcaaggtca ttgccagcgc tcagtctggc atgtcacagc 3180
ccctcctgga ccgcacggtc ccagattaca caaccttcac gacagttggt gattggctgg 3240
atgccatcaa gatggggcgg tacaaggaga gcttcgtcag tgcggggttt gcatcttttg 3300
acctggtggc ccagatgacg gcagaagacc tgctccgtat tggggtcacc ctggccggcc 3360
accagaagaa gatcctgagc agtatccagg acatgcggct gcagatgaac cagacgctgc 3420
ctgtgcaggt ctgacaccgg ctcccacggg gaccctgagg accgtgcagg gatgccaagc 3480
agccggctgg actttcggac tcttggactt ttggatgcct ggccttaggc tgtggcccag 3540
aagctggaag tttgggaaag gcccaagctg ggacttctcc aggcctgtgt tccctcccca 3600
ggaagtgcgc cccaaacctc ttcatattga agatggatta ggagaggggg tgatgacccc 3660
tccccaagcc cctcagggcc cagaccttcc tgctctccag caggggatcc ccacaacctc 3720
acacttgtct gttcttcagt gctggaggtc ctggcagggt caggctgggg taagccgggg 3780
ttccacaggg cccagccctg gcaggggtct ggcccccccag gtaggcggag agcagtccct 3840
ccctcaggaa ctggaggagg ggactccagg aatggggaaa tgtgacacca ccatcctgaa 3900
gccagcttgc acctccagtt tgcacaggga tttgttctgg gggctgaggg ccctgtcccc 3960
acccccgccc ttggtgctgt cataaaaggg caggcagggg caggctgagg agttgccctt 4020
tgccccccag agactgactc tcagagccag agatgggatg tgtgagtgtg tgtgtgtgtg 4080
tgtgtgtgtg cgcgcgcgcg cgcgtgtgtg tgtgcacgca ctggcctgca cagagagcat 4140
gggtgagcgt gtaaaagctt ggccctgtgc cctacaatgg ggccagctgg gccgacagca 4200
gaataaaggc aataagatga aaaaaaaaaa aaaa 4234

<210> 6
<211> 1047
<212> DNA
<213> Homo sapiens

<400> 6
tttctcaggc ataagggctg tagtgtgagg attgggagga actcgaccta ctccgctaac 60

ccagtggcct gagccaatca caaagaggat tggagcctca ctcgagcgct ccttcccttc 120

tcctctctct gtgacagcct cttggaaaga gggacactgg aggggtgtgt ttgcaattta 180

aatcactgga tttttgccca ccctctttcc aaataagaag gcaggagctg cttgctgagg 240

tgtaaagggt cttctgagct gcagtggcaa ttagaccaga agatccccgc tcctgtctct 300

aaagagggga aagggcaagg atggtggagg ctttctgtgc tacctggaag ctgaccaaca 360

gtcagaactt tgatgagtac atgaaggctc taggcgtggg ctttgccact aggcaggtgg 420

gaaatgtgac caaaccaacg gtaattatca gtcaagaagg agacaaagtg gtcatcagga 480

ctctcagcac attcaagaac acggagatta gtttccagct gggagaagag tttgatgaaa 540

ccactgcaga tgatagaaac tgtaagtctg ttgttagcct ggatggagac aaacttgttc 600

acatacagaa atgggatggc aaagaaacaa attttgtaag agaaattaag gatggcaaaa 660

tggttatgac ccttactttt ggtgatgtgg ttgctgttcg ccactatgag aaggcataaa 720

aatgttcctg gtcggggctt ggaagagctc ttcagttttt ctgtttcctc aagtctcagt 780

gctatcctat tacaacatgg ctgatcatta attagaaggt tatccttggt gtggaggtgg 840

aaaatggtga tttaaaaact tgttactcca agcaacttgc ccaattttaa tctgaaaatt 900

tatcatgttt tataatttga attaaagttt tgtccccccc cccctttttt ttataaacaa 960

gtgaatacat tttataattt cttttggaat gtaaatcaaa tttgaataaa aatcttacac 1020

gtgaaattta aaaaaaaaaa aaaaaaa 1047

<210> 7
<211> 3097
<212> DNA
<213> Homo sapiens

<400> 7
atcgccagtc tagcccactc cttcataaag ccctcgcatc ccaggagcga gcagagccag 60

agcaggatgg agaggagacg catcacctcc gctgctcgcc gctcctacgt ctcctcaggg 120

gagatgatgg tgggggcct ggctcctggc cgccgtctgg gtcctggcac ccgcctctcc 180

ctggctcgaa tgccccctcc actcccgacc cgggtggatt tctccctggc tggggcactc 240

aatgctggct tcaaggagac ccgggccagt gagcgggcag agatgatgga gctcaatgac 300

cgctttgcca gctacatcga gaaggttcgc ttcctggaac agcaaaacaa ggcgctggct 360

gctgagctga accagctgcg ggccaaggag cccaccaagc tggcagacgt ctaccaggct 420
gagctgcgag agctgcggct gcggctcgat caactcaccg ccaacagcgc ccggctggag 480
gttgagaggg acaatctggc acaggacctg gccactgtga ggcagaagct ccaggatgaa 540
accaacctga ggctggaagc cgagaacaac ctggctgcct atagacagga agcagatgaa 600
gccaccctgg cccgtctgga tctggagagg aagattgagt cgctggagga ggagatccgg 660
ttcttgagga agatccacga ggaggaggtt cgggaactcc aggagcagct ggcccgacag 720
caggtccatg tggagcttga cgtggccaag ccagacctca ccgcagccct gaaagagatc 780
cgcacgcagt atgaggcaat ggcgtccagc aacatgcatg aagccgaaga gtggtaccgc 840
tccaagtttg cagacctgac agacgctgct gcccgcaacg cggagctgct ccgccaggcc 900
aagcacgaag ccaacgacta ccggcgccag ttgcagtcct tgacctgcga cctggagtct 960
ctgcgcggca cgaacgagtc cctggagagg cagatgcgcg agcaggagga gcggcacgtg 1020
cgggaggcgg ccagttatca ggaggcgctg gcgcggctgg aggaagaggg gcagagcctc 1080
aaggacgaga tggcccgcca cttgcaggag taccaggacc tgctcaatgt caagctggcc 1140
ctggacatcg agatcgccac ctacaggaag ctgctagagg gcgaggagaa ccggatcacc 1200
attcccgtgc agaccttctc caacctgcag attcgagaaa ccagcctgga caccaagtct 1260
gtgtcagaag gccacctcaa gaggaacatc gtggtgaaga ccgtggagat gcgggatgga 1320
gaggtcatta aggagtccaa gcaggagcac aaggatgtga tgtgaggcag gacccacctg 1380
gtggcctctg ccccgtctca tgaggggccc gagcagaagc aggatagttg ctccgcctct 1440
gctggcacat ttccccagac ctgagctccc caccacccca gctgctcccc tccctcctct 1500
gtccctaggt cagcttgctg ccctaggctc cgtcagtatc aggcctgcca gacggcaccc 1560
acccagcacc cagcaactcc aactaacaag aaactcaccc ccaaggggca gtctggaggg 1620
gcatggccag cagcttgcgt tagaatgagg aggaaggaga gaaggggagg agggcggggg 1680
gcacctacta catcgccctc cacatccctg attcctgttg ttatggaaac tgttgccaga 1740
gatggaggtt ctctcggagt atctgggaac tgtgcctttg agtttcctca ggctgctgga 1800
ggaaaactga gactcagaca ggaaagggaa ggccccacag acaaggtagc cctggccaga 1860
ggcttgtttt gtcttttggt ttttatgagg tgggatatcc ctatgctgcc taggctgacc 1920
ttgaactcct gggctcaagc agtctaccca cctcagcctc ctgtgtagct gggattatag 1980
attggagcca ccatgcccag ctcagagggt tgttctccta gactgaccct gatcagtcta 2040
agatgggtgg ggacgtcctg ccacctgggg cagtcacctg cccagatccc agaaggacct 2100
cctgagcgat gactcaagtg tctcagtcca cctgagctgc catccaggga tgccatctgt 2160
gggcacgctg tgggcaggtg ggagcttgat tctcagcact tgggggatct gttgtgtacg 2220
tggagaggga tgaggtgctg ggagggatag aggggggctg cctggccccc agctgtgggt 2280

acagagaggt caagcccagg aggactgccc cgtgcagact ggaggggacg ctggtagaga 2340

tggaggagga ggcaattggg atggcgctag gcatacaagt aggggttgtg ggtgaccagt 2400

tgcacttggc ctctggattg tgggaattaa ggaagtgact catcctcttg aagatgctga 2460

aacaggagag aaaggggatg tatccatggg ggcagggcat gactttgtcc catttctaaa 2520

ggcctcttcc ttgctgtgtc ataccaggcc gccccagcct ctgagcccct gggactgctg 2580

cttcttaacc ccagtaagcc actgccacac gtctgaccct ctccacccca tagtgaccgg 2640

ctgcttttcc ctaagccaag ggcctcttgc ggtcccttct tactcacaca caaaatgtac 2700

ccagtattct aggtagtgcc ctattttaca attgtaaaac tgaggcacga gcaaagtgaa 2760

gacactggct catattcctg cagcctggag gccgggtgct cagggctgac acgtccaccc 2820

cagtgcaccc actctgcttt gactgagcag actggtgagc agactggtgg gatctgtgcc 2880

cagagatggg actgggaggg cccacttcag ggttctcctc tcccctctaa ggccgaagaa 2940

gggtccttcc ctctccccaa gacttggtgt cctttccctc cactccttcc tgccacctgc 3000

tgctgctgct gctgctaatc ttcagggcac tgctgctgcc tttagtcgct gaggaaaaat 3060

aaagacaaat gctgcgccct tccccaaaaa aaaaaaa 3097

<210> 8
<211> 1895
<212> DNA
<213> Homo sapiens

<400> 8
atgacaggaa gtgacccgtt aaggaagcag cacatcgctg cattcggctg gttttcaggg 60

tcttgttccc aatcagtttc cagccaacac cagggtgtcc tagtccgcag aggtgtgggg 120

gacacactcc ataatctcta cttttctttt tgtgcagctg agtcatggag ctttcagccc 180

cagcacatgg ctcctcctta actgcgtctg ctcaacctcc ctcagccctg tgaacagcat 240

ccccgcacac agacgcagag caggactctc tctgctgcca cttcaccttc ctgagagagg 300

accagcggcc agagcctcag tgactgccac cctggaggac agggcacaac aaccgtttct 360

ggagagaatg ggaggattcc agaggggcaa atatggaact atggctgaag gtagatcaga 420

agataacttg tctgcaacac caccggcatt gaggattatc ctagtgggca aaacaggctg 480

cgggaaaagt gccacaggga acagcatcct tggccagccc gtgtttgagt ccaagctgag 540

ggcccagtca gtgaccagga cgtgccaggt gaaaacagga acatggaacg ggaggaaagt 600

cctggtggtt gacacgccct ccatctttga gtcacaggcc gatacccaag agctgtacaa 660

gaacatcggg gactgctacc tgctctctgc cccggggccc cacgtcctgc ttctggtgat 720

ccagctgggg cgtttcactg ctcaggacac agtggccatc aggaaggtga aagaggtctt 780

tgggacaggg gccatgagac atgtggtcat cctcttcacc cacaaagagg acttaggggg 840

```
ccaggccctg gatgactatg tagcaaacac ggacaactgc agcctgaaag acctggtgcg      900

ggagtgtgag agaaggtact gtgccttcaa caactggggc tctgtggagg agcagaggca      960

gcagcaggca gagctcctgg ctgtgattga gaggctgggg agggagcgag agggctcctt     1020

ccacagcaat gacctcttct tggatgccca gctgctccaa agaactggag ctggggcctg     1080

ccaggaagac tacaggcagt accaggccaa agtggaatgg caggtggaga agcacaagca     1140

agagctgagg gagaacgaga gtaactgggc atacaaggcg ctcctcagag tcaaacactt     1200

gatgcttttg cattatgaga tttttgtttt tctattgttg tgcagcatac tttttttcat     1260

tatttttctg ttcatctttc attacattta aatctctgga ccctggagca cttctaatgt     1320

atcaccccat ggagtcattg ttctaataat caccaattca gactcagatc ctcgtggtct     1380

atggagcatg ctgcttgctg tctgtgcagc tcccatttcc ccttcttcct gatagacttg     1440

gagctgtgtg cctccactcc aaggctgcct gcctgctgta aacactattc cactctgtct     1500

gccaacaact gcttcaggaa tgggcctgag atcccatgca ggtccctgag aagtgagtaa     1560

aagtccgcag aggtggggat ggaagatctc tccttagata gaacctgtct tcctccctgg     1620

cattgtgggg tctgggcgtg acactgggac tctcagcagc tttgtgctgc caacctgaga     1680

ttgaaggcag tgcctcagag cagcacagag agttggggcc ccctgagccc tgagccacca     1740

gccctgcagc ctgccctatc tccgcatttc cagttgtatt agccaataga tttcctactt     1800

atttaagcta tttgagctcc gggtctcttc tacctgcatt ctaaaacatt caaagtaata     1860

aaaatttctc cacattcaaa aaaaaaaaaa aaaaa                                1895
```

<210> 9
<211> 1475
<212> DNA
<213> Homo sapiens

<400> 9

```
gggatcacac aggatccgga gctggtgctg ataacagcgg aatcccccgt ctacctctct       60

ccttggtcct ggaacagcgc tactgatcac caagtagcca caaaatataa taaaccctca      120

gcacttgctc agtagttttg tgaaagtctc aagtaaaaga gacacaaaca aaaaattctt      180

tttcgtgaag aactccaaaa ataaaattct ctagagataa aaaaaaaaaa aaaaggaaaa      240

tgccagctga tataatggag aaaaattcct cgtccccggt ggctgctacc ccagccagtg      300

tcaacacgac accggataaa ccaaagacag catctgagca cagaaagtca tcaaagccta      360

ttatggagaa aagacgaaga gcaagaataa atgaaagtct gagccagctg aaaacactga      420

ttttggatgc tctgaagaaa gatagctcgc ggcattccaa gctggagaag gcggacattc      480

tggaaatgac agtgaagcac ctccggaacc tgcagcgggc gcagatgacg gctgcgctga      540

gcacagaccc aagtgtgctg gggaagtacc gagccggctt cagcgagtgc atgaacgagg      600
```

tgacccgctt cctgtccacg tgcgagggcg ttaataccga ggtgcgcact cggctgctcg 660

gccacctggc caactgcatg acccagatca atgccatgac ctaccccggg cagccgcacc 720

ccgccttgca ggcgccgcca ccgcccccac cgggacccgg cggcccccag cacgcgccgt 780

tcgcgccgcc gccgccactc gtgcccatcc ccgggggcgc ggcgccccct cccggcggcg 840

ccccctgcaa gctgggcagc caggctggag aggcggctaa ggtgtttgga ggcttccagg 900

tggtaccggc tcccgatggc cagtttgctt tcctcattcc caacggggcc ttcgcgcaca 960

gcggccctgt catccccgtc tacaccagca acagcggcac ctccgtgggc cccaacgcag 1020

tgtcaccttc cagcggcccc tcgcttacgg cggactccat gtggaggccg tggcggaact 1080

gagggggctc aggccacccc tcctcctaaa ctccccaacc cacctctctt ccctccggac 1140

tctaaacagg aacttgaata ctgggagaga agaggacttt tttgattaag tggttacttt 1200

gtgttttttt aatttctaag aagttacttt ttgtagagag agctgtatta agtgactgac 1260

catgcactat atttgtatat attttatatg ttcatattgg attgcgcctt tgtattataa 1320

aagctcagat gacatttcgt tttttacacg agatttcttt tttatgtgat gccaaagatg 1380

tttgaaaatg ctcttaaaat atcttccttt ggggaagttt atttgagaaa atataataaa 1440

agaaaaaagt aaaggctttt aaaaaaaaaa aaaaa 1475

<210> 10
<211> 962
<212> DNA
<213> Homo sapiens

<400> 10
gggaaagaat gcggagccgg gttcacacac cccgcggcgg cgaggcctta aatagggaaa 60

cggcctgagg cgcgcgcggg cctggagccg ggatccgccc taggggctcg gatcgccgcg 120

cgctcgccgc tcgcccgcca gcccgcccgt ggtccgtggc ggcgcgctcc acccggcacg 180

gggaggcgcg gggcgcacca tggccgcaga cacgccgggg aaaccgagcg cctcgccgat 240

ggcaggagcg ccggccagcg ccagccggac cccagacaag ccccggagcg cggccgagca 300

ccgcaagtcc tccaagccgg tcatggagaa gcggcgccga gcgcgtatta acgagagcct 360

cgctcagctc aaaaccctca tcctggacgc cctcagaaaa gagagctccc gccactcgaa 420

gctggagaag gcggacatcc tggagatgac cgtgagacac ctgcggagcc tgcgtcgcgt 480

gcaggtgacg gccgcgctca gcgccgaccc cgccgttctg ggcaagtacc gcgccggctt 540

ccacgagtgt ctggcggagg tgaaccgctt cctggccggc tgcgagggcg tcccggccga 600

cgtgcgctcc cgcctgctgg gccacctggc agcctgcctg cgccagctgg gaccctcccg 660

ccgcccggcc tcgctgtccc cggctgcccc cgcagaggcc ccagcgcccg aggtctacgc 720

gggccgcccg ctgctgccat cgctcggcgg ccccttccct ctgctcgcgc cgccgctgct 780

gccgggtctg acccgggcgc tgcccgccgc ccccagggcg gggccgcagg gcccgggtgg 840

gccctggagg ccgtggctgc gctgaggctg tggccctgag actgcatcgg aggcggcgcc 900

ccgttctagg gccgtggcct ttgccgagac tgtagcagag aaaacgtatt tattattcca 960

ga 962

<210> 11
<211> 1319
<212> DNA
<213> Homo sapiens

<400> 11
cgcgcttggc cttgcccgcg cccgctcgcc tcgtctcgcc cggcctcccc gcgtcgcctc 60

gtcgcctgtt ccgcgccagg catggccccc agcactgtgg ccgtggagct gctcagcccc 120

aaagagaaaa accgactgcg gaagccggtg gtggagaaga tgcgccgcga ccgcatcaac 180

agcagcatcg agcagctgaa gctgctgctg gagcaggagt tcgcgcggca ccagcccaac 240

tccaagctgg agaaggccga catcctggag atggctgtca gctacctgaa gcacagcaaa 300

gccttcgtcg ccgccgccgg ccccaagagc ctgcaccagg actacagcga aggctactcg 360

tggtgcctgc aggaggccgt gcagttcctg acgctccacg ccgccagcga cacgcagatg 420

aagctgctgt accacttcca gcggcccccg gccgcgcccg ccgcgcccgc caaggagccc 480

aaggcgccgg gcgccgcgcc cccgcccgcg ctctccgcca aggccaccgc cgccgccgcc 540

gccgcgcacc agcccgcctg cggcctctgg cggccctggt gacccggcgg gacctgcggg 600

cgcgcggccc gacgaccaga gggcgagcct gctcctctcg cctgtaggga agcgccttcc 660

cgccgtcgtc cgcccccggc ttggacgcgc ccttctccgg aaggctctgg ccccaagctg 720

gccggcccgc aggagcccca ttctcagaga atgtgtgtgc agagtccctg ccgttttagg 780

acaatcaggg cccatcttct gccaagtgtc tgaccccatg ggttgttct gtgtttgcat 840

ttaagcaagt gacttctggg aagtccccgg ccgcccgggg ttctatgata tttgtagtgc 900

cggggctcgc acactgctgc cccagcctg tagaggactt tcttcagggc ccgtagctgc 960

tgggcgtacc cctggcaggc gggctgtgcc gcgggcacat ttgcctttg tgaaggccga 1020

actcgagctg tatcctcata ggaaacagtg atcaccccgg acgggcgtcc aggaccctga 1080

gggccatggc caaaaggctc ctgagtgtgc ctggtggtct ggctggggct cacggtgggc 1140

tgtctgggga gggtgggtgc ctccactatg atccttaaag gattcctctg tgtgggtgga 1200

tgcgtgtggg cacgactttg tactcagaaa ttgaactctc agtcacgtgg aagccacggg 1260

actgctccga agccgccata ataaaatctg attgttcagc ccccaaaaaa aaaaaaaaa 1319

<210> 12
<211> 1684

<212> DNA
<213> Homo sapiens

<400> 12
gaggagcaat ggtcacccgg gatcgagctg agaataggga cggccccaag atgctcaagc 60

cgcttgtgga gaagcggcgc cgggaccgca tcaaccgcag cctggaagag ctgaggctgc 120

tgctgctgga gcggacccgg gaccagaacc tccggaaccc gaagctggag aaagcggaga 180

tattggagtt cgccgtgggc tacttgaggg agcgaagccg ggtggagccc ccggggggttc 240

cccggtcccc agtccaggac gccgaggcgc tcgccagctg ctacttgtcc ggtttccgcg 300

agtgcctgct tcgcttggcg gccttcgcgc acgacgccag cccggccgcc cgcgcccagc 360

tcttctccgc gctgcacggc tatctgcgcc ccaaaccgcc ccggcccaag ccggtagatc 420

cgaggcctcc agcgccgcgc ccatccctgg accccgccgc accggccctt ggccctgcgc 480

tgcaccagcg ccccccagtg caccagggcc accctagccc gcgctgcgca tggtccccat 540

ccctctgctc cccgcgcgcc ggggattctg gcgcgccggc gcccctcacc ggactgctgc 600

cgccgccacc gccgcctcac agacaagacg gggcgcccaa ggccccgctg cccccgccgc 660

ccgctttctg gagaccttgg ccctgagcct tggggggtgg tgggggcggg gtctaggggt 720

ggggtagaga ctccagcccg agggcagcag agggacccgg gcgtccgggc gagcaggtgt 780

tggggagggc agtggggcgc gcgggctcag cgcgcgggtg agatgtggtc tatattagag 840

tatctatata aatatatatt tccctggttc ctgtcccttt tccctgcccc aacttctccc 900

ttgcgtctag gattgtactc tctctgcccc tcagcccagt cccagtccct tcccgagtcc 960

ctagtgcatg gaataaagtg gttattaaat ccccgtgtgt ccccgagcca ggggcctgcc 1020

tttatctcga cgtccacgcc cactttccct tcccttctgt ctcccaccct cagtcctgct 1080

ctccatggcc caagccccgg ggcagacagg taagtaaaga agagagcaga gcgggaactg 1140

agatcgaaat tgaaaccagg tggaaagaga gagatagggt agggggagaa gggatggggg 1200

cctttaagaa aaaaacggat aaaaaggaaa aattgaaata aaatcgactc tggtgggatt 1260

cgaacccaca acctttgaat tgctctattc gtcactagaa gtccaatgcg ctatccattg 1320

cgccacagag ccacccgacg aacggcggcg tcttgtagct tacgggtact agagtgggaa 1380

tggggcaggg ttggggagcg gggctaaggg acttgggcgg gacatgccag gagggcgcgg 1440

tttggatctc agaggccaag ccaggtagag gtagcgggcg caaagcatgt tagccaggtg 1500

agagagaggg cgcacatggg tcgaaaaaac agggagggag agcaaccgaa aatggctgag 1560

cgagcgagtg cagagctccg gctgcccgct tgggggtgt ttccggctca ggcgctcccc 1620

actcccagat atagtcccac ccaaataaac tagttttgtt gtaaattaaa aaaaaaaaaa 1680

aaaa 1684

<210> 13
<211> 2319
<212> DNA
<213> Homo sapiens

<400> 13
ttccccactc ccccgccctc cccagggccc tgggaagggg ctcagcgtgg gaaaggatgg 60

ttgagtttta accagaggca aagcgtgagc gggatcagtg tgtgcggaac gcaagcagcc 120

gagagcggag aggcgccgct gtagttaact cctccctgcc cgccgcgccg accctcccca 180

ggaaccccca gggagccagc atgaagcgag ctcaccccga gtacagctcc tcggacagcg 240

agctggacga gaccatcgag gtggagaagg agagtgcgga cgagaatgga aacttgagtt 300

cggctctagg ttccatgtcc ccaactacat cttcccagat tttggccaga aaaagacgga 360

gaggaataat tgagaagcgc cgacgagacc ggatcaataa cagtttgtct gagctgagaa 420

ggctggtacc cagtgctttt gagaagcagg gatctgctaa gctagaaaaa gccgagatcc 480

tgcagatgac cgtggatcac ctgaaaatgc tgcatacggc aggagggaaa ggttactttg 540

acgcgcacgc ccttgctatg gactatcgga gtttgggatt tcgggaatgc ctggcagaag 600

ttgcgcgtta tctgagcatc attgaaggac tagatgcctc tgacccgctt cgagttcgac 660

tggtttcgca tctcaacaac tacgcttccc agcgggaagc cgcgagcggc gcccacgcgg 720

gcctcggaca cattccctgg gggaccgtct tcggacatca cccgcacatc gcgcacccgc 780

tgttgctgcc ccagaacggc cacgggaacg cgggcaccac ggcctcaccc acggaaccgc 840

accaccaggg caggctgggc tcggcacatc cggaggcgcc tgctttgcga gcgcccccta 900

gcggcagcct cggaccggtg ctccctgtgg tcacctccgc ctccaaactg tcgccgcctc 960

tgctctcctc agtggcctcc ctgtcggcct tccccttctc tttcggctcc ttccacttac 1020

tgtctcccaa tgcactgagc ccttcagcac ccacgcaggc tgcaaacctt ggcaagccct 1080

atagaccttg ggggacggag atcggagctt tttaaagaac tgatgtagaa tgagggaggg 1140

gaaagtttaa aatcccagct gggctggact gttgccaaca tcaccttaaa gtcgtcagta 1200

aaagtaaaaa ggaaaaaggt acactttcag ataatttttt ttttaaagac taaaggtttg 1260

ttggtttact tttatctttt ttaatgtttt tttcatcatg tcatgtatta gcagttttta 1320

aaaactagtt gttaaatttt gttcaagaca ttaaattgaa atagtgagta taagccaaca 1380

ctttgtgata ggtttgtact gtgcctaatt tactttgtaa accagaatga ttccgttttt 1440

gcctcaaaat ttggggaatc ttaacattta gtatttttgg tctgtttttc tccttgtata 1500

gttatggtct gtttttagaa ttaattttcc aaaccactat gcttaatgtt aacatgattc 1560

tgtttgttaa tattttgaca gattaaggtg ttgtataaat aatattcttt tgggggggagg 1620

ggaactatat tgaattttat atttctgagc aaagcgttga caaatcagat gatcagcttt 1680

atccaagaaa gaagactagt aaattgtctg cctcctatag cagaaaggtg aatgtacaaa 1740

```
ctgttggtgg ccctgaatcc atctgaccag ctgctggtat ctgccaggac tggcagttct    1800

gatttagtta ggagagagcc gctgataggt taggtctcat ttggagtgtt ggtggaaagg    1860

aaactgaagg taattgaata gaatacgcct gcatttacca gccccagcaa cacaaagaat    1920

ttttaatcac acggatctca aattcacaaa tgttaacatg gataagtgat catggtgtgc    1980

gagtggtcaa ttgagtagta cagtggaaac tgttaaatgc ataacctaat tttcctggga    2040

ctgccatatt ttcttttaac tggaaatttt tatgtgagtt ttccttttgg tgcatggaac    2100

tgtggttgcc aaggtattta aaagggcttt cctgcctcct tctctttgat ttatttaatt    2160

tgatttgggc tataaaatat catttttcag gtttattctt ttagcaggtg tagttaaacg    2220

acctccactg aactgggttt gacctctgtt gtactgatgt gttgtgacta aataaaaaag    2280

aaagaacaaa gtaaaaaaaa aaaaaaaaaa aaaaaaaaa                           2319
```

```
<210>  14
<211>  2672
<212>  DNA
<213>  Homo sapiens

<400>  14
gcgtggccgg cgccggctct tgcggccgag cagagttgcg gcgtgggaaa gagccgctag      60

gagcagaccg cgccgccgcc ggagccgcgc ctgcccaggc ccggggaggg aggaggcggg     120

cgtcagggtg ctgcgccccg ctcggcgtcc gagcttccgg ccgggctgtg ccccgcgcgg     180

tcttcgccgg gatgaagcgc ccctgcgagg agacgacctc cgagagcgac atggacgaga     240

ccatcgacgt ggggagcgag aacaattact cggggcaaag tactagctct gtgattagat     300

tgaattctcc aacaacaaca tctcagatta tggcaagaaa gaaaaggaga gggattatag     360

agaaaaggcg tcgggatcgg ataaataaca gtttatctga gttgagaaga cttgtgccaa     420

ctgcttttga aaaacaagga tctgcaaagt tagaaaaagc tgaaatattg caaatgacag     480

tggatcattt gaagatgctt caggcaacag gggtaaagg ctactttgac gcacacgctc     540

ttgccatgga cttcatgagc ataggattcc gagagtgcct aacagaagtt gcgcggtacc     600

tgagctccgt ggaaggcctg gactcctcgg atccgctgcg ggtgcggctt gtgtctcatc     660

tcagcacttg cgccacccag cgggaggcgg cggccatgac atcctccatg gcccaccacc     720

atcatccgct ccacccgcat cactgggccg ccgccttcca ccacctgccc gcagccctgc     780

tccagcccaa cggcctccat gcctcagagt caacccctg tcgcctctcc acaacttcag     840

aagtgcctcc tgcccacggc tctgctctcc tcacggccac gtttgcccat gcggattcag     900

ccctccgaat gccatccacg ggcagcgtcg ccccctgcgt gccacctctc tccacctctc     960

tcttgtccct ctctgccacc gtccacgccg cagccgcagc agccaccgcg gctgcacaca    1020

gcttccctct gtccttcgcg ggggcattcc ccatgcttcc cccaaacgca gcagcagcag    1080
```

tggccgcggc cacagccatc agcccgccct tgtcagtatc agccacgtcc agtcctcagc 1140

agaccagcag tggaacaaac aataaacctt accgaccctg ggggacagaa gttggagctt 1200

tttaaatttt tcttgaactt cttgcaatag taactgaatg tcctccattt cagagtcagc 1260

ttaaaacctc tgcaccctga aggtagccat acagatgccg acagatccac aaaggaacaa 1320

taaagctatt tgagacacaa acctcacgag tggaaatgtg gtattctctt ttttttctct 1380

cccttttttg tttggttcaa ggcagctcgg taactgacat cagcaacttt tgaaaacttc 1440

acacttgtta ccatttagaa gtttcctgga aaatatatgg accgtaccat ccagcagtgc 1500

atcagtatgt ctgaattggg gaagtaaaat gccctgactg aattctcttg agactagatg 1560

ggacatacat atatagagag agagtgagag agtcgtgttt cgtaagtgcc tgagcttagg 1620

aagttttctt ctggatatat aacattgcac aagggaagac gagtgtggag gataggttaa 1680

gaaaggaaag ggacagaagt cttgcaatag gctgcagaca ttttaatacc atgccagaga 1740

agagtattct gctgaaacca acaggtttta ctggtcaaaa tgactgctga aaataatttt 1800

caagttgaaa gatctagttt tatcttagtt tgccttcttt gtacagacat gccaagaggt 1860

gacatttagc agtgcattgg tataagcaat tatttcatca gttctcagat taacaagcat 1920

ttctgctctg cctgcaggcc cccaggcact tttttttttg gatggctcaa aatatggtgc 1980

tgctttatat aaaccttaca tttatatagt gcacctatga gcagttgcct accatgtgtc 2040

caccagaggc tatttaattc atgccaactt gaaaactctc cagtttgtag gagtttggtt 2100

taatttattc agtttcatta ggactatttt tatatattta tcctcttcat tttctcctaa 2160

tgatgcaaca tctattcttg tcaccctttg ggagaagtta catttctgga ggtgatgaag 2220

caaggaggga gcactaggaa gagaaaagct acaattttta aagctctttg tcaagttagt 2280

gattgcattt gatcccaaaa caagatgaat gtatgcaatg ggatgtacat aagttatttt 2340

tgcccatgcc taaactagtg ctatgtaatg gggttgtggt tttgtttttt tcgatttcgt 2400

ttaatgacaa aataatctct taatatgctg aaatcaagca cgtgagagtt tttgtttaaa 2460

agataagaga cacagcatgt attatgcact tcatttctct actgtgtgga gaaagcaata 2520

aacattatga gaatgttaaa cgttatgcaa aattatactt ttaaatattt gttttgaaat 2580

tactgtacct agtctttttt gcattacttt gtaacctttt tctatgcaag agtctttaca 2640

taccactaat taaatgaagt cctttttgac ta 2672

<210> 15
<211> 4131
<212> DNA
<213> Homo sapiens

<400> 15
ccgactggga gccttagccg cggggctgag accaggcagc ctgcgttcgc catgaagcga 60

cccaaggagc cgagcggctc cgacggggag tccgacggac ccatcgacgt gggccaagag 120
ggccagctga gccagatggc caggccgctg tccaccccca gctcttcgca gatgcaagcc 180
aggaagaaac gcagagggat catagagaaa cggcgtcgag accgcatcaa cagtagcctt 240
tctgaattgc gacgcttggt ccccactgcc tttgagaaac agggctcttc caagctggag 300
aaagccgagg tcttgcagat gacggtggat cacttgaaaa tgctccatgc cactggtggg 360
acaggattct ttgatgcccg agccctggca gttgacttcc ggagcattgg ttttcgggag 420
tgcctcactg aggtcatcag gtacctgggg gtccttgaag ggcccagcag ccgtgcagac 480
cccgtccgga ttcgccttct ctcccacctc aacagctacg cagccgagat ggagccttcg 540
cccacgccca ctggcccttt ggccttccct gcctggccct ggtctttctt ccatagctgt 600
ccagggctgc cagccctgag caaccagctc gccatcctgg gaagagtgcc cagccctgtc 660
ctccccggtg tctcctctcc tgcttacccc atcccagccc tccgaaccgc tccccttcgc 720
agagccacag gcatcatcct gccagcccgg aggaatgtgc tgcccagtcg aggggcatct 780
tccacccgga gggcccgccc cctagagagg ccagcgaccc ctgtgcctgt cgcccccagc 840
agcagggctg ccaggagcag ccacatcgct cccctcctgc agtcttcctc cccaacaccc 900
cctggtccta cagggtcggc tgcttacgtg gctgttccca cccccaactc atcctcccca 960
gggccagctg ggaggccagc gggagccatg ctctaccact cctgggtctc tgaaatcact 1020
gaaatcgggg ctttctgagc tgccccttca ccaccccgcc ccaaggaata aggaaggttc 1080
ttttaccagg agcccaaaaa agggcactgc cttttctgct ttgcttcatg gactggctca 1140
tatgtgaagg cacgttctcc agccatcaga ggccccctcc tcctccaacc catctctcct 1200
tctcactgtt atcccagctt atccacccag ctctcctgga gctgttctgg tctcagaggc 1260
ttggttccat ttctcacctg aacagatgag tcctgggaga gaccctcaga gatccgccca 1320
gacccctctc ctgccctctg cacaccagca gcaggcatga accttgggtc tgggaaaaag 1380
ctttaacctg cagggcacca ggacccaagg caggctgttc cttggggcgg tcagacccca 1440
gtcaggagca atgactgact ggctgcagcc ttcccacgcc aagaggctgg aacatagtgt 1500
ctgcctcgct tcctggagat agtaactgag caggggctac aaagaggtct cctgggaacc 1560
ctgtctgccc cttcccacct gtccttgggc cacaccatca cactgaacca caggacagac 1620
cctttctcca ccacagccaa ggcctggaga ctgggggccc agcagagcct gctcccaccc 1680
tcctcccagc agcagacacc caccctctca ctgactaaca ggtccctgca cacagctggc 1740
ctggtaaacc cagctgggag gtttctaggc agcagcaaaa ctctgtgaca gggtgtcctc 1800
acaccaggcc ttggacagct ctcccagaca ggagccaggg ttgagcaatg gagagcccag 1860
cccccacgtc ttacagtcgc catcctccag gcgtgtggtc cctccccatt gggtgcacag 1920

tgcagagggg ccgtggcccc atgtgatggt gcgcagagag gaacctcttg ggattcagca 1980
ccagacgtct gtgctgcctg gtttgcatcc ggctcacaga gcccagactg ctggaacagc 2040
caaggactgt caggctggac aaaaataact gcaaggaggg gcaagagaaa ggatgattcg 2100
aggcaccttg gcccttcaag gtcatgcagt gggtcgagcg cctgagatcc tgttcaccag 2160
gactccacag agctggctct gctcagaagc catttcattc cccggctcca ccctaggcca 2220
ctttttctaa cagaggaaac aaatggtcca gcagtcgttc ccagcagaac agcggagcct 2280
ggactgacac ccagtgggac cagtgttgcc acaccagttg ataaaatgca gaaacccttc 2340
tgtactcgtt ggtaaatatc tactccccca agtgactcca ggtgcccccc accgcctggc 2400
acttccccca ggactcctac gatctggtta ctgcctggcc gatccaaggc tgtggagtcc 2460
cagagccagc agttcactgg tgctcattcc acactggtta gatacttcag ttgtcacccc 2520
tgggaagatt ctcccacctc ctccctttga tggaaccacc ctccccagag gctgcattga 2580
ggagactcca cagactgaaa agtgagtttg cagaaacctt ggggaaaagg gccctttcaa 2640
agaagtggat aagagggagg agatcattga gtgacccaga aagctctttt gaaaagacag 2700
actcctcaag gagagataaa gaggaaagca cctctttcat tttttagtgt gagctaattc 2760
catcagactg ctgtcctcct ggacccatct gagatgtgca gtagcaagga gagggggat 2820
cattttagag agtgggtcat tggcagggag tgctccggag ggaggcagag gggagactgt 2880
ggtagaagga agacagaact cacacatgct cccaggattg gggacaggga cagaggaggt 2940
aacagaaggc aaaggccagt ttccccgtta tcatgaaggg gcccactcag gacaggaaca 3000
aggacaactc ctcctcctcc tcctcctctc ctgctgctcc tgggatacca ggtcagtgat 3060
gtagtcttgc agtttggcaa cttcctagcc tgagaatccc tagtggggct gtgggaaaca 3120
catttccacg ttgcaagcat gcaactccaa agaatctgtg atgccactga aatgagatgg 3180
gaatgatcca gctctttcag catcttggtt gaacttgctt tcattgtccc tgggatattg 3240
tggaaggaaa ggtgactgtg tgatctgatt ctgtggtcaa ggacttgcat cttgtgtttc 3300
tatccccaag ccttcctggt gtctccaact cctaccccat tgcatgggtt gttgcggaca 3360
tccaataaag attttttag tgcttctgga aacttccagt agattctact tctaaactat 3420
ctctggagtc catccacttc tgtctgcacc cacagccatc ctggccaggc cacatcacct 3480
cccccagatc actgccctgg cctcagaaag gtcttccctc ttgctttgtc aatcagttct 3540
cagtagcagc agagagaaat tgaaagctgc aggtcatatc gtatcatctt tagtttgaaa 3600
acctcactct cttaccctat tgttctaaag gtcttctttt ggtcccaacc tcatttccag 3660
cctcatttct tgccagtccc agacttgctc cctgagcttc tgccacctgc ccttccttca 3720
tttcctcgac attccagcct tgttcccacc tccagcactt tgcatatgct gttccctttg 3780
ccaagaatgc tcttccccta ccctgtgcat ggctgagttc tgcagaccct caggccttgg 3840

cttcaacgtt gcctcgtcca agaggccttc ctcgactact ttacttgtgg agttcctcta 3900

tcacaaggcc tctgttcttt cccttcatgg agaatttgcc actgcatatc catttgtgta 3960

atttacttgt tggttgactg tgcctcccac tcgagtgtaa gctcatgagg ccaggtgcca 4020

tgcctggttc agtctccact ctgtacccag cattgagcac agggcctggt ccatagttgg 4080

cgttcaataa atacttgttg aagaagtgaa ctgaaaaaa aaaaaaaaaa a 4131

<210> 16
<211> 2969
<212> DNA
<213> Homo sapiens

<400> 16
actttagctc agacctttct tttaaccttg cctatcatgt ttcgagtcag aatttaaata 60

ctgtgcagtt taagctacaa tacgcttggc ctataacttg gttccaggca tttatattta 120

tgtcactttt gtctacttat tatactaaca aggtggaaaa agcaatccca gtctctccaa 180

aagacaagat gtgaaatgga gaagtatctg acacctcagc ttcctccagt tcctataatt 240

ccagagcata aaaagtatag acgagacagt gcctcagtcg tagaccagtt cttcactgac 300

actgaagggt taccttacag tatcaacatg aacgtcttcc tccctgacat cactcacctg 360

agaactggcc tctacaaatc ccagagaccg tgcgtaacac acatcaagac agaacctgtt 420

gccattttca gccaccagag tgaaacgact gcccctcctc cggccccgac ccaggccctc 480

cctgagttca ccagtatatt cagctcacac cagaccgcag ctccagaggt gaacaatatt 540

ttcatcaaac aagaacttcc tacaccagat cttcatcttt ctgtccctac ccagcagggc 600

cacctgtacc agctactgaa tacaccggat ctagatatgc ccagttctac aaatcagaca 660

gcagcaatgg acactcttaa tgtttctatg tcagctgcca tggcaggcct taacacacac 720

acctctgctg ttccgcagac tgcagtgaaa caattccagg gcatgccccc ttgcacatac 780

acaatgccaa gtcagtttct tccacaacag gccacttact ttcccccgtc accaccaagc 840

tcagagcctg gaagtccaga tagacaagca gagatgctcc agaatttaac cccacctcca 900

tcctatgctg ctacaattgc ttctaaactg gcaattcaca atccaaattt acccaccacc 960

ctgccagtta actcacaaaa catccaacct gtcagataca atagaaggag taaccccgat 1020

ttggagaaac gacgcatcca ctactgcgat taccctggtt gcacaaaagt ttataccaag 1080

tcttctcatt taaaagctca cctgaggact cacactggtg aaaagccata caagtgtacc 1140

tgggaaggct gcgactggag gttcgcgcga tcggatgagc tgacccgcca ctaccggaag 1200

cacacaggcg ccaagccctt ccagtgcggg gtgtgcaacc gcagcttctc gcgctctgac 1260

cacctggccc tgcatatgaa gaggcaccag aactgagcac tgcccgtgtg acccgttcca 1320

ggtcccctgg gctccctcaa atgacagacc taactattcc tgtgtaaaaa caacaaaaac 1380

aaacaaaagc aagaaaacca caactaaaac tggaaatgta tattttgtat atttgagaaa 1440
acagggaata cattgtatta ataccaaagt gtttggtcat tttaagaatc tggaatgctt 1500
gctgtaatgt atatggcttt actcaagcag atctcatctc atgacaggca gccacgtctc 1560
aacatgggta aggggtgggg gtggagggga gtgtgtgcag cgtttttacc taggcaccat 1620
catttaatgt gacagtgttc agtaaacaaa tcagttggca ggcaccagaa gaagaatgga 1680
ttgtatgtca agattttact tggcattgag tagtttttt caatagtagg taattcctta 1740
gagatacagt atacctggca attcacaaat agccattgaa caaatgtgtg ggtttttaaa 1800
aattatatac atatatgagt tgcctatatt tgctattcaa aattttgtaa atatgcaaat 1860
cagctttata ggtttattac aagtttttta ggattctttt ggggaagagt cataattctt 1920
ttgaaaataa ccatgaatac acttacagtt aggatttgtg gtaaggtacc tctcaacatt 1980
accaaaatca tttctttaga gggaaggaat aatcattcaa atgaacttta aaaaagcaaa 2040
tttcatgcac tgattaaaat aggattattt taaatacaaa aggcatttta tatgaattat 2100
aaactgaaga gcttaaagat agttacaaaa tacaaaagtt caacctctta caataagcta 2160
aacgcaatgt catttttaaa aagaaggact tagggtgtcg ttttcacata tgacaatgtt 2220
gcatttatga tgcagtttca agtaccaaaa cgttgaattg atgatgcagt tttcatatat 2280
cgagatgttc gctcgtgcag tactgttggt taaatgacaa tttatgtgga ttttgcatgt 2340
aatacacagt gagacacagt aattttatct aaattacagt gcagtttagt taatctatta 2400
atactgactc agtgtctgcc tttaaatata aatgatatgt tgaaaactta aggaagcaaa 2460
tgctacatat atgcaatata aaatagtaat gtgatgctga tgctgttaac caaagggcag 2520
aataaataag caaaatgcca aaaggggtct taattgaaat gaaaatttaa ttttgttttt 2580
aaaatattgt ttatctttat ttattttgtg gtaatatagt aagttttttt agaagacaat 2640
tttcataact tgataaatta tagttttgtt tgttagaaaa gttgctctta aaagatgtaa 2700
atagatgaca aacgatgtaa ataattttgt aagaggcttc aaaatgttta tacgtggaaa 2760
cacacctaca tgaaaagcag aaatcggttg ctgttttgct tctttttccc tcttattttt 2820
gtattgtggt catttcctat gcaaataatg gagcaaacag ctgtatagtt gtagaatttt 2880
ttgagagaat gagatgttta tatattaacg acaatttttt ttttggaaaa taaaaagtgc 2940
ctaaaagatg taaaaaaaaa aaaaaaaaa 2969

<210> 17
<211> 4518
<212> DNA
<213> Homo sapiens

<400> 17
ggagtttatt cataacgcgc tctccaagta tacgtggcaa tgcgttgctg ggttatttta 60

atcattctag gcatcgtttt cctccttatg cctctatcat tcctccctat ctacactaac 120

atcccacgct ctgaacgcgc gcccattaat acccttcttt cctccactct ccctgggact 180

cttgatcaaa gcgcggccct ttccccagcc ttagcgaggc gccctgcagc ctggtacgcg 240

cgtggcgtgg cggtgggcgc gcagtgcgtt ctcggtgtgg agggcagctg ttccgcctgc 300

gatgatttat actcacagga caaggatgcg gtttgtcaaa cagtactgct acggaggagc 360

agcagagaaa gggagagggt ttgagaggga gcaaaagaaa atggtaggcg cgcgtagtta 420

attcatgcgg ctctcttact ctgtttacat cctagagcta gagtgctcgg ctgcccggct 480

gagtctcctc cccaccttcc ccaccctccc caccctcccc ataagcgccc ctcccgggtt 540

cccaaagcag agggcgtggg ggaaaagaaa aaagatcctc tctcgctaat ctccgcccac 600

cggcccttta taatgcgagg gtctggacgg ctgaggaccc ccgagctgtg ctgctcgcgg 660

ccgccaccgc cgggccccgg ccgtccctgg ctcccctcct gcctcgagaa gggcagggct 720

tctcagaggc ttggcgggaa aaagaacgga gggagggatc gcgctgagta taaaagccgg 780

ttttcggggc tttatctaac tcgctgtagt aattccagcg agaggcagag ggagcgagcg 840

ggcggccggc tagggtggaa gagccgggcg agcagagctg cgctgcgggc gtcctgggaa 900

gggagatccg gagcgaatag ggggcttcgc ctctggccca gccctcccgc tgatccccca 960

gccagcggtc cgcaaccctt gccgcatcca cgaaactttg cccatagcag cgggcgggca 1020

ctttgcactg gaacttacaa cacccgagca aggacgcgac tctcccgacg cggggaggct 1080

attctgccca tttggggaca cttccccgcc gctgccagga cccgcttctc tgaaaggctc 1140

tccttgcagc tgcttagacg ctggattttt ttcgggtagt ggaaaaccag cagcctcccg 1200

cgacgatgcc cctcaacgtt agcttcacca acaggaacta tgacctcgac tacgactcgg 1260

tgcagccgta tttctactgc gacgaggagg agaacttcta ccagcagcag cagcagagcg 1320

agctgcagcc cccggcgccc agcgaggata tctggaagaa attcgagctg ctgcccaccc 1380

cgcccctgtc ccctagccgc cgctccgggc tctgctcgcc ctcctacgtt gcggtcacac 1440

ccttctccct tcggggagac aacgacggcg gtggcgggag cttctccacg gccgaccagc 1500

tggagatggt gaccgagctg ctgggaggag acatggtgaa ccagagtttc atctgcgacc 1560

cggacgacga gaccttcatc aaaaacatca tcatccagga ctgtatgtgg agcggcttct 1620

cggccgccgc caagctcgtc tcagagaagc tggcctccta ccaggctgcg cgcaaagaca 1680

gcggcagccc gaaccccgcc cgcggccaca gcgtctgctc cacctccagc ttgtacctgc 1740

aggatctgag cgccgccgcc tcagagtgca tcgacccctc ggtggtcttc ccctaccctc 1800

tcaacgacag cagctcgccc aagtcctgcg cctcgcaaga ctccagcgcc ttctctccgt 1860

cctcggattc tctgctctcc tcgacggagt cctccccgca gggcagcccc gagcccctgg 1920

tgctccatga ggagacaccg cccaccacca gcagcgactc tgaggaggaa caagaagatg 1980
aggaagaaat cgatgttgtt tctgtggaaa agaggcaggc tcctggcaaa aggtcagagt 2040
ctggatcacc ttctgctgga ggccacagca aacctcctca cagcccactg gtcctcaaga 2100
ggtgccacgt ctccacacat cagcacaact acgcagcgcc tccctccact cggaaggact 2160
atcctgctgc caagagggtc aagttggaca gtgtcagagt cctgagacag atcagcaaca 2220
accgaaaatg caccagcccc aggtcctcgg acaccgagga gaatgtcaag aggcgaacac 2280
acaacgtctt ggagcgccag aggaggaacg agctaaaacg gagctttttt gccctgcgtg 2340
accagatccc ggagttggaa aacaatgaaa aggcccccaa ggtagttatc cttaaaaaag 2400
ccacagcata catcctgtcc gtccaagcag aggagcaaaa gctcatttct gaagaggact 2460
tgttgcggaa acgacgagaa cagttgaaac acaaacttga acagctacgg aactcttgtg 2520
cgtaaggaaa agtaaggaaa acgattcctt ctaacagaaa tgtcctgagc aatcacctat 2580
gaacttgttt caaatgcatg atcaaatgca acctcacaac cttggctgag tcttgagact 2640
gaaagattta gccataatgt aaactgcctc aaattggact ttgggcataa aagaactttt 2700
ttatgcttac catctttttt ttttctttaa cagatttgta tttaagaatt gtttttaaaa 2760
aattttaaga tttacacaat gtttctctgt aaatattgcc attaaatgta aataacttta 2820
ataaaacgtt tatagcagtt acacagaatt tcaatcctag tatatagtac ctagtattat 2880
aggtactata aaccctaatt ttttttattt aagtacattt tgctttttaa agttgatttt 2940
tttctattgt tttagaaaa aataaaataa ctggcaaata tatcattgag ccaaatctta 3000
agttgtgaat gttttgtttc gtttcttccc cctcccaacc accaccatcc ctgtttgttt 3060
tcatcaattg ccccttcaga gggtggtctt aagaaaggca agagttttcc tctgttgaaa 3120
tgggtctggg ggccttaagg tctttaagtt cttggaggtt ctaagatgct tcctggagac 3180
tatgataaca gccagagttg acagttagaa ggaatggcag aaggcaggtg agaaggtgag 3240
aggtaggcaa aggagataca agaggtcaaa ggtagcagtt aagtacacaa agaggcataa 3300
ggactgggga gttgggagga aggtgaggaa gaaactcctg ttactttagt taaccagtgc 3360
cagtcccctg ctcactccaa acccaggaat tctgcccagt tgatggggac acggtgggaa 3420
ccagcttctg ctgccttcac aaccaggcgc cagtcctgtc catgggttat ctcgcaaacc 3480
ccagaggatc tctgggagga atgctactat taaccctatt tcacaaacaa ggaaatagaa 3540
gagctcaaag aggttatgta acttatctgt agccacgcag ataatacaaa gcagcaatct 3600
ggacccattc tgttcaaaac acttaaccct tcgctatcat gccttggttc atctgggtct 3660
aatgtgctga gatcaagaag gtttaggacc taatggacag actcaagtca taacaatgct 3720
aagctctatt tgtgtcccaa gcactcctaa gcattttatc cctaactcta catcaacccc 3780
atgaaggaga tactgttgat ttccccatat tagaagtaga gagggaagct gaggcacaca 3840

aagactcatc cacatgccca agattcactg atagggaaaa gtggaagcga gatttgaacc 3900

caggctgttt actcctaacc tgtccaagcc acctctcaga cgacggtagg aatcagctgg 3960

ctgcttgtga gtacaggagt tacagtccag tgggttatgt tttttaagtc tcaacatcta 4020

agcctggtca ggcatcagtt ccccttttt tgtgatttat tttgttttta ttttgttgtt 4080

cattgtttaa tttttccttt tacaatgaga aggtcaccat cttgactcct accttagcca 4140

tttgttgaat cagactcatg acggctcctg ggaagaagcc agttcagatc ataaaataaa 4200

acatatttat tctttgtcat gggagtcatt attttagaaa ctacaaactc tccttgcttc 4260

catccttttt tacatactca tgacacatgc tcatcctgag tccttgaaaa ggtatttttg 4320

aacatgtgta ttaattataa gcctctgaaa acctatggcc caaaccagaa atgatgttga 4380

ttatataggt aaatgaagga tgctattgct gttctaatta cctcattgtc tcagtctcaa 4440

agtaggtctt cagctccctg tactttggga ttttaatcta ccaccaccca taaatcaata 4500

aataattact ttctttga 4518

<210> 18
<211> 3125
<212> DNA
<213> Homo sapiens

<400> 18
ccgcaaccag agccgccgcc acggtgagtg gctggattca gacccctggg tggccgggac 60

aagagaaaag agggaggagg gcctttagcg gacagcgcct ggggctggag agcagcagct 120

gcacacagcc ggaaagggcg cgcaggcgac gacactcgga tccacgtcga caccgttgta 180

caaagatacg cggacccgcg ggcgtctaaa attctgggaa gcagaacctg gccggagcca 240

ctagacagag ccgggcctag cccagagaca tggagagttg ctacaaccca ggtctggatg 300

gtattattga atatgatgat ttcaaattga actcctccat tgtggaaccc aaggagccag 360

ccccagaaac agctgatggc ccctacctgg tgatcgtgga acagcctaag cagagaggct 420

tccgatttcg atatggctgt gaaggcccct cccatggagg actgcccggt gcctccagtg 480

agaagggccg aaagacctat cccactgtca agatctgtaa ctacgaggga ccagccaaga 540

tcgaggtgga cctggtaaca cacagtgacc cacctcgtgc tcatgcccac agtctggtgg 600

gcaagcaatg ctcggagctg ggatctgcg ccgtttctgt ggggcccaag gacatgactg 660

cccaatttaa caacctgggt gtcctgcatg tgactaagaa gaacatgatg ggactatga 720

tacaaaaact tcagaggcag cggctccgct ctaggcccca gggccttacg gaggccgagc 780

agcgggagct ggagcaagag gccaaagaac tgaagaaggt gatggatctg agtatagtgc 840

ggctgcgctt ctctgccttc cttagagcca gtgatggctc cttctccctg cccctgaagc 900

cagtcatctc ccagcccatc catgacagca aatctccggg ggcatcaaac ctgaagattt 960

ctcgaatgga caagacagca ggctctgtgc ggggtggaga tgaagtttat ctgctttgtg 1020
acaaggtgca gaaagatgac attgaggttc ggttctatga ggatgatgag aatggatggc 1080
aggcctttgg ggacttctct cccacagatg tgcataaaca gtatgccatt gtgttccgga 1140
caccccccta tcacaagatg aagattgagc ggcctgtaac agtgtttctg caactgaaac 1200
gcaagcgagg aggggacgtg tctgattcca aacagttcac ctattaccct ctggtggaag 1260
acaaggaaga ggtgcagcgg aagcggagga aggccttgcc caccttctcc cagcccttcg 1320
ggggtggctc ccacatgggt ggaggctctg gggtgcagc cgggggctac ggaggagctg 1380
gaggaggtgg cagcctcggt ttcttcccct cctccctggc ctacagcccc taccagtccg 1440
gcgcgggccc catgggctgc tacccgggag gcgggggcgg ggcgcagatg gccgccacgg 1500
tgcccagcag ggactccggg gaggaagccg cggagccgag cgccccctcc aggacccccc 1560
agtgcgagcc gcaggccccg gagatgctgc agcgagctcg agagtacaac gcgcgcctgt 1620
tcggcctggc gcagcgcagc gcccgagccc tactcgacta cggcgtcacc gcggacgcgc 1680
gcgcgctgct ggcgggacag cgccacctgc tgacggcgca ggacgagaac ggagacacac 1740
cactgcacct agccatcatc cacgggcaga ccagtgtcat tgagcagata gtctatgtca 1800
tccaccacgc ccaggacctc ggcgttgtca acctcaccaa ccacctgcac cagacgcccc 1860
tgcacctggc ggtgatcacg gggcagacga gtgtggtgag ctttctgctg cgggtaggtg 1920
cagacccagc tctgctggat cggcatggag actcagccat gcatctggcg ctgcgggcag 1980
gcgctggtgc tcctgagctg ctgcgtgcac tgcttcagag tggagctcct gctgtgcccc 2040
agctgttgca tatgcctgac tttgagggac tgtatccagt acacctggcg gtccgagccc 2100
gaagccctga gtgcctggat ctgctggtgg acagtggggc tgaagtggag gccacagagc 2160
ggcagggggg acgaacagcc ttgcatctag ccacagagat ggaggagctg gggttggtca 2220
cccatctggt caccaagctc cgggccaacg tgaacgctcg cacctttgcg ggaaacacac 2280
ccctgcacct ggcagctgga ctggggtacc cgaccctcac ccgcctcctt ctgaaggctg 2340
gtgctgacat ccatgctgaa aacgaggagc ccctgtgccc actgccttca ccccctacct 2400
ctgatagcga ctcggactct gaagggcctg agaaggacac ccgaagcagc ttccggggcc 2460
acacgcctct tgacctcact tgcagcacca aggtgaagac cttgctgcta aatgctgctc 2520
agaacaccat ggagccaccc ctgaccccgc ccagcccagc agggccggga ctgtcacttg 2580
gtgatacagc tctgcagaac ctggagcagc tgctagacgg gccagaagcc cagggcagct 2640
gggcagagct ggcagagcgt ctggggctgc gcagcctggt agacacgtac cgacagacaa 2700
cctcacccag tggcagcctc ctgcgcagct acgagctggc tggcggggac ctggcaggtc 2760
tactggaggc cctgtctgac atgggcctag aggagggagt gaggctgctg aggggtccag 2820

aaacccgaga caagctgccc agcacagcag aggtgaagga agacagtgcg tacgggagcc 2880

agtcagtgga gcaggaggca gagaagctgg gcccaccccc tgagccacca ggagggctct 2940

gccacgggca cccccagcct caggtgcact gacctgctgc ctgcccccag cccccttccc 3000

ggacccccctg tacagcgtcc ccacctattt caaatcttat ttaacacccc acacccaccc 3060

ctcagttggg acaaataaag gattctcatg ggaaggggag gacccctcct tcccaactta 3120

tggca 3125

<210> 19
<211> 2529
<212> DNA
<213> Homo sapiens

<400> 19
gctgatagca cagttctgtc cagagaagga aggcagaata aacttattca ttcccaggaa 60

ctcttggggt aggtgtgtgt ttttcacatc ttaaaggctc acagaccctg cgctggacaa 120

atgttccatt cctgaaggac ctctccagaa tccggattgc tgaatcttcc ctgttgccta 180

gaagggctcc aaaccacctc ttgacaatgg gaaactgggt ggttaaccac tggttttcag 240

ttttgtttct ggttgtttgg ttagggctga atgttttcct gtttgtggat gccttcctga 300

aatatgagaa ggccgacaaa tactactaca caagaaaaat ccttgggtca acattggcct 360

gtgcccgagc gtctgctctc tgcttgaatt ttaacagcac gctgatcctg cttcctgtgt 420

gtcgcaatct gctgtccttc ctgaggggca cctgctcatt ttgcagccgc acactgagaa 480

agcaattgga tcacaacctc accttccaca agctggtggc ctatatgatc tgcctacata 540

cagctattca catcattgca cacctgttta actttgactg ctatagcaga agccgacagg 600

ccacagatgg ctcccttgcc tccattctct ccagcctatc tcatgatgag aaaaaggggg 660

gttcttggct aaatcccatc cagtcccgaa acacgacagt ggagtatgtg acattcacca 720

gcattgctgg tctcactgga gtgatcatga caatagcctt gattctcatg gtaacttcag 780

ctactgagtt catccggagg agttattttg aagtcttctg gtatactcac cacctttttta 840

tcttctatat ccttggctta gggattcacg gcattggtgg aattgtccgg ggtcaaacag 900

aggagagcat gaatgagagt catcctcgca agtgtgcaga gtcttttgag atgtgggatg 960

atcgtgactc ccactgtagg cgccctaagt ttgaagggca tccccctgag tcttggaagt 1020

ggatccttgc accggtcatt ctttatatct gtgaaaggat cctccggttt taccgctccc 1080

agcagaaggt tgtgattacc aaggttgtta tgcacccatc caaagttttg gaattgcaga 1140

tgaacaagcg tggcttcagc atggaagtgg ggcagtatat ctttgttaat tgcccctcaa 1200

tctctctcct ggaatggcat ccttttactt tgacctctgc tccagaggaa gatttcttct 1260

ccattcatat ccgagcagca ggggactgga cagaaaatct cataagggct ttcgaacaac 1320

aatattcacc aattcccagg attgaagtgg atggtccctt tggcacagcc agtgaggatg 1380

ttttccagta tgaagtggct gtgctggttg gagcaggaat tggggtcacc ccctttgctt 1440

ctatcttgaa atccatctgg tacaaattcc agtgtgcaga ccacaacctc aaaacaaaaa 1500

agatctattt ctactggatc tgcagggaga caggtgcctt ttcctggttc aacaacctgt 1560

tgacttccct ggaacaggag atggaggaat taggcaaagt gggttttcta aactaccgtc 1620

tcttcctcac cggatgggac agcaatattg ttggtcatgc agcattaaac tttgacaagg 1680

ccactgacat cgtgacaggt ctgaaacaga aaacctcctt tgggagacca atgtgggaca 1740

atgagttttc tacaatagct acctcccacc ccaagtctgt agtgggagtt ttcttatgtg 1800

gccctcggac tttggcaaag agcctgcgca aatgctgtca ccgatattcc agtctggatc 1860

ctagaaaggt tcaattctac ttcaacaaag aaaatttttg agttatagga ataaggacgg 1920

taatctgcat tttgtctctt tgtatcttca gtaatttact tggtctcgtc aggtttgagc 1980

agtcacttta ggataagaat gtgcctctca agccttgact ccctggtatt ctttttttga 2040

ttgcattcaa cttcgttact tgagcttcag caacttaaga acttctgaag ttcttaaagt 2100

tctgaagttc ttaaagccca tggatccttt ctcagaaaaa taactgtaaa tctttctgga 2160

cagccatgac tgtagcaagg cttgatagca gaggtttggt ggttcagagt tatacaacta 2220

atcccaggtg attttatcaa ttccagtgtt accatctcct gagttttggt ttgtaatctt 2280

ttgtccctcc cacccccaca gaagatttct aagtagggtg actttttaaa taaaaattta 2340

ttgaataatt aatgataaaa cataataata aacataaata ataaacaaaa ttaccgagaa 2400

ccccatcccc atataacacc aacagtgtac atgtttactg tcacttttga tatggtctta 2460

tccagtgtga acagcaattt attcttattt ttgctcatca aaaaataaag gattttcttc 2520

ttcacttga 2529

<210> 20
<211> 2639
<212> DNA
<213> Homo sapiens

<400> 20
ggcgccttgg gaccgcgtgg gagccgcagc cgaaccgagt agggaccggg accgcgcggc 60

gccgccgtcc ccggccgggc ccggcccccg cgagccgagc gcgcgccccc gtcgcccacc 120

cgggcgcggc tggatgcggc ggggtccccg cggcggcgac ccccggcccc gagcgcccgg 180

agcgcccaga ggcggcgtgc ggggcccggg gacgccgcgc cctccatgcg ccgaggcgcg 240

ccccgagaca gccgggggcc cgcgccgcag ccgccgcccg cgctgagccc cggcccggcc 300

cgcggcccgc gcccggcggc agcatgagcc aggccgagct gtccacctgc tccgcgccgc 360

agacgcagcg catcttccag gaggctgtgc gcaagggcaa cacgcaggag ctgcagtcgc 420

```
tgctgcagaa catgaccaac tgcgagttca acgtgaactc gttcgggccc gagggccaga     480
cggcgctgca ccagtcggtc atcgacggca acctggagct cgtgaagctg ctggtcaagt     540
tcggcgccga catccgcctg gccaaccgcg acggctggag cgcgctgcac atcgccgcgt     600
tcggtggcca ccaggacatc gtgctctatc tcatcaccaa ggcgaagtac gcggccagcg     660
gccggtgatg cccgccggga ccccggaccc cggccctgcg cccgcgtcgt ctctgctgta     720
ccttcccgcc aactacctcg gtgcgcgccc ggctcgcagg ccccgccaga aggcccgtgg     780
ccacggcgaa tacggcgcgt gcgtcccggc cccagggtcc ggcagccccg ccggccgagc     840
gcctccctgc ggcctagccg ggcccggccg ggccggagca gcttcccacg gcccccaccc     900
gctcgcctgc ccgccgcctc gcgggtgggg gcggggcgcg ggctccagcc ccttttgaaa     960
tttgagtctc gcaaccagca agttcggaat cccgagatac cggatcctct gcgcaaaatg    1020
ttttctcccg aaggtgaaag gcgggcggcg ggagccgaag gcggactcgg agcgctccgc    1080
cgccgccttc aggacccgcc cgcaggcccg ggacgcgccg atgccggctg cagccgagga    1140
gcagccccga ggtccgaggt ccgcgccgct ggcgcgcggc cgaggagacg ctcggctgtt    1200
cgctgttgct ggtgttctaa actatttatc ttgtgtgtgt acatttgtgg gtggagtttg    1260
tgcgcctggt tttttttgttt ggaaaacact gcgtggtcaa tgtggttatg gggggggagtg    1320
atgcattttt ttctagtctt aaaactaaaa acttgagtct accatttctt ggttgcactg    1380
aaaataccgc ccagcctgat ggtgttcccg tgctgtccct ccccccttccc ttctccccgc    1440
gtctacctcc ccaccccgtt ctgttccccc tccctccttc tccctctccc tcaaatccgt    1500
gagttttgga agccccaggg cctctctccc ccgcccctcc tggatgaggc caccatcccc    1560
caaaccggct tgttttgcag tttccccagg atcctggaag ctcgctggcg ctcgagggtg    1620
gcggggacac ggggggggtgg gtgaaggttc gttacctttt ctagtgcgtt ctatcatagt    1680
taacggttgc acactttttt aaaaaaagta aatggatttg ccacaattaa atgtcataac    1740
atttatgaca gaatataaaa tattaacata ttttaagcca agttttaggt gtattttttg    1800
aatcttggtt ataaacccaa ttttaaaggg cgatgtatcc agcgttgtga aggcaacaga    1860
gtgtacccat atttatattt ttataaaata cctataagac tgtgaatctc ttgtgctaat    1920
ggctgagtta attgaaggat cgttttgccc ctttttagcc tcccagagct tcgaggactc    1980
aattcgaacc cgaaatcctg ccgtggggga ggggttgcgt cgagacctgg gcccggggag    2040
gttctcctgc gtcactttct gtcctgaaag gcgcccttcc tggtttctgt ggctccaatt    2100
ttctatgcag ccccacaccc cttgttgttt tgatcctgag aaataaaagg gaggctgaat    2160
tattcaaatt taaatgaggt ttccccttca tggaagtgct gctgaccctt cgtgcagaaa    2220
tggggagcac ttgaggacac aggtgggtgg aggccctttg tgcgtggctg gtcgtattcg    2280
ggcagccctc cgtcgctttt tataaaactt tgtgtgagaa gaatatattg ataatgtcag    2340
```

tgaaacaagc agacattgaa atggaggcac agattactcc acaaggagtt cttctgtata 2400

tttttttctag atgcaaatac ctttttaatt atgttaatta atgttaagac tttctaggct 2460

tatatcgaag ctgtgtgtgg gtcacggggt gatcactgct aactggataa agtttgtgca 2520

gcacattcct gagtgtacga tattgacctg tagcccagcg tgaaaaattt ataaataaat 2580

ttttcattga tctttttata ttaaaaaaaa gtttcttggt caaaaaaaaa aaaaaaaaa 2639

<210> 21
<211> 6918
<212> DNA
<213> Homo sapiens

<400> 21
aaagtttgca ttgcaatccc cctgccttcc tctcctttct cccgatcaat gcatatttgc 60

aaaaggatta agccacagat ttaagcgccg ggagcccatt tctgccttgc aaaggagacc 120

ggactgaaaa acctaaagcc agctctgatt tcttttcgcc aagtgggaag gtggtttatt 180

tttcttgctt tttggagtca acacccttcc ccaccagccc ttatccccac cctcaccccg 240

caaccccttc acgcccccctc cccctccccc tcctcatcct cccaccatcc tctaaagagg 300

caaagggatt tttttttttct tttggtcttc tttttcccc cttccctgtt tatcctgaaa 360

aggatttgaa gacaagcttg aaggataaaa agccttggtg cttcccagga gccgagccga 420

ggagcagaag aggaagagcc gggggctgcc gtagcctttg gagatggacg agcagcccag 480

gctgatgcat tcccatgctg ggtcgggat ggccggacac cccggcctgt cccagcactt 540

gcaggatggg gccggaggga ccgaggggga gggcgggagg aagcaggaca ttggagacat 600

tttacagcaa attatgacca tcacagacca gagtttggat gaggcgcagg ccagaaaaca 660

tgctttaaac tgccacagaa tgaagcctgc cttgtttaat gtgttgtgtg aaatcaaaga 720

aaaaacagtt ttgagtatcc gaggagccca ggaggaggaa cccacagacc cccagctgat 780

gcggctggac aacatgctgt tagcggaagg cgtggcgggg cctgagaagg gcggagggtc 840

ggcggcagcg gcggcagcgg cggcggcttc tggaggggca ggttcagaca actcagtgga 900

gcattcagat tacagagcca aactctcaca gatcagacaa atctaccata cggagctgga 960

gaaatacgag caggcctgca acgagttcac cacccacgtg atgaatctcc tgcgagagca 1020

aagccggacc aggcccatct ccccaaagga gattgagcgg atggtcagca tcatccaccg 1080

caagttcagc tccatccaga tgcagctcaa gcagagcacg tgcgaggcgg tgatgatcct 1140

gcgttcccga tttctggatg cgcggcggaa gagacggaat ttcaacaagc aagcgacaga 1200

aatcctgaat gaatatttct attcccatct cagcaaccct taccccagtg aggaagccaa 1260

agaggagtta gccaagaagt gtggcatcac agtctcccag gtatcaaact ggtttggaaa 1320

taagcgaatc cggtacaaga agaacatagg taaatttcaa gaggaagcca atatttatgc 1380

tgccaaaaca gctgtcactg ctaccaatgt gtcagcccat ggaagccaag ctaactcgcc 1440

ctcaactccc aactcggctg gttcttccag ttcttttaac atgtcaaact ctggagattt 1500

gttcatgagc gtgcagtcac tcaatgggga ttcttaccaa ggggcccagg ttggagccaa 1560

cgtgcaatca caggtggata cccttcgcca tgttatcagc cagacaggag gatacagtga 1620

tggactcgca gccagtcaga tgtacagtcc gcagggcatc agtgctaatg gaggttggca 1680

ggatgctact accccttcat cagtgacctc ccctacagaa ggccctggca gtgttcactc 1740

tgatacctcc aactgatctc ccagcaatcg catcccggct gaccctgtgc cccagttggg 1800

gcaggggcag gagggagggt ttctctccca acgctgaagc ggtcagactg gaggtcgaag 1860

caatcagcaa acacaataag agtctccttc tcttctcttc tttgggatgc tatttcagcc 1920

aatctggaca cttctttata ctctcttccc tttttttct gggtagaagc caccccttccc 1980

tgcctccagc tgtcagcctg gttttcgtca tcttccctgc ccctgtgcct ctgtcctaga 2040

ctcccggggt ccccgccctc tctcatatca ctgaaggata ttttcaacaa ttagaggaat 2100

ttaaagagga aaaaaattac aaagaaaata ataaaagtgt ttgtacgttt tcatgctggt 2160

ggtttgagga gccaaattta cctcactcga atccctcact ccctatgtta acaggcaatc 2220

cttctctgtt tctcttatta ctctcactac ctcttagcag gaatactcca cattgcccta 2280

ttcattccag gcctccctgc ttcctcttgc tcttcctccc tggggacagt actgattgga 2340

acactttcct cctcttcctt cctagcccca gctattcact ggggactgtc atagctggga 2400

ttctaaaggt gccacatttt tcagtttcat ctccactagg ttggttcccg ggcaggaagt 2460

caggcagcag ggaaggacac gggaacagca ggtggagaat tcctacagtc tttcttaccc 2520

tgctagcaat agctctcagt ttcagaggca cagtctttgg agaccattca gcactgagaa 2580

agcaatattt agaacctatt gcaaaactgg gcctgagtta ggcatggtga tgaatgcatc 2640

agcaaggaat agaaagttct tatcgtgaaa cccttcaacc tcaactatgc cttcatagac 2700

acacacgttc atgcacatgt aggcacatgt accatctcac atcttcactt tcccgagatg 2760

ccatatacaa ttacctacat taataactgt agcactatgc cttttgagcc cgagagaggg 2820

aattagtgac tctaagtgaa ggtcactgac acagagaagc agtatgtgtc tggggcttcc 2880

aggacctgca ggcccactag cgtgcactta ccagaatggc atacacagga cctgatcatg 2940

aggaagacca ggtttccagt gtaaactact cttgttccca ccacctctgg agcactcagg 3000

gagccccata cagtacttac aatgtcttta atggacttga ttctgtttaa ttttttgttt 3060

tatattaggc acactgtatt aattttccaa aatgttatac cacactatgt tcttggtcct 3120

gacctattgc tctggaggaa agagttgtat aagaacgtgg ctcatgtgaa cttttgctag 3180

cttcatttga ggacctgaga atcatgggga aagggaaggt aatgttttca ttgaaatcat 3240

cacagtgatt tttattccct gggaacacag cgtgtactaa aaatacatga gaaaatagca 3300

tgtatatgaa agctattctc aaaagtcacc tgagctcacc atcttcatag ccaaccctac 3360

cagttataaa gatggcagct ctatcacttg attaagtggg aggtggtcaa atattttggt 3420

gcctcatttt cttcatctgt gagatgggaa ctgttatgcc tggcttacta agagtcttgt 3480

gagagactga gaagttgatt ttgttcatat ccaatctgta aatgcgaagt caggggaagt 3540

aatgtccctg aaataaacgg gttcatgcca tctagggaca ataaatggtt ttcttgttgt 3600

aacttctggt taatatcagt accttgatgt catcaccgtg atgacaaaga gaagagttat 3660

tgttgatctt cttggttttg gtctgtctct tttcttagga taaagaaaaa cttccaaact 3720

agaaaaacag gccctggttc ccttagtttg cacttgaacc caatatgttg ccttgtacat 3780

acttggtccc tgtcacattg actgcttggg aggcttccag ggagaagtat gagaccctga 3840

ggggtgagaa tgggcagcta gcaagaacat ggaaattctg cttggcacta cagtcataaa 3900

tagaaaacac tgtgtgtgct caggggagca ggggatgcca ctgaagaaac tcaagggaat 3960

gtgtatttga aggaaatgca aaaactaagt atttagcaaa atgaaattat gccttgatga 4020

ctaaaaggca ctagaaaggt tgtgtctact aacttcagcc ctaatcagaa cagatgccta 4080

gaaggagcat ttttgtgaca acttcatagt gattagaatc agtggagaac tccatcttag 4140

tggcaggaat ataatgaaac tacccacgca agaacatggt tgaatcacat ttgcttgact 4200

tagggcaaag tacgaaagag agacaaaagg gttctcttgg aaacaagaag agtgactcca 4260

gatgtggcct gaataattgc catgttaagt taatgcaaaa gatcagaaca gggctacatt 4320

tgcacaggca gtttctctcc gggccgtagt tttcactgat gatcaccttt cacagcattt 4380

tccccaacca gcatttcact tagtcttctc tatacccagc acctcccccg gcacccccgg 4440

caagcccact atcacttccg acttccaacg tggcatccgt gagatctgtc cacattaggc 4500

gaagcaggag aacactgaga gcagcaggat gggtttggaa agagcatgcc tctggaaaca 4560

cagcttcctg ggaattcaca tgaggccagt cctacagaga gcaagatgca ccccaggatt 4620

tcttcatttt ctaatagatg tgggagtgct ccattttccc cgacagcgaa tttcccctga 4680

gaaacgatac tagaccctgg gtttgcccac cttgtaactc ttccttatct cctccttttc 4740

atccctaatc catcctccct ctggcatgga attgacgccc gtgcagtaca tttgccaagt 4800

ggcaccttct ttcaatttat gttttatttt gctatggtgg tgattcttta tttgctggtt 4860

gtcttttctc acacatcttt ctctctgtct ctctctttcc tgctctttgt ttttctgccc 4920

agaaaaacct gacttcgata ccaaaaaaga tgaaactaca gaaactcaaa tttaaaaaaa 4980

actttaaaag aaacaaaaaa atactcaacg attctttcag ctttattaac attttccatt 5040

gtttcttgcg acttgtgtct cgttctttgt agtattgatg atgaacattt gataatgaat 5100

gttcttgtat attcagataa agaaaaaaaa aaccaaaaaa gcggtctgaa tttaatagtg 5160

tttataataa aaattttaaa aatgaccctc atagcacgca aaacaggatg gggaatttcc 5220

cctcttcttt ctgtgacaat gcgcatcatt cctgcattag tttttaacac cagactacct 5280

acattcatca tttccctcat ttttctttta ttttcttgca tttgtgaatt agttcaagaa 5340

tgctagaaaa gtgtcgagtt gtgcacatcc atttcttgtt tcacaatgtt taaaagtgac 5400

agtaattcat tttgtaaact aaaaaaaaaa aaaaaaaggt tggaatagtg agcataatag 5460

gtacaaccta acacattatt atgtttatta actttgagac ccagaaataa attcttttct 5520

tttcttgatt cttgctctta aaaatacaaa aaaaaaaatg ttttgttttg tgttattttt 5580

ggtttgttta ttgggggggct ttttttaatt gtcaggatta tgatcttgct gtttttcttc 5640

aatatgtata caaggtgatg tgaaaagatg acttgggcag aggagtaaga acaagtaggc 5700

ttgttcttct actttgcttc agaattcagt taatgccaaa agcgaagatc aagcccatgt 5760

tgatgtctcg ttgctcacct gcatttccag agagtgtgac actcatgcag tccctgagaa 5820

aaataaaatc agggacatac ttctcctttt agccttttaa aaattcaaaa acgtttagtc 5880

caagggaact ttttatgcta tcaggaaagg tttttgctgt ttttgattct gattatcaca 5940

gccaagtact ttgttttatt tctccctaat taataactac attccatgag gcctcttcca 6000

accaaagagg ccttttcttc caggagagtc ccgcaggaga tgctggtatg atgggcacca 6060

ttggttaagt aaactacatg caggaagaag tccttggggc cagtctgcca gctgagtcct 6120

ggttttggat gaagagttaa tgagatattg ggccaggctc aatgctgtag ttttaatgct 6180

aagaggttac gtttacttca cagagtacac ctcttagtaa cctctgactt aggcagctgc 6240

ttaaagcaaa ttgcaaaact ggcttgattt ggaatgtttt tattagagga aaaaagaaag 6300

ccatattatc tggaaaaaaa ttcattttaa ataccatcat tcaacaaatt atgttcagaa 6360

agtggtcaga acttaagcaa gaaaagtaaa gaaagaatgc agaattgtgg agcaatgctt 6420

taggaaatat ttctacctga acacttgtac tcttgaagtc acaacaaaat aatgatgagc 6480

ttttcacatc acctttatgg tttcaatccc tagctcaaag cttcctggaa tcttttattt 6540

tttgtaaact tttttttctt ttgttaaaat aaataaaaca ttcaatgttt ttctcctttt 6600

ctctcttatt acttctttcc tttggcattt tcaatttgaa atgctttcct ttggttgttg 6660

gttttattct ccccctaccc ctcccctttt cttattattc agaatataaa cctgcaaagc 6720

tctgctctgt tttggttttg aaagtttaag cttttctgct tctgtgagag cacaggcttc 6780

tgtccctttt gattccaact gaacttttgt gttctctaat gatactaaca cggtgtaggt 6840

tttacagtct cctaatttgt actggtaatg catattccaa ataaatagtt tcttttgttg 6900

caaaaaaaaa aaaaaaaa 6918

<210> 22

<211> 1138
<212> DNA
<213> Homo sapiens

<400> 22
ggaccgttag ggagcccaat gggcgtcgcc gccaggcccc gttgcagagc gcgtctagcc 60

aataggcagc ggcggcgggc gggcgcgggc gacaggcggc gcagctgagg cggagcaggc 120

gctgcggcag gagggaagat ggcggacgag gagaagctgc cgcccggctg ggagaagcgc 180

atgagccgca gctcaggccg agtgtactac ttcaaccaca tcactaacgc cagccagtgg 240

gagcggccca gcggcaacag cagcagtggt ggcaaaaacg ggcaggggga gcctgccagg 300

gtccgctgct cgcacctgct ggtgaagcac agccagtcac ggcggccctc gtcctggcgg 360

caggagaaga tcacccggac caaggaggag gccctggagc tgatcaacgg ctacatccag 420

aagatcaagt cgggagagga ggactttgag tctctggcct cacagttcag cgactgcagc 480

tcagccaagg ccaggggaga cctgggtgcc ttcagcagag gtcagatgca gaagccattt 540

gaagacgcct cgtttgcgct gcggacgggg gagatgagcg ggcccgtgtt cacggattcc 600

ggcatccaca tcatcctccg cactgagtga gggtggggag cccaggcctg gcctcggggc 660

agggcagggc ggctaggccg gccagctccc ccttgcccgc cagccagtgg ccgaaccccc 720

cactccctgc caccgtcaca cagtatttat tgttcccaca atggctggga gggggccctt 780

ccagattggg ggccctgggg tccccactcc ctgtccatcc ccagttgggg ctgcgaccgc 840

cagattctcc cttaaggaat tgacttcagc aggggtggga ggctcccaga cccagggcag 900

tgtggtggga ggggtgttcc aaagagaagg cctggtcagc agagccgccc cgtgtccccc 960

caggtgctgg aggcagactc gagggccgaa ttgtttctag ttaggccacg ctcctctgtt 1020

cagtcgcaaa ggtgaacact catgcggccc agccatgggc cctctgagca actgtgcagc 1080

accctttcac ccccaattaa acccagaacc actgctctgc aaaaaaaaaa aaaaaaa 1138

<210> 23
<211> 6977
<212> DNA
<213> Homo sapiens

<400> 23
cccgggcccg ccccccgcct cccgccgcct ccgggctccc ggctcccggc cgcgcctcgc 60

cccatgcact cgccgcgccg cgcagcccgc gcacgcccgg atggctcctc gcgccgcggg 120

cggcgcaccc cttagcgccc gggccgccgc cgccagcccc ccgccgttcc agacgccgcc 180

gcggtgcccg gtgccgctgc tgttgctgct gctcctgggg gcggcgcggg ccggcgccct 240

ggagatccag cgtcggttcc cctcgcccac gcccaccaac aacttcgccc tggacggcgc 300

ggcggggacc gtgtacctgg cggccgtcaa ccgcctctat cagctgtcgg gcgccaacct 360

gagcctggag gccgaggcgg ccgtgggccc ggtgcccgac agcccgctgt gtcacgctcc 420

gcagctgccg caggcctcgt gcgagcaccc gcggcgcctc acggacaact acaacaagat 480
cctgcagctg gaccccggcc agggcctggt agtcgtgtgc gggtccatct accagggctt 540
ctgccagctg cggcgccggg gcaacatctc ggccgtggcc gtgcgcttcc cgcccgccgc 600
gccgcccgcc gagcccgtca cggtgttccc cagcatgctg aacgtggcgg ccaaccaccc 660
gaacgcgtcc accgtggggc tagttctgcc tcccgccgcg ggcgcggggg gcagccgcct 720
gctcgtgggc gccacgtaca ccggttacgg cagctccttc ttcccgcgca accgcagcct 780
ggaggaccac cgcttcgaga acacgcccga gatcgccatc cgctccctgg acacgcgcgg 840
cgacctggcc aagctcttca ccttcgacct caacccctcc gacgacaaca tcctcaagat 900
caagcagggc gccaaggagc agcacaagct gggcttcgtg agcgccttcc tgcacccgtc 960
cgacccgccg ccgggtgcac agtcctacgc gtacctggcg ctcaacagcg aggcgcgcgc 1020
gggcgacaag gagagccagg cgcggagcct gctggcgcgc atctgcctgc cccacggcgc 1080
cggcggcgac gccaagaagc tcaccgagtc ctacatccag ttgggcttgc agtgcgcggg 1140
cggcgcgggc cgcggcgacc tctacagccg cctggtgtcg gtcttcccag cccgggagcg 1200
gctctttgct gtcttcgagc ggccccaggg gtcccccgcg gcccgcgctg ctccggccgc 1260
actctgcgcc ttccgcttcg ccgacgtgcg agccgccatc cgagctgcgc gcaccgcctg 1320
cttcgtggaa ccggcgcccg acgtggtggc ggtgctcgac agcgtggtgc agggcacggg 1380
accggcctgc gagcgcaagc tcaacatcca gctccagcca gagcagctgg actgtggagc 1440
tgctcacctg cagcacccgc tgtccatcct gcagcccctg aaggccacgc ccgtgttccg 1500
cgcccccggc ctcacctccg tggccgtggc cagcgtcaac aactacacag cggtcttcct 1560
gggcacggtc aacgggaggc ttctcaagat caacctgaac gagagcatgc aggtggtgag 1620
caggcgggtg gtgactgtgg cctatgggga gcccgtgcac catgtcatgc agtttgaccc 1680
agcagactcc ggttaccttt acctgatgac gtcccaccag atggccaggg tgaaggtcgc 1740
cgcctgcaac gtgcactcca cctgtgggga ctgcgtgggt gcggcggacg cctactgcgg 1800
ctggtgtgcc ctggagacgc ggtgcacctt gcagcaggac tgcaccaatt ccagccagca 1860
gcatttctgg accagtgcca gcgagggccc cagccgctgt cctgccatga ccgtcctgcc 1920
ttccgagatc gatgtgcgcc aggagtaccc aggcatgatc ctgcagatct cgggcagcct 1980
gcccagcctc agtggcatgg agatggcctg tgactatggg aacaacatcc gcactgtggc 2040
tcgggtccca ggccctgcct ttggtcacca gattgcctac tgcaacctcc tgccgaggga 2100
ccagtttccg cccttccccc ccaaccagga ccacgtgact gttgagatgt ctgtgagggt 2160
caatgggcgg aacatcgtca aggccaattt caccatctac gactgcagcc gcactgcaca 2220
agtgtacccc cacacagcct gtaccagctg cctgtcggca cagtggccct gtttctggtg 2280

cagccagcag cactcctgtg tttccaacca gtctcggtgc gaggcctcac caaaccccac 2340
gagccctcag gactgccccc ggaccctgct ctcacccctg gcacccgtgc ctacgggtgg 2400
ctcccagaac atcctggtgc ctctggccaa cactgccttt ttccagggtg cagccctgga 2460
gtgtagtttt gggctggagg agatcttcga ggctgtgtgg gtgaatgagt ctgttgtacg 2520
ctgtgaccag gtggtgctgc acacgacccg gaagagccag gtgttcccgc tcagcctcca 2580
actaaagggg cggccagccc gattcctgga cagccctgag cccatgacag tcatggtcta 2640
taactgtgcc atgggcagcc ccgactgttc ccagtgcctg ggccgcgaag acctgggtca 2700
cctgtgcatg tggagtgatg gctgccgcct gcgggggcct ctgcagccca tggctggcac 2760
ctgccccgcc cccgagatcc gcgcgattga gcccctgagt ggcccgttgg acggtgggac 2820
cctgctgacc atccgaggaa ggaacctggg ccggcggctc agtgacgtgg cccacggcgt 2880
gtggattggt ggtgtggcct gtgagccact gcctgacaga tacacggtgt cggaggagat 2940
cgtgtgtgtc acagggccag ccccaggacc actctcaggt gtggtgaccg tgaacgcctc 3000
taaggagggc aagtcccggg accgcttctc ctacgtgctg cccctggtcc actccctgga 3060
gcctaccatg ggccccaagg ccgggggcac caggatcacc atccatggga atgacctcca 3120
tgtaggctcc gagctccagg tcctggtgaa cgacacagac ccctgcacgg agctgatgcg 3180
cacagatacc agcatcgcct gcaccatgcc tgaggggggcc ctgccggctc cggtgcctgt 3240
gtgtgtgcgc ttcgagcgtc ggggctgcgt gcacggcaac ctcaccttct ggtacatgca 3300
gaacccggtc atcacggcca tcagtccccg ccgcagccct gtcagtggcg gcaggaccat 3360
cacagtggct ggtgagcgtt tccacatggt gcagaatgtg tccatggccg tccaccacat 3420
tggccgggag cccacgctct gcaaggttct caactccacc ctcatcacct gcccgtcccc 3480
cggggccctg agcaacgcat cagcgccagt ggacttcttc atcaatgggc gggcctacgc 3540
agacgaggtg gctgtggctg aggagctact ggaccccgag gaggcacagc ggggcagcag 3600
gttccgcctg gactacctcc ccaacccaca gttctctacg gccaagaggg agaagtggat 3660
caagcaccac cccggggagc ctctcaccct cgttatccac aaggagcagg acagcctggg 3720
gctccagagt cacgagtacc gggtcaagat aggccaagta agctgcgaca tccagattgt 3780
ctctgacaga atcatccact gctcggtcaa cgagtccctg ggcgcggccg tggggcagct 3840
gcccatcaca atccaggtag ggaacttcaa ccagaccatc gccacactgc agctgggggg 3900
cagcgagacg gccatcatcg tgtccatcgt catctgcagc gtcctgctgc tgctctccgt 3960
ggtggccctg ttcgtcttct gtaccaagag ccgacgtgct gagcgttact ggcagaagac 4020
gctgctgcag atggaggaga tggaatctca gatccgagag gaaatccgca aaggcttcgc 4080
tgagctgcag acagacatga cagatctcac caaggagctg aaccgcagcc agggcatccc 4140
cttcctggag tataagcact tcgtgacccg caccttcttc cccaagtgtt cctcccttta 4200

tgaagagcgt tacgtgctgc cctcccagac cctcaactcc cagggcagct cccaggcaca 4260

ggaaacccac ccactgctgg gagagtggaa gattcctgag agctgccggc ccaacatgga 4320

agagggaatt agcttgttct cctcactact caacaacaag cacttcctca tcgtctttgt 4380

ccacgcgctg gagcagcaga aggactttgc ggtgcgcgac aggtgcagcc tggcctcgct 4440

gctgaccatc gcgctgcacg gcaagctgga gtactacacc agcatcatga aggagctgct 4500

ggtggacctc attgacgcct cggccgccaa gaaccccaag ctcatgctgc ggcgcacaga 4560

gtctgtggtg gagaagatgc tcaccaactg gatgtccatc tgcatgtaca gctgtctgcg 4620

ggagacggtg ggggagccat tcttcctgct gctgtgtgcc atcaagcagc aaatcaacaa 4680

gggctccatc gacgccatca caggcaaggc ccgctacaca ctcaatgagg agtggctgct 4740

gcgggagaac atcgaggcca agccccggaa cctgaacgtg tccttccagg gctgtggcat 4800

ggactcgctg agcgtgcggg ccatggacac cgacacgctg acacaggtca aggagaagat 4860

cctggaggcc ttctgcaaga atgtgcccta ctcccagtgg ccgcgtgcag aggacgtcga 4920

ccttgagtgg ttcgcctcca gcacacagag ctacatcctt cgggacctgg acgacacctc 4980

agtggtggaa gacggccgca agaagcttaa cacgctggcc cattacaaga tccctgaagg 5040

tgcctccctg gccatgagtc tcatagacaa gaaggacaac acactgggcc gagtgaaaga 5100

cttggacaca gagaagtatt tccatttggt gctgcctacg gacgagctgg cggagcccaa 5160

gaagtctcac cggcagagcc atcgcaagaa ggtgctcccg gaaatctacc tgacccgcct 5220

gctctccacc aagggcacgt tgcagaagtt tctggatgac ctgttcaagg ccattctgag 5280

tatccgtgaa gacaagcccc cactggctgt caagtacttt ttcgacttcc tggaggagca 5340

ggctgagaag aggggaatct ccgaccccga caccctacac atctggaaga ccaacagcct 5400

tcctctccgg ttctgggtga acatcctgaa gaacccccag tttgtctttg acatcgacaa 5460

gacagaccac atcgacgcct gcctttcagt catcgcgcag gccttcatcg acgcctgctc 5520

catctctgac ctgcagctgg gcaaggattc gccaaccaac aagctcctct acgccaagga 5580

gattcctgag taccggaaga tcgtgcagcg ctactacaag cagatccagg acatgacgcc 5640

gctcagcgag caagagatga atgcccatct ggccgaggag tcgaggaaat accagaatga 5700

gttcaacacc aatgtggcca tggcagagat ttataagtac gccaagaggt atcggccgca 5760

gatcatggcc gcgctggagg ccaaccccac ggcccggagg acacaactgc agcacaagtt 5820

tgagcaggtg gtggctttga tggaggacaa catctacgag tgctacagtg aggcctgaga 5880

cacatggaga gttggtcagg ctgctgctgg gagaaatgga cgcccactgg gcctcaactt 5940

gatcttctac cccgtgcctg tgactcagac tgggaaatac tgagcagaga cggctggggc 6000

gggggcagga ggaggggctg ctctctgaga caggggcgcc cccgccttga cccctgggca 6060

cctccatccc ctcccacctg tccccagatc agtctctggg atggaggcca gagagctggt 6120

caggctcccc catctgccca gcacggcctg cactgtgccc acccacttgc tccacaacgt 6180

ccagttggtc ctgctgccaa gagccccgtg catccaggcg gccaagcaca aactggggga 6240

gaggaggccg ccagcccgga ggctgcagcc cagaaactct acctcatcca cactggtgca 6300

gggagccctc cttgaactga cctttgattg gtttctgctt caactaccaa aatgttatct 6360

ccacttcccc ctcacccgta gaggatcctg gccacagaca gtttcaagta gtgtcagatt 6420

tttgttgctt gggcggctgt tggtagagtg ggcagtgccc gcgccatggg gtgctctgtg 6480

ggcttctcca ggagcaggga gggtggaggg gagggatggg gggcacagga gctgggagcc 6540

ccgtctccag gaaaaggaga ggggttaaga tgcaccgagg ctgtagctgg gctacttgat 6600

cttgctgaaa gtgtttctaa agatagcacc actttttttt ttaaagcttt tatatattaa 6660

aaaacgtatc atgcaccaac tgtgaatagc tgccgcttgc gcagaggacc cggggagggg 6720

tcccgagagg ctccccatgc aacactggaa atgactgttc cagagagcgg gcagacctgg 6780

cagagcgccc ctggcgcctg agactaccac ccactccgtt cctgccagaa acgaccctct 6840

gtggccgatg ggccatgcgg gcccctcgca gccaactcag ccagtgttgg gactggctca 6900

gagcccatgg gggctggagg ggggcagctg ggactctgga atcttcttta taataaaagc 6960

cttacggaca aacctac 6977

<210> 24
<211> 1097
<212> DNA
<213> Homo sapiens

<400> 24
tagaaggcag tcttgtgggt gcctcctccc ccagccgcaa ctcaggtctg cagctgggtc 60

ctgcctcctt ccgagtgggc catggccggt acatggctgc tacttctcct ggcccttggg 120

tgtccagccc tacccacagg tgtgggcggc acaccctttc cttctctggc cccaccaatc 180

atgctgctgg tggatggaaa gcagcagatg gtggtggtct gcctggtcct tgatgttgca 240

ccccctggcc ttgacagccc catctggttc tcagccggca atggcagtgc actggatgcc 300

ttcacctatg gcccttcccc agcaacggat ggcacctgga ccaacttggc ccatctctcc 360

ctgccttctg aggagctggc atcctgggag cctttggtct gccacactgg gcctggggct 420

gagggtcaca gcaggagtac acagcccatg catctgtcag gagaggcttc tacagccagg 480

acctgccccc aggagcctct caggggggaca ccgggtgggg cgctgtggct gggggtcctg 540

cggctgctgc tcttcaagct gctgctgttt gacctgctcc tgacctgcag ctgcctgtgc 600

gaccccgcgg gcccgctgcc ttcccccgca accaccaccc gcctgcgagc cctcggctcc 660

catcgactgc acccggccac ggagactggg ggacgagagg ccaccagctc acccagaccc 720

cagcctcggg accgccgctg gggtgacacc cctccgggtc ggaagcccgg gagcccagta 780

tgggggggaag ggtcttacct cagcagttac cccacttgcc cagcacaggc ctggtgctca 840

agatctgccc tcagggctcc ttcctccagt cttggagcat tttttgcagg tgacctgcct 900

cctcctctgc aggctggagc tgcctgaggg cagggctcta cctcccctgc gtcacactgt 960

gtgaggctgt gtctctgcca tccaaaaggg ggccccttga gaatggtgat ccacccagtt 1020

acaggggcat ttagggagca gatgactgag aacattaaaa aagaacttaa atgacacagc 1080

aaaaaaaaaa aaaaaaa 1097

<210> 25
<211> 3963
<212> DNA
<213> Homo sapiens

<400> 25
ggagagccga aagcggagct cgaaactgac tggaaacttc agtggcgcgg agactcgcca 60

gtttcaaccc cggaaacttt tctttgcagg aggagaagag aaggggtgca agcgccccca 120

cttttgctct ttttcctccc ctcctcctcc tctccaattc gcctcccccc acttggagcg 180

ggcagctgtg aactggccac cccgcgcctt cctaagtgct cgccgcggta gccggccgac 240

gcgccagctt ccccgggagc cgcttgctcc gcatccgggc agccgagggg agaggagccc 300

gcgcctcgag tccccgagcc gccgcggctt ctcgcctttc ccggccacca gccccctgcc 360

ccgggcccgc gtatgaatct cctggacccc ttcatgaaga tgaccgacga gcaggagaag 420

ggcctgtccg gcgcccccag ccccaccatg tccgaggact ccgcgggctc gccctgcccg 480

tcgggctccg gctcggacac cgagaacacg cggccccagg agaacacgtt ccccaagggc 540

gagcccgatc tgaagaagga gagcgaggag gacaagttcc ccgtgtgcat ccgcgaggcg 600

gtcagccagg tgctcaaagg ctacgactgg acgctggtgc ccatgccggt gcgcgtcaac 660

ggctccagca agaacaagcc gcacgtcaag cggcccatga acgccttcat ggtgtgggcg 720

caggcggcgc gcaggaagct cgcggaccag tacccgcact tgcacaacgc cgagctcagc 780

aagacgctgg gcaagctctg gagacttctg aacgagagcg agaagcggcc cttcgtggag 840

gaggcggagc ggctgcgcgt gcagcacaag aaggaccacc cggattacaa gtaccagccg 900

cggcggagga agtcggtgaa gaacgggcag gcggaggcag aggaggccac ggagcagacg 960

cacatctccc ccaacgccat cttcaaggcg ctgcaggccg actcgccaca ctcctcctcc 1020

ggcatgagcg aggtgcactc ccccggcgag cactcggggc aatcccaggg cccaccgacc 1080

ccacccacca cccccaaaac cgacgtgcag ccgggcaagg ctgacctgaa gcgagagggg 1140

cgccccttgc cagagggggg cagacagccc cctatcgact tccgcgacgt ggacatcggc 1200

gagctgagca gcgacgtcat ctccaacatc gagaccttcg atgtcaacga gtttgaccag 1260

tacctgccgc ccaacggcca cccgggggtg ccggccacgc acggccaggt cacctacacg 1320

ggcagctacg gcatcagcag caccgcggcc accccggcga gcgcgggcca cgtgtggatg 1380

tccaagcagc aggcgccgcc gccacccccg cagcagcccc cacaggcccc gccggccccg 1440

caggcgcccc cgcagccgca ggcggcgccc ccacagcagc cggcggcacc cccgcagcag 1500

ccacaggcgc acacgctgac cacgctgagc agcgagccgg gccagtccca gcgaacgcac 1560

atcaagacgg agcagctgag ccccagccac tacagcgagc agcagcagca ctcgccccaa 1620

cagatcgcct acagcccctt caacctccca cactacagcc cctcctaccc gcccatcacc 1680

cgctcacagt acgactacac cgaccaccag aactccagct cctactacag ccacgcggca 1740

ggccagggca ccggcctcta ctccaccttc acctacatga accccgctca gcgccccatg 1800

tacaccccca tcgccgacac ctctggggtc ccttccatcc cgcagaccca cagcccccag 1860

cactgggaac aacccgtcta cacacagctc actcgacctt gaggaggcct cccacgaagg 1920

gcgaagatgg ccgagatgat cctaaaaata accgaagaaa gagaggacca accagaattc 1980

cctttggaca tttgtgtttt tttgtttttt tattttgttt tgttttttct tcttcttctt 2040

cttccttaaa gacatttaag ctaaaggcaa ctcgtaccca aatttccaag acacaaacat 2100

gacctatcca agcgcattac ccacttgtgg ccaatcagtg gccaggccaa ccttggctaa 2160

atggagcagc gaaatcaacg agaaactgga ctttttaaac cctcttcaga gcaagcgtgg 2220

aggatgatgg agaatcgtgt gatcagtgtg ctaaatctct ctgcctgttt ggactttgta 2280

attatttttt tagcagtaat taaagaaaaa agtcctctgt gaggaatatt ctctatttta 2340

aatattttta gtatgtactg tgtatgattc attaccattt tgaggggatt tatacatatt 2400

tttagataaa attaaatgct cttatttttc caacagctaa actactctta gttgaacagt 2460

gtgccctagc ttttcttgca accagagtat ttttgtacag atttgctttc tcttacaaaa 2520

agaaaaaaaa aatcctgttg tattaacatt taaaaacaga attgtgttat gtgatcagtt 2580

ttgggggtta actttgctta attcctcagg ctttgcgatt taaggaggag ctgccttaaa 2640

aaaaaataaa ggccttattt tgcaattatg ggagtaaaca atagtctaga gaagcatttg 2700

gtaagcttta tcatatatat attttttaaa gaagagaaaa acaccttgag ccttaaaacg 2760

gtgctgctgg gaaacatttg cactctttta gtgcatttcc tcctgccttt gcttgttcac 2820

tgcagtctta agaaagaggt aaaaggcaag caaaggagat gaaatctgtt ctgggaatgt 2880

ttcagcagcc aataagtgcc cgagcacact gcccccggtt gcctgcctgg gccccatgtg 2940

gaaggcagat gcctgctcgc tctgtcacct gtgcctctca gaacaccagc agttaacctt 3000

caagacattc cacttgctaa aattatttat tttgtaagga gaggttttaa ttaaaacaaa 3060

aaaaaattct tttttttttt tttttccaat tttaccttct ttaaaatagg ttgttggagc 3120

tttcctcaaa gggtatggtc atctgttgtt aaattatgtt cttaactgta accagttttt 3180

ttttatttat ctctttaatc ttttttttatt attaaaagca agtttctttg tattcctcac 3240

cctagatttg tataaatgcc tttttgtcca tcccttttttt ctttgttgtt tttgttgaaa 3300

acaaactgga aacttgtttc tttttttgta taaatgagag attgcaaatg tagtgtatca 3360

ctgagtcatt tgcagtgttt tctgccacag acctttgggc tgccttatat tgtgtgtgtg 3420

tgtgggtgtg tgtgtgtttt gacacaaaaa caatgcaagc atgtgtcatc catatttctc 3480

tgcatcttct cttggagtga gggaggctac ctggagggga tcagcccact gacagacctt 3540

aatcttaatt actgctgtgg ctagagagtt tgaggattgc tttttaaaaa agacagcaaa 3600

ctttttttttt tatttaaaaa aagatatatt aacagtttta gaagtcagta gaataaaatc 3660

ttaaagcact cataatatgg catccttcaa tttctgtata aaagcagatc tttttaaaaa 3720

gatacttctg taacttaaga aacctggcat ttaaatcata ttttgtcttt aggtaaaagc 3780

tttggtttgt gttcgtgttt tgtttgtttc acttgtttcc ctcccagccc caaacctttt 3840

gttctctccg tgaaacttac ctttcccttt ttctttctct tttttttttt tgtatattat 3900

tgtttacaat aaatatacat tgcattaaaa agaaaaaaaa aaaaaaaaaa aaaaaaaaaa 3960

aaa 3963

<210> 26
<211> 8605
<212> DNA
<213> Homo sapiens

<400> 26
aattcgccaa ctgaaaaagt gggaaaggat gtctggaggc gaggcgtccc attacagagg 60

aaggagctcg ctatataagc cagccaaagt tggctgcacc ggccacagcc tgcctactgt 120

cacccgcctc tcccgcgcgc agatacacgc ccccgcctcc gtgggcacaa aggcagcgct 180

gctggggaac tcgggggaac gcgcacgtgg gaaccgccgc agctccacac tccaggtact 240

tcttccaagg acctaggtct ctcgcccatc ggaaagaaaa taattctttc aagaagatca 300

gggacaactg atttgaagtc tactctgtgc ttctaaatcc ccaattctgc tgaaagtgag 360

ataccctaga gccctagagc cccagcagca cccagccaaa cccacctcca ccatgggggc 420

catgactcag ctgttggcag gtgtctttct tgctttcctt gccctcgcta ccgaaggtgg 480

ggtcctcaag aaagtcatcc ggcacaagcg acagagtggg gtgaacgcca ccctgccaga 540

agagaaccag ccagtggtgt ttaaccacgt ttacaacatc aagctgccag tgggatccca 600

gtgttcggtg gatctggagt cagccagtgg ggagaaagac ctggcaccgc cttcagagcc 660

cagcgaaagc tttcaggagc acacagtgga tggggaaaac cagattgtct tcacacatcg 720

catcaacatc ccccgccggg cctgtggctg tgccgcagcc cctgatgtta aggagctgct 780

gagcagactg gaggagctgg agaacctggt gtcttccctg agggagcaat gtactgcagg 840

agcaggctgc tgtctccagc ctgccacagg ccgcttggac accaggccct tctgtagcgg 900
tcggggcaac ttcagcactg aaggatgtgg ctgtgtctgc gaacctggct ggaaaggccc 960
caactgctct gagcccgaat gtccaggcaa ctgtcacctt cgaggccggt gcattgatgg 1020
gcagtgcatc tgtgacgacg gcttcacggg cgaggactgc agccagctgg cttgccccag 1080
cgactgcaat gaccagggca agtgcgtaaa tggagtctgc atctgtttcg aaggctacgc 1140
cggggctgac tgcagccgtg aaatctgccc agtgccctgc agtgaggagc acggcacatg 1200
tgtagatggc ttgtgtgtgt gccacgatgg ctttgcaggc gatgactgca acaagcctct 1260
gtgtctcaac aattgctaca accgtggacg atgcgtggag aatgagtgcg tgtgtgatga 1320
gggtttcacg ggcgaagact gcagtgagct catctgcccc aatgactgct tcgaccgggg 1380
ccgctgcatc aatggcacct gctactgcga agaaggcttc acaggtgaag actgcgggaa 1440
acccacctgc ccacatgcct gccacaccca gggccggtgt gaggaggggc agtgtgtatg 1500
tgatgagggc tttgccggtg tggactgcag cgagaagagg tgtcctgctg actgtcacaa 1560
tcgtggccgc tgtgtagacg ggcggtgtga gtgtgatgat ggtttcactg gagctgactg 1620
tggggagctc aagtgtccca atggctgcag tggccatggc cgctgtgtca atgggcagtg 1680
tgtgtgtgat gagggctata ctggggagga ctgcagccag ctacggtgcc ccaatgactg 1740
tcacagtcgg ggccgctgtg tcgagggcaa atgtgtatgt gagcaaggct tcaagggcta 1800
tgactgcagt gacatgagct gccctaatga ctgtcaccag cacggccgct gtgtgaatgg 1860
catgtgtgtt tgtgatgacg gctacacagg ggaagactgc cgggatcgcc aatgccccag 1920
ggactgcagc aacaggggcc tctgtgtgga cggacagtgc gtctgtgagg acggcttcac 1980
cggccctgac tgtgcagaac tctcctgtcc aaatgactgc catggccagg gtcgctgtgt 2040
gaatgggcag tgcgtgtgcc atgaaggatt tatgggcaaa gactgcaagg agcaaagatg 2100
tcccagtgac tgtcatggcc agggccgctg cgtggacggc cagtgcatct gccacgaggg 2160
cttcacaggc ctggactgtg gccagcactc ctgccccagt gactgcaaca acttaggaca 2220
atgcgtctcg ggccgctgca tctgcaacga gggctacagc ggagaagact gctcagaggt 2280
gtctcctccc aaagacctcg ttgtgacaga agtgacggaa gagacggtca acctggcctg 2340
ggacaatgag atgcgggtca cagagtacct tgtcgtgtac acgcccaccc acgagggtgg 2400
tctggaaatg cagttccgtg tgcctgggga ccagacgtcc accatcatcc aggagctgga 2460
gcctggtgtg gagtacttta tccgtgtatt tgccatcctg gagaacaaga agagcattcc 2520
tgtcagcgcc agggtggcca cgtacttacc tgcacctgaa ggcctgaaat tcaagtccat 2580
caaggagaca tctgtggaag tggagtggga tcctctagac attgcttttg aaacctggga 2640
gatcatcttc cggaatatga ataaagaaga tgagggagag atcaccaaaa gcctgaggag 2700

gccagagacc tcttaccggc aaactggtct agctcctggg caagagtatg agatatctct 2760
gcacatagtg aaaaacaata cccggggccc tggcctgaag agggtgacca ccacacgctt 2820
ggatgccccc agccagatcg aggtgaaaga tgtcacagac accactgcct tgatcacctg 2880
gttcaagccc ctggctgaga tcgatggcat tgagctgacc tacggcatca aagacgtgcc 2940
aggagaccgt accaccatcg atctcacaga ggacgagaac cagtactcca tcgggaacct 3000
gaagcctgac actgagtacg aggtgtccct catctcccgc agaggtgaca tgtcaagcaa 3060
cccagccaaa gagaccttca caacaggcct cgatgctccc aggaatcttc gacgtgtttc 3120
ccagacagat aacagcatca ccctggaatg gaggaatggc aaggcagcta ttgacagtta 3180
cagaattaag tatgccccca tctctggagg ggaccacgct gaggttgatg ttccaaagag 3240
ccaacaagcc acaaccaaaa ccacactcac aggtctgagg ccgggaactg aatatgggat 3300
tggagtttct gctgtgaagg aagacaagga gagcaatcca gcgaccatca acgcagccac 3360
agagttggac acgcccaagg accttcaggt ttctgaaact gcagagacca gcctgaccct 3420
gctctggaag acaccgttgg ccaaatttga ccgctaccgc ctcaattaca gtctccccac 3480
aggccagtgg gtgggagtgc agcttccaag aaacaccact tcctatgtcc tgagaggcct 3540
ggaaccagga caggagtaca atgtcctcct gacagccgag aaaggcagac acaagagcaa 3600
gcccgcacgt gtgaaggcat ccactgaaca agcccctgag ctggaaaacc tcaccgtgac 3660
tgaggttggc tgggatggcc tcagactcaa ctggaccgca gctgaccagg cctatgagca 3720
ctttatcatt caggtgcagg aggccaacaa ggtggaggca gctcggaacc tcaccgtgcc 3780
tggcagcctt cgggctgtgg acataccggg cctcaaggct gctacgcctt atacagtctc 3840
catctatggg gtgatccagg gctatagaac accagtgctc tctgctgagg cctccacagg 3900
ggaaactccc aatttgggag aggtcgtggt ggccgaggtg ggctgggatg ccctcaaact 3960
caactggact gctccagaag ggcctatga gtactttttc attcaggtgc aggaggctga 4020
cacagtagag gcagcccaga acctcaccgt cccaggagga ctgaggtcca cagacctgcc 4080
tgggctcaaa gcagccactc attataccat caccatccgc ggggtcactc aggacttcag 4140
cacaacccct ctctctgttg aagtcttgac agaggaggtt ccagatatgg gaaacctcac 4200
agtgaccgag gttagctggg atgctctcag actgaactgg accacgccag atggaaccta 4260
tgaccagttt actattcagg tccaggaggc tgaccaggtg gaagaggctc acaatctcac 4320
ggttcctggc agcctgcgtt ccatggaaat cccaggcctc agggctggca ctccttacac 4380
agtcaccctg cacggcgagg tcaggggcca cagcactcga ccccttgctg tagaggtcgt 4440
cacagaggat ctcccacagc tgggagattt agccgtgtct gaggttggct gggatggcct 4500
cagactcaac tggaccgcag ctgacaatgc ctatgagcac tttgtcattc aggtgcagga 4560
ggtcaacaaa gtggaggcag cccagaacct cacgttgcct ggcagcctca gggctgtgga 4620

catcccgggc ctcgaggctg ccacgcctta tagagtctcc atctatgggg tgatccgggg 4680
ctatagaaca ccagtactct ctgctgaggc ctccacagcc aaagaacctg aaattggaaa 4740
cttaaatgtt tctgacataa ctcccgagag cttcaatctc tcctggatgg ctaccgatgg 4800
gatcttcgag acctttacca ttgaaattat tgattccaat aggttgctgg agactgtgga 4860
atataatatc tctggtgctg aacgaactgc ccatatctca gggctacccc ctagtactga 4920
ttttattgtc tacctctctg gacttgctcc cagcatccgg accaaaacca tcagtgccac 4980
agccacgaca gaggccctgc cccttctgga aaacctaacc atttccgaca ttaatcccta 5040
cgggttcaca gtttcctgga tggcatcgga gaatgccttt gacagctttc tagtaacggt 5100
ggtggattct gggaagctgc tggacccccca ggaattcaca ctttcaggaa cccagaggaa 5160
gctggagctt agaggcctca taactggcat tggctatgag gttatggtct ctggcttcac 5220
ccaagggcat caaaccaagc ccttgagggc tgagattgtt acagaagccg aaccggaagt 5280
tgacaacctt ctggtttcag atgccacccc agacggtttc cgtctgtcct ggacagctga 5340
tgaaggggtc ttcgacaatt ttgttctcaa aatcagagat accaaaaagc agtctgagcc 5400
actggaaata accctacttg cccccgaacg taccagggac ataacaggtc tcagagaggc 5460
tactgaatac gaaattgaac tctatggaat aagcaaagga aggcgatccc agacagtcag 5520
tgctatagca acaacagcca tgggctcccc aaaggaagtc attttctcag acatcactga 5580
aaattcggct actgtcagct ggagggcacc cacagcccaa gtggagagct tccggattac 5640
ctatgtgccc attacaggag gtacaccctc catggtaact gtggacggaa ccaagactca 5700
gaccaggctg gtgaaactca tacctggcgt ggagtacctt gtcagcatca tcgccatgaa 5760
gggctttgag gaaagtgaac ctgtctcagg gtcattcacc acagctctgg atggcccatc 5820
tggcctggtg acagccaaca tcactgactc agaagccttg gccaggtggc agccagccat 5880
tgccactgtg gacagttatg tcatctccta cacaggcgag aaagtgccag aaattacacg 5940
cacggtgtcc gggaacacag tggagtatgc tctgaccgac ctcgagcctg ccacggaata 6000
cacactgaga atctttgcag agaaagggcc ccagaagagc tcaaccatca ctgccaagtt 6060
cacaacagac ctcgattctc caagagactt gactgctact gaggttcagt cggaaactgc 6120
cctccttacc tggcgacccc cccgggcatc agtcaccggt tacctgctgg tctatgaatc 6180
agtggatggc acagtcaagg aagtcattgt gggtccagat accacctcct acagcctggc 6240
agacctgagc ccatccaccc actacacagc caagatccag gcactcaatg ggcccctgag 6300
gagcaatatg atccagacca tcttcaccac aattggactc ctgtacccct tccccaagga 6360
ctgctcccaa gcaatgctga atggagacac gacctctggc ctctacacca tttatctgaa 6420
tggtgataag gctgaggcgc tggaagtctt ctgtgacatg acctctgatg gggtggatg 6480

gattgtgttc ctgagacgca aaaacggacg cgagaacttc taccaaaact ggaaggcata 6540
tgctgctgga tttggggacc gcagagaaga attctggctt gggctggaca acctgaacaa 6600
aatcacagcc caggggcagt acgagctccg ggtggacctg cgggaccatg gggagacagc 6660
ctttgctgtc tatgacaagt tcagcgtggg agatgccaag actcgctaca agctgaaggt 6720
ggaggggtac agtgggacag caggtgactc catggcctac cacaatggca gatccttctc 6780
cacctttgac aaggacacag attcagccat caccaactgt gctctgtcct acaaaggggc 6840
tttctggtac aggaactgtc accgtgtcaa cctgatgggg agatatgggg acaataacca 6900
cagtcagggc gttaactggt tccactggaa gggccacgaa cactcaatcc agtttgctga 6960
gatgaagctg agaccaagca acttcagaaa tcttgaaggc aggcgcaaac gggcataaat 7020
tccagggacc actgggtgag agaggaataa ggcccagagc gaggaaagga ttttaccaaa 7080
gcatcaatac aaccagccca accatcggtc cacacctggg catttggtga gagtcaaagc 7140
tgaccatgga tccctggggc caacggcaac agcatgggcc tcacctcctc tgtgatttct 7200
ttctttgcac caaagacatc agtctccaac atgtttctgt tttgttgttt gattcagcaa 7260
aaatctccca gtgacaacat cgcaatagtt ttttacttct cttaggtggc tctgggaatg 7320
ggagaggggt aggatgtaca ggggtagttt gttttagaac cagccgtatt ttacatgaag 7380
ctgtataatt aattgtcatt atttttgtta gcaaagatta aatgtgtcat tggaagccat 7440
cccttttttt acatttcata caacagaaac cagaaaagca atactgtttc cattttaagg 7500
atatgattaa tattattaat ataataatga tgatgatgat gatgaaaact aaggattttt 7560
caagagatct ttctttccaa aacatttctg gacagtacct gattgtattt ttttttaaa 7620
taaaagcaca agtacttttg agtttgttat tttgctttga attgttgagt ctgaatttca 7680
ccaaagccaa tcatttgaac aaagcgggga atgttgggat aggaaaggta agtagggata 7740
gtggtcaagt gggaggggtg gaaaggagac taaagactgg gagagaggga agcacttttt 7800
ttaaataaag ttgaacacac ttgggaaaag cttacaggcc aggcctgtaa tcccaacact 7860
ttgggaggcc aaggtgggag gatagcttaa ccccaggagt ttgagaccag cctgagcaac 7920
atagtgagaa cttgtctcta cagaaaaaaa aaaaaaaaaa aatttaatta ggcaagcgtg 7980
gtagtgcgca cctgtcgtcc cagctactca ggaggctgag gtaggaaaat cactggagcc 8040
caggagttag aggttacagt gagctatgat cacactactg cactccagcc tgggcaacag 8100
agggagaccc tgtctctaaa taaaaaaaga aaagaaaaaa aaagcttaca acttgagatt 8160
cagcatcttg ctcagtattt ccaagactaa tagattatgg tttaaaagat gcttttatac 8220
tcattttcta atgcaactcc tagaaactct atgatatagt tgaggtaagt attgttacca 8280
cacatgggct aagatcccca gaggcagact gcctgagttc aattcttggc tccaccattc 8340
ccaagttccc taacctctct atgcctcagt ttcctcttct gtaaagtagg gacactcata 8400

```
cttctcattt cagaacattt ttgtgaagaa taaattatgt tatccatttg aggcccttag     8460
aatggtaccc ggtgtatatt aagtgctagt acatgttagc tatcatcatt atcactttat     8520
atgagatgga ctggggttca tagaaaccca atgacttgat tgtggctact actcaataaa     8580
taatagaatt tggatttaaa aaaaa                                           8605
```

**Description**

FIELD OF THE INVENTION

The present invention generally relates to the field of bioinformatics, genomic processing, proteomic processing, and related arts. More particularly, the present invention relates to a computer-implemented method for inferring activity of a Notch cellular signaling pathway in a subject performed by a digital processing device, wherein the inferring is based on expression levels of three or more target genes of the Notch cellular signaling pathway measured in a sample of the subject. The present invention further relates to an apparatus for inferring activity of a Notch cellular signaling pathway in a subject comprising a digital processor configured to perform the method, to a non-transitory storage medium for inferring activity of a Notch cellular signaling pathway in a subject storing instructions that are executable by a digital processing device to perform the method, and to a computer program for inferring activity of a Notch cellular signaling pathway in a subject comprising program code means for causing a digital processing device to perform the method, when the computer program is run on the digital processing device. The present invention further relates to a kit for measuring expression levels of three or more target genes of the Notch cellular signaling pathway in a sample of a subject, to a kit for inferring activity of a Notch cellular signaling pathway in a subject, and to uses of the kits in performing the method.

BACKGROUND OF THE INVENTION

Genomic and proteomic analyses have substantial realized and potential promise for clinical application in medical fields such as oncology, where various cancers are known to be associated with specific combinations of genomic mutations/variations and/or high or low expression levels for specific genes, which play a role in growth and evolution of cancer, e.g., cell proliferation and metastasis.

Notch is an inducible transcription factor that regulates the expression of many genes involved in embryonic development, the immune response, and in cancer. Regarding pathological disorders, such as cancer (e.g., breast or ovarian cancer), abnormal Notch pathway activity plays an important role (see Aster J.C. et al., "The varied roles of Notch in cancer", Annual Review of Pathology, Vol. 12, No. 1, December 2016, pages 245 to 275). The Notch cellular signaling pathway consists of a protein receptor from the Notch family, and a family of (cell-bound) ligands (DSL family) which induce cleavage of the bound receptor, upon which the cleaved intracellular fragment moves to the nucleus, where it forms, together with other proteins, an active transcription factor complex which binds and transactivates a well-defined set of target genes (see also Fig. 1, which is based on Guruharsha K.G. et al., "The Notch signaling system: recent insights into the complexity of a conserved pathway", Nature Reviews Genetics, Vol. 13, September 2012, pages 654 to 666).

With respect to the Notch signaling in e.g. cancer, it is important to be able to detect abnormal Notch signaling activity in order to enable the right choice of targeted drug treatment. Currently anti-Notch therapies are being developed (see Espinoza I. and Miele L., "Notch inhibitors for cancer treatment", Pharmacology & Therapeutics, Vol. 139, No. 2, August 2013, pages 95 to 110). However, today there is no clinical assay available to assess the functional state resp. activity of the Notch cellular signaling pathway, which in its active state indicates that it is, for instance, more likely to be tumor-promoting compared to its passive state. It is therefore desirable to be able to improve the possibilities of characterizing patients that have a disease, such as a cancer, e.g., a breast, cervical, endometrial, ovarian, pancreatic or prostate cancer, or an immune disorder, which is at least partially driven by an abnormal activity of the Notch cellular signaling pathway, and that are therefore likely to respond to inhibitors of the Notch cellular signaling pathway.

SUMMARY OF THE INVENTION

In accordance with a main aspect of the present invention, the above problem is solved by a computer-implemented method for inferring activity of a Notch cellular signaling pathway in a subject performed by a digital processing device, wherein the inferring comprises:

receiving expression levels of three or more, for example, three, four, five, six, seven, eight, nine, ten, eleven, twelve or more, target gene(s) of the Notch cellular signaling pathway measured in a sample of the subject,

determining an activity level of a Notch transcription factor (TF) element in the sample of the subject, the Notch TF element controlling transcription of the three or more Notch target gene(s), the determining being based on evaluating a calibrated mathematical model pathway relating the expression levels of the three or more Notch target gene(s) to the activity level of the Notch TF element, and

inferring the activity of the Notch cellular signaling pathway in the subject based on the determined activity level of the Notch TF element in the sample of the subject,

wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND 1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

Herein, the "activity level" of a TF element denotes the level of activity of the TF element regarding transcription of its target genes.

The present invention is based on the innovation of the inventors that a suitable way of identifying effects occurring in the Notch cellular signaling pathway can be based on a measurement of the signaling output of the Notch cellular signaling pathway, which is - amongst others - the transcription of the target genes, which is controlled by a Notch transcription factor (TF) element that is controlled by the Notch cellular signaling pathway. This innovation by the inventors assumes that the TF activity level is at a quasi-steady state in the sample, which can be detected by means of - amongst others - the expression values of the Notch target genes. The Notch cellular signaling pathway targeted herein is known to control many functions in many cell types in humans, such as proliferation, differentiation and wound healing. Regarding pathological disorders, such as cancer (e.g., breast, cervical, endometrial, ovarian, pancreatic or prostate cancer), the abnormal Notch cellular signaling activity plays an important role, which is detectable in the expression profiles of the target genes and thus exploited by means of a calibrated mathematical pathway model.

The present invention makes it possible to determine the activity of the Notch cellular signaling pathway in a subject by (i) determining an activity level of a Notch TF element in the sample of the subject, wherein the determining is based on evaluating a calibrated mathematical model relating the expression levels of three or more target gene(s) of the Notch cellular signaling pathway, the transcription of which is controlled by the Notch TF element, to the activity level of the Notch TF element, and by (ii) inferring the activity of the Notch cellular signaling pathway in the subject based on the determined activity level of the Notch TF element in the sample of the subject. This preferably allows improving the possibilities of characterizing patients that have a disease, such as cancer, e.g., a breast, cervical, endometrial, ovarian, pancreatic or prostate cancer, which is at least partially driven by an abnormal activity of the Notch cellular signaling pathway, and that are therefore likely to respond to inhibitors of the Notch cellular signaling pathway. In particular embodiments, treatment determination can be based on a specific Notch cellular signaling pathway activity. In a particular embodiment, the Notch cellular signaling status can be set at a cutoff value of odds of the Notch cellular signaling pathway being active of, for example, 10:1, 5:1,4:1,2:1, 1:1,1:2, 1:4,1:5, or 1:10.

Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-Jκ, Su(H) and LAG-1), which is capable of binding to specific DNA sequences, and preferably one co-activator protein from the mastermind-like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

The calibrated mathematical pathway model may be a probabilistic model, preferably a Bayesian network model, based on conditional probabilities relating the activity level of the Notch TF element and the expression levels of the three or more Notch target genes, or the calibrated mathematical pathway model may be based on one or more linear combination(s) of the expression levels of the three or more Notch target genes. In particular, the inferring of the activity of the Notch cellular signaling pathway may be performed as disclosed in the published international patent application WO 2013/011479 A2 ("Assessment of cellular signaling pathway activity using probabilistic modeling of target gene expression") or as described in the published international patent application WO 2014/102668 A2 ("Assessment of cellular signaling pathway activity using linear combination(s) of target gene expressions"), the contents of which are herewith incorporated in their entirety. Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

The term "subject", as used herein, refers to any living being. In some embodiments, the subject is an animal, preferably a mammal. In certain embodiments, the subject is a human being, preferably a medical subject. In still other embodiments, the subject is a cell line.

The term "target gene" as used herein, means a gene whose transcription is directly or indirectly controlled by a Notch transcription factor element. The "target gene" may be a "direct target gene" and/or an "indirect target gene" (as described herein). Moreover, the "target gene(s)" may be "direct target genes" and/or "indirect target genes" (as described herein).

Particularly preferred is a method (as described herein), wherein the three or more Notch target gene(s) are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: CD44, EPHB3, HES7, HEY1, HEYL, NFKB2, NOX1, PBX1, PIN1, PLXND1, and SOX9. Particularly preferred is a method (as described herein), wherein the three or more Notch target gene(s) are selected from the group consisting of: DTX1, EPHB3, HES1, HES4, HES5, HEY2, MYC, NFKB2, NRARP, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: EPHB3, NFKB2, PIN1, PLXND1, and SOX9.

Particularly suitable Notch target genes are described in the following text passages as well as the examples below (see, e.g., Tables 1 to 3 below).

Thus, according to a preferred embodiment the Notch target genes are selected from the group consisting of the Notch target genes listed in Table 1, Table 2, or Table 3 below.

It has been found by the present inventors that the Notch target genes in the successively shorter lists become more and more probative for determining the activity of the Notch cellular signaling pathway.

Another aspect of the present invention relates to a method (as described herein), further comprising:

determining whether the Notch cellular signaling pathway is operating abnormally in the subject based on the inferred activity of the Notch cellular signaling pathway in the subject.

The present invention also relates to a method (as described herein), further comprising:

recommending prescribing a drug for the subject that corrects for the abnormal operation of the Notch cellular signaling pathway,
wherein the recommending is performed if the Notch cellular signaling pathway is determined to be operating abnormally in the subject based on the inferred activity of the Notch cellular signaling pathway.

The phrase "the cellular signaling pathway is operating abnormally" refers to the case where the "activity" of the pathway is not as expected, wherein the term "activity" may refer to the activity of the transcription factor complex in driving the target genes to expression, i.e., the speed by which the target genes are transcribed. "Normal" may be when it is inactive in tissue where it is expected to be inactive and active where it is expected to be active. Furthermore, there may be a certain level of activity that is considered "normal", and anything higher or lower maybe considered "abnormal".

The present invention also relates to a method (as described herein), wherein the abnormal operation of the Notch cellular signaling pathway is an operation in which the Notch cellular signaling pathway operates as a tumor promoter in the subject.

The sample(s) to be used in accordance with the present invention can be an extracted sample, that is, a sample that has been extracted from the subject. Examples of the sample include, but are not limited to, a tissue, cells, blood and/or a body fluid of a subject. If the subject is a medical subject that has or may have cancer, it can be, e.g., a sample obtained from a cancer lesion, or from a lesion suspected for cancer, or from a metastatic tumor, or from a body cavity in which fluid is present which is contaminated with cancer cells (e.g., pleural or abdominal cavity or bladder cavity), or from other body fluids containing cancer cells, and so forth, preferably via a biopsy procedure or other sample extraction procedure. The cells of which a sample is extracted may also be tumorous cells from hematologic malignancies (such as leukemia or lymphoma). In some cases, the cell sample may also be circulating tumor cells, that is, tumor cells that have entered the bloodstream and may be extracted using suitable isolation techniques, e.g., apheresis or conventional venous blood withdrawal. Aside from blood, a body fluid of which a sample is extracted may be urine, gastrointestinal contents, or an extravasate. The term "sample", as used herein, also encompasses the case where e.g. a tissue and/or cells and/or a body fluid of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques. In addition, the term "sample", as used herein, also encompasses the case where e.g. a tissue and/or cells and/or a body fluid of the subject have been taken from the subject and have been put on a microscope slide, and the claimed method is performed on the slide. In addition, the term "sample", as used herein, also encompasses the case where e.g. a cell line and/or cell culture has been generated based on the cells/tissue/body fluid that have been taken from the subject.

In accordance with another disclosed aspect, an apparatus for inferring activity of a Notch cellular signaling pathway in a subject comprises a digital processor configured to perform the method of the present invention as described herein.

In accordance with another disclosed aspect, a non-transitory storage medium for inferring activity of a Notch cellular signaling pathway in a subject stores instructions that are executable by a digital processing device to perform the method of the present invention as described herein. The non-transitory storage medium may be a computer-readable storage medium, such as a hard drive or other magnetic storage medium, an optical disk or other optical storage medium, a random access memory (RAM), read only memory (ROM), flash memory, or other electronic storage medium, a network server, or so forth. The digital processing device may be a handheld device (e.g., a personal data assistant or smartphone), a notebook computer, a desktop computer, a tablet computer or device, a remote network server, or so forth.

In accordance with another disclosed aspect, a computer program for inferring activity of a Notch cellular signaling pathway in a subject comprises program code means for causing a digital processing device to perform the method of the present invention as described herein, when the computer program is run on the digital processing device. The digital processing device may be a handheld device (e.g., a personal data assistant or smartphone), a notebook computer, a desktop computer, a tablet computer or device, a remote network server, or so forth.

In accordance with another disclosed aspect, a kit for measuring expression levels of three or more, for example, three, four, five, six, seven, eight, nine, ten, eleven, twelve or more, target gene(s) of the Notch cellular signaling pathway in a sample of a subject comprises:

one or more components for determining the expression levels of the three or more Notch target gene(s) in the sample of the subject,
wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC. Particularly preferred is a kit (as described herein), wherein the three or more Notch target gene(s) are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: CD44, EPHB3, HES7, HEY1, HEYL, NFKB2, NOX1, PBX1, PIN1, PLXND1, and SOX9.

Particularly preferred is a kit (as described herein), wherein the three or more Notch target gene(s) are selected from the group consisting of: DTX1, EPHB3, HES1, HES4, HES5, HEY2, MYC, NFKB2, NRARP, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: EPHB3, NFKB2, PIN1, PLXND1, and SOX9.

The one or more components or means for measuring the expression levels of the three or more Notch target genes can be selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers. In an embodiment, the kit includes a set of labeled probes directed to a portion of an mRNA or cDNA sequence of the three or more Notch target genes as described herein. In an embodiment, the kit includes a set of primers and probes directed to a portion of an mRNA or cDNA sequence of the three or more Notch target gene(s). In an embodiment, the labeled probes are contained in a standardized 96-well plate. In an embodiment, the kit further includes primers or probes directed to a set of reference genes. Such reference genes can be, for example, constitutively expressed genes useful in normalizing or standardizing expression levels of the target gene expression levels described herein.

In an embodiment, the kit for measuring the expression levels of three or more, for example, three, four, five, six, seven, eight, nine, ten, eleven, twelve or more, target gene(s) of the Notch cellular signaling pathway in a sample of a subject comprises:

polymerase chain reaction primers directed to the three or more Notch target gene(s),
probes directed to the three or more Notch target gene(s),
wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

Particularly preferred is a kit (as described herein), wherein the three or more Notch target gene(s) are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: CD44, EPHB3, HES7, HEY1, HEYL, NFKB2, NOX1, PBX1, PIN1, PLXND1, and SOX9.

Particularly preferred is a kit (as described herein), wherein the three or more Notch target gene(s) are selected from the group consisting of: DTX1, EPHB3, HES1, HES4, HES5, HEY2, MYC, NFKB2, NRARP, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: EPHB3, NFKB2, PIN1, PLXND1, and SOX9.

In accordance with another disclosed aspect, a kit for inferring activity of a Notch cellular signaling pathway in a subject comprises:

the kit of the present invention as described herein, and
the apparatus of the present invention as described herein, the non-transitory storage medium of the present invention as described herein, or the computer program of the present invention as described herein.

In accordance with another disclosed aspect, the kits of the present invention as described herein are used in performing the method of the present invention as described herein.

The present invention as described herein can, e.g., also advantageously be used in at least one of the following activities:

diagnosis based on the inferred activity of the Notch cellular signaling pathway in the subject;
prognosis based on the inferred activity of the Notch cellular signaling pathway in the subject;
drug prescription based on the inferred activity of the Notch cellular signaling pathway in the subject;
prediction of drug efficacy based on the inferred activity of the Notch cellular signaling pathway in the subject;
prediction of adverse effects based on the inferred activity of the Notch cellular signaling pathway in the subject;
monitoring of drug efficacy;
drug development;
assay development;
pathway research;
cancer staging;
enrollment of the subject in a clinical trial based on the inferred activity of the Notch cellular signaling pathway in the subject;
selection of subsequent test to be performed; and
selection of companion diagnostics tests.

Further advantages will be apparent to those of ordinary skill in the art upon reading and understanding the attached figures, the following description and, in particular, upon reading the detailed examples provided herein below.

It shall be understood that the method of claim 1, the apparatus of claim 9, the non-transitory storage medium of claim 10, the computer program of claim 11, the kits of claims 12 to 14, and the use of the kits of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

## BRIEF DESCRIPTION OF THE DRAWINGS

Fig. 1 shows schematically and exemplarily the Notch cellular signaling pathway. The pathway is activated when the Notch extracellular domain binds to a DSL-ligand. After cleavage of the receptor the Notch intracellular domain moves to the nucleus and forms, together with other proteins, an active transcription factor complex (see Guruharsha K.G. et al., "The Notch signaling system: recent insights into the complexity of a conserved pathway" Nature Reviews Genetics, Vol. 13, September 2012, pages 654 to 666; "TS" = transcriptional switch; "TG" = target genes).

Fig. 2 shows schematically and exemplarily a mathematical model, herein, a Bayesian network model, used to model the transcriptional program of the Notch cellular signaling pathway.

Fig. 3 shows a flow chart exemplarily illustrating a process for inferring activity of the Notch cellular signaling pathway in a subject based on expression levels of target genes of the Notch cellular signaling pathway measured in a sample of a subject.

Fig. 4 shows a flow chart exemplarily illustrating a process for obtaining a calibrated mathematical pathway model as described herein.

Fig. 5 shows a flow chart exemplarily illustrating a process for determining an activity level of a Notch transcription factor (TF) element in a sample of a subject as described herein.

Fig. 6 shows a flow chart exemplarily illustrating a process for inferring activity of a Notch cellular signaling pathway in a subject using discretized observables.

Fig. 7 shows a flow chart exemplarily illustrating a process for inferring activity of a Notch cellular signaling pathway in a subject using continuous observables.

Fig. 8 shows a flow chart exemplarily illustrating a process for determining Cq values from RT-qPCR analysis of the target genes of the Notch cellular signaling pathway.

Fig. 9 shows calibration results of the Bayesian network model based on the 18 target genes shortlist from Table 2 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668).

Fig. 10 shows calibration results of the Bayesian network model based on the evidence curated list of target genes (26 target genes list) from Table 1 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668).

Fig. 11 shows Notch cellular signaling pathway activity predictions of the trained exemplary Bayesian network model using the 18 target genes shortlist from Table 2 on three independent cultures of the MOLT4 cell line from data set GSE6495.

Fig. 12 shows Notch cellular signaling pathway activity predictions of the trained exemplary Bayesian network model using the evidence curated list of target genes (26 target genes list) from Table 2 on three independent cultures of the MOLT4 cell line from data set GSE6495.

Fig. 13 shows Notch cellular signaling pathway activity predictions of the trained exemplary Bayesian network model using the 18 target genes shortlist from Table 2 on IMR32 cells that were transfected with an inducible Notch3-intracellular construct.

Fig. 14 shows Notch cellular signaling pathway activity predictions of the trained exemplary Bayesian network model using the 18 target genes shortlist from Table 2 on CD34+CD45RA-Lin-HPCs that were cultured for 72 hrs with graded doses of plastic-immobilized Notch ligand Deltalext-IgG (data set GSE29524).

Fig. 15 shows Notch cellular signaling pathway activity predictions of the trained exemplary Bayesian network model using the 18 target genes shortlist from Table 2 on CUTLL1 cells, which are known to have high Notch activity.

Fig. 16 shows Notch cellular signaling pathway activity predictions of the trained exemplary Bayesian network model using the evidence curated list of target genes (26 target gene list) from Table 1 on CUTLL1 cells, which are known to have high Notch activity.

Fig. 17 shows Notch cellular signaling pathway activity predictions of the trained exemplary Bayesian network model using the 18 target genes shortlist from Table 2 on HUVEC cells that were transfected with COUP-TFII siRNA (data set GSE33301).

Fig. 18 shows Notch cellular signaling pathway activity predictions of the trained exemplary Bayesian network model using the 18 target genes shortlist from Table 2 on breast cancer subgroups in samples from GSE6532, GSE9195, GSE12276, GSE20685, GSE21653 and EMTAB365.

Fig. 19 shows Notch cellular signaling pathway activity predictions of the trained exemplary Bayesian network model using the 12 target genes shortlist from Table 3 on CD34+CD45RA-Lin-HPCs that were cultured for 72 hrs with graded doses of plastic-immobilized Notch ligand Deltalext-IgG (data set GSE29524).

Fig. 20 shows Notch cellular signaling pathway activity predictions of the trained exemplary Bayesian network model using the 12 target genes shortlist from Table 3 on CUTLL1 cells, which are known to have high Notch activity.

Fig. 21 shows the correlation between the trained exemplary Bayesian network mode using the evidence curated list of target genes (26 target genes list) from Table 1 and the 12 target genes shortlist from Table 3, respectively.

Fig. 22 shows a comparison of the Notch cellular signaling pathway activity predictions using the list of 7 Notch target genes vs. the list of 10 Notch target genes.

Fig. 23 shows a comparison of the Notch cellular signaling pathway activity predictions using the list of 8 Notch target genes vs. the list of 12 Notch target genes.

DETAILED DESCRIPTION OF EMBODIMENTS

The following examples merely illustrate particularly preferred methods and selected aspects in connection therewith. The teaching provided therein may be used for constructing several tests and/or kits, e.g., to detect, predict and/or diagnose the abnormal activity of the Notch cellular signaling pathway. Furthermore, upon using methods as described herein drug prescription can advantageously be guided, drug response prediction and monitoring of drug efficacy (and/or adverse effects) can be made, drug resistance can be predicted and monitored, e.g., to select subsequent test(s) to be performed (like a companion diagnostic test). The following examples are not to be construed as limiting the scope of the present invention.

**Example 1: Mathematical model construction**

As described in detail in the published international patent application WO 2013/011479 A2 ("Assessment of cellular signaling pathway activity using probabilistic modeling of target gene expression"), by constructing a probabilistic model, e.g., a Bayesian network model, and incorporating conditional probabilistic relationships between the expression levels of three or more target gene(s) of a cellular signaling pathway, herein, the Notch cellular signaling pathway, and the activity level of a transcription factor (TF) element, herein, the Notch TF element, the TF element controlling transcription of the three or more target gene(s) of the cellular signaling pathway, such a model may be used to determine the activity of the cellular signaling pathway with a high degree of accuracy. Moreover, the probabilistic model can be readily updated to incorporate additional knowledge obtained by later clinical studies, by adjusting the conditional probabilities and/or adding new nodes to the model to represent additional information sources. In this way, the probabilistic model can be updated as appropriate to embody the most recent medical knowledge.

In another easy to comprehend and interpret approach described in detail in the published international patent application WO 2014/102668 A2 ("Assessment of cellular signaling pathway activity using linear combination(s) of target gene expressions"), the activity of a cellular signaling pathway, herein, the Notch cellular signaling pathway, may be determined by constructing and evaluating a linear or (pseudo-)linear model incorporating relationships between expression levels of three or more target gene(s) of the cellular signaling pathway and the level of a transcription factor (TF) element, herein, the Notch TF element, the TF element controlling transcription of the three or more target gene(s) of the cellular signaling pathway, the model being based on one or more linear combination(s) of expression levels of the three or more target gene(s).

In both approaches, the expression levels of the three or more target gene(s) may preferably be measurements of the level of mRNA, which can be the result of, e.g., (RT)-PCR and microarray techniques using probes associated with the target gene(s) mRNA sequences, and of RNA-sequencing. In another embodiment, the expression levels of the three or more target gene(s) can be measured by protein levels, e.g., the concentrations and/or activity of the protein(s) encoded by the target gene(s).

The aforementioned expression levels may optionally be converted in many ways that might or might not suit the application better. For example, four different transformations of the expression levels, e.g., microarray-based mRNA levels, may be:

- "continuous data", i.e., expression levels as obtained after preprocessing of microarrays using well known algorithms such as MAS5.0 and fRMA,
- "z-score", i.e., continuous expression levels scaled such that the average across all samples is 0 and the standard deviation is 1,
- "discrete", i.e., every expression above a certain threshold is set to 1 and below it to 0 (e.g., the threshold for a probeset may be chosen as the (weighted) median of its value in a set of a number of positive and the same number of negative clinical samples),
- "fuzzy", i.e., the continuous expression levels are converted to values between 0 and 1 using a sigmoid function of the following format: 1 / (1 + *exp*((*thr* - *expr*) / *se*)), with *expr* being the continuous expression levels, *thr* being the threshold as mentioned before and *se* being a softening parameter influencing the difference between 0 and 1.

One of the simplest linear models that can be constructed is a model having a node representing the transcription factor (TF) element, herein, the Notch TF element, in a first layer and weighted nodes representing direct measurements of the target genes expression levels, e.g., by one probeset that is particularly highly correlated with the particular target gene, e.g., in microarray or (q)PCR experiments, in a second layer. The weights can be based either on calculations from a training data set or based on expert knowledge. This approach of using, in the case where possibly multiple expression levels are measured per target gene (e.g., in the case of microarray experiments, where one target gene can be measured with multiple probesets), only one expression level per target gene is particularly simple. A specific way of selecting the one expression level that is used for a particular target gene is to use the expression level from the probeset that is able to separate active and passive samples of a training data set the best. One method to determine this probeset is to perform a statistical test, e.g., the t-test, and select the probeset with the lowest p-value. The training data set's expression levels of the probeset with the lowest p-value is by definition the probeset with the least likely probability that the expression levels of the (known) active and passive samples overlap. Another selection method is based on odds-ratios. In such a model, one or more expression level(s) are provided for each of the three or more target gene(s) and the one or more linear combination(s) comprise a linear combination including for each of the three or more target gene(s) a weighted term, each weighted term being based on only one expression level of the one or more expression level(s) provided for the respective target gene. If only one expression level is chosen per target gene as described above, the model may be called a "most discriminant probesets" model.

In an alternative to the "most discriminant probesets" model, it is possible, in the case where possibly multiple expression levels are measured per target gene, to make use of all the expression levels that are provided per target gene. In such a model, one or more expression level(s) are provided for each of the three or more target gene(s) and the one or more linear combination(s) comprise a linear combination of all expression levels of the one or more expression level(s) provided for the three or more target gene(s). In other words, for each of the three or more target gene(s), each of the one or more expression level(s) provided for the respective target gene may be weighted in the linear combination by its own (individual) weight. This variant may be called an "all probesets" model. It has an advantage of being relatively simple while making use of all the provided expression levels.

Both models as described above have in common that they are what may be regarded as "single-layer" models, in which the activity level of the TF element is calculated based on a linear combination of expression levels of the one or more probeset of the three or more target gene(s).

After the activity level of the TF element, herein, the Notch TF element, has been determined by evaluating the respective model, the determined TF element activity level can be thresholded in order to infer the activity of the cellular signaling pathway, herein, the Notch cellular signaling pathway. A preferred method to calculate such an appropriate threshold is by comparing the determined TF element activity levels *wlc* (weighted linear combination) of training samples known to have a passive cellular signaling pathway and training samples with an active cellular signaling pathway. A method that does so and also takes into account the variance in these groups is given by using a threshold

$$thr = \frac{\sigma_{wlc_{pas}}\mu_{wlc_{act}} + \sigma_{wlc_{act}}\mu_{wlc_{pas}}}{\sigma_{wlc_{pas}} + \sigma_{wlc_{act}}} \qquad (1)$$

where $\sigma$ and $\mu$ are the standard deviation and the mean of the determined TF element activity levels w*lc* for the training samples. In case only a small number of samples are available in the active and/or passive training samples, a pseudo count may be added to the calculated variances based on the average of the variances of the two groups:

$$\tilde{v} = \frac{v_{wlc_{act}} + v_{wlc_{pas}}}{2}$$

$$\tilde{v}_{wlc_{act}} = \frac{x\,\tilde{v} + (n_{act} - 1)v_{wlc_{act}}}{x + n_{act} - \mathbf{1}} \qquad (2)$$

$$\tilde{v}_{wlc_{pas}} = \frac{x\,\tilde{v} + \left(n_{pas} - 1\right)v_{wlc_{pas}}}{x + n_{pas} - 1}$$

where *v* is the variance of the determined TF element activity levels *wlc* of the groups, x is a positive pseudo count, e.g., 1 or 10, and $n_{act}$ and $n_{pas}$ are the number of active and passive samples, respectively. The standard deviation $\sigma$ can next be obtained by taking the square root of the variance v.

The threshold can be subtracted from the determined TF element activity levels *wlc* for ease of interpretation, resulting in a cellular signaling pathway's activity score in which negative values correspond to a passive cellular signaling pathway and positive values correspond to an active cellular signaling pathway.

As an alternative to the above-described "single-layer" models, a "two-layer" may also be used in an example. In such a model, a summary value is calculated for every target gene using a linear combination based on the measured intensities of its associated probesets ("first (bottom) layer"). The calculated summary value is subsequently combined with the summary values of the other target genes of the cellular signaling pathway using a further linear combination ("second (upper) layer"). Again, the weights can be either learned from a training data set or based on expert knowledge or a combination thereof. Phrased differently, in the "two-layer" model, one or more expression level(s) are provided for each of the three or more target gene(s) and the one or more linear combination(s) comprise for each of the three or more target gene(s) a first linear combination of all expression levels of the one or more expression level(s) provided for the respective target gene ("first (bottom) layer"). The model is further based on a further linear combination including for each of the three or more target gene(s) a weighted term, each weighted term being based on the first linear combination for the respective target gene ("second (upper) layer").

The calculation of the summary values can, in a preferred version of the "two-layer" model, include defining a threshold for each target gene using the training data and subtracting the threshold from the calculated linear combination, yielding the target gene summary. Here the threshold may be chosen such that a negative target gene summary value corresponds to a down-regulated target gene and that a positive target gene summary value corresponds to an up-regulated target gene. Also, it is possible that the target gene summary values are transformed using, e.g., one of the above-described transformations (fuzzy, discrete, etc.), before they are combined in the "second (upper) layer".

After the activity level of the TF element has been determined by evaluating the "two-layer" model, the determined TF element activity level can be thresholded in order to infer the activity of the cellular signaling pathway, as described above.

In the following, the models described above are collectively denoted as "(pseudo-)linear" models. A more detailed description of the training and use of probabilistic models, e.g., a Bayesian network model, is provided in Example 3 below.

**Example 2: Selection of target genes**

A transcription factor (TF) is a protein complex (i.e., a combination of proteins bound together in a specific structure) or a protein that is able to regulate transcription from target genes by binding to specific DNA sequences, thereby controlling the transcription of genetic information from DNA to mRNA. The mRNA directly produced due to this action of the TF complex is herein referred to as a "direct target gene" (of the transcription factor). Cellular signaling pathway activation may also result in more secondary gene transcription, referred to as "indirect target genes". In the following, (pseudo-)linear models or Bayesian network models (as exemplary mathematical models) comprising or consisting of direct target genes as direct links between cellular signaling pathway activity and mRNA level, are preferred, however the distinction between direct and indirect target genes is not always evident. Herein, a method to select direct target genes using a scoring function based on available scientific literature data is presented. Nonetheless, an accidental selection of indirect target genes cannot be ruled out due to limited information as well as biological variations and uncertainties. In order to select the target genes, the MEDLINE database of the National Institute of Health accessible at "www.ncbi.nlm.nih.gov/pubmed" and herein further referred to as "Pubmed" was employed to generate a list of target genes. Furthermore, two additional lists of target genes were selected based on the probative nature of their expression.

Publications containing putative Notch target genes were searched for by using queries such as ("Notch" AND "target gene") in the period of the fourth quarter of 2016 and the first quarter of 2017. The Notch pathway is an embryonic pathway that activates different (but overlapping) target gene profiles depending on the embryonic lineage (see Meier-Stiegen F. et al., "Activated Notch1 target genes during embryonic cell differentiation depend on the cellular context and include lineage determinants and inhibitors", PLoS One, Vol. 5, No. 7, July 2010). The search was focused on sets of target genes that are differentially expressed between cell type / tissue / organ derivatives from the three different embryonic lineages (ectoderm, endoderm, mesoderm), with a specific emphasis on target genes that are expressed in ectodermal and endodermal derived organs/tissues/cells. The resulting publications were further analyzed manually following the methodology described in more detail below.

Specific cellular signaling pathway mRNA target genes were selected from the scientific literature, by using a ranking system in which scientific evidence for a specific target gene was given a rating, depending on the type of scientific experiments in which the evidence was accumulated. While some experimental evidence is merely suggestive of a gene being a direct target gene, like for example an mRNA increasing as detected by means of an increasing intensity of a probeset on a microarray of a cell line in which it is known that the Notch cellular signaling pathway is active, other evidence can be very strong, like the combination of an identified Notch cellular signaling pathway TF binding site and retrieval of this site in a chromatin immunoprecipitation (ChIP) assay after stimulation of the specific cellular signaling pathway in the cell and increase in mRNA after specific stimulation of the cellular signaling pathway in a cell line.

Several types of experiments to find specific cellular signaling pathway target genes can be identified in the scientific literature:

1. ChIP experiments in which direct binding of a TF of the cellular signaling pathway of interest to its binding site on the genome is shown. Example: By using chromatin immunoprecipitation (ChIP) technology putative functional Notch TF binding sites in the DNA of cell lines with and without active induction of the Notch cellular signaling pathway, e.g., by stimulation with a Notch ligand or transfection with NICD, were identified, as a subset of the binding sites recognized purely based on nucleotide sequence. Putative functionality was identified as ChIP-derived evidence that the TF was found to bind to the DNA binding site.
2. Electrophoretic Mobility Shift (EMSA) assays which show in vitro binding of a TF to a fragment of DNA containing the binding sequence. Compared to ChIP-based evidence EMSA-based evidence is less strong, since it cannot be translated to the in vivo situation.
3. Stimulation of the cellular signaling pathway and measuring mRNA expression using a microarray, RNA sequencing, quantitative PCR or other techniques, using Notch cellular signaling pathway-inducible cell lines and measuring mRNA profiles measured at least one, but preferably several time points after induction - in the presence of cycloheximide, which inhibits translation to protein, thus the induced mRNAs are assumed to be direct target genes.
4. Similar to 3, but alternatively measure the mRNAs expression further downstream with protein abundance measurements, such as western blot.
5. Inhibition of the cellular signaling pathway using a Notch inhibitor, e.g., a Gamma-Secretase Inhibitor (GSI) and measuring mRNA expression using a microarray, RNA sequencing, quantitative PCR or other techniques, using Notch cellular signaling pathway-active cell lines and measuring mRNA profiles measured at least one, but preferably several time points after inhibition.
6. Similar to 5, but alternatively measure the mRNAs expression further downstream with protein abundance measurements, such as western blot.
7. Identification of TF binding sites in the genome using a bioinformatics approach. Example for the Notch TF element: Using the CSL/RBP-J binding motif 5'-CGTGGGAA-3', a software program was run on the human genome sequence, and potential binding sites were identified, both in gene promoter regions and in other genomic regions.
8. Similar as 3, only in the absence of cycloheximide.
9. Similar to 4, only in the absence of cycloheximide.

In the simplest form one can give every potential gene 1 point for each of these experimental approaches in which the gene was identified as being a target gene of the Notch family of transcription factors. Using this relative ranking strategy, one can make a list of most reliable target genes.

Alternatively, ranking in another way can be used to identify the target genes that are most likely to be direct target genes, by giving a higher number of points to the technology that provides most evidence for an in vivo direct target gene. In the list above, this would mean 9 points for experimental approach 1), 8 for 2), and going down to 1 point for experimental approach 8). Such a list may be called a "general list of target genes".

Despite the biological variations and uncertainties, the inventors assumed that the direct target genes are the most likely to be induced in a tissue-independent manner. A list of these target genes may be called an "evidence curated list of target genes". Such an evidence curated list of target genes has been used to construct computational models of the Notch cellular signaling pathway that can be applied to samples coming from different tissue sources.

The following will illustrate exemplary how the selection of an evidence curated target gene list specifically was constructed for the Notch cellular signaling pathway.

A scoring function was introduced that gave a point for each type of experimental evidence, such as ChIP, EMSA, differential expression, knock down/out, luciferase gene reporter assay, sequence analysis, that was reported in a publication. Further analysis was performed to allow only for genes that had diverse types of experimental evidence and not only one or two types of experimental evidence, e.g., differential expression. Those genes that had more than two types of experimental evidence available were selected (as shown in Table 1).

A further selection of the evidence curated list of target genes (listed in Table 2, "18 target genes shortlist") was made by the inventors. This selection was made by removing target genes of the evidence curated list that had relatively little evidence, e.g. evidence was found in only one manuscript, and/or were highly specific, e.g. for blood or brain tissue. The target genes of the "18 target genes shortlist" that were proven to be more probative in determining the activity of the Notch signaling pathway from the training samples were selected for the "12 target genes shortlist" (listed in Table 3, "12 target genes shortlist"). Herein, the 12 target genes that had the highest odds ratio (see below) between patient samples from respectively a set of high grade papillary serous ovarian cancer patients (Notch active, subset taken from GSE2109 and GSE9891, from the gene expression omnibus (GEO, www.ncbi.nlm.nih.gov/ geo/, last accessed December 3$^{rd}$, 2016, and a corresponding set of normal ovarian tissue samples (Notch inactive, subset taken from GSE7307, GSE18520, GSE29450 and GSE36668), and/or scored very high on the evidence ranking, were selected.

Table 1: "Evidence curated list of target genes" (26 target genes list) of the Notch cellular signaling pathway used in the Notch cellular signaling pathway models and associated probesets used to measure the mRNA expression level of the target genes.

| Target gene | Probeset | Target gene | Probeset |
|---|---|---|---|
| CD28 | 206545_at | HEY2 | 219743_at |
| | 211856_x_at | | 222921_s_at |
| | 211861_x_at | HEYL | 220662_s_at |
| CD44 | 1557905_s_at | | 226828_s_at |
| | 204489_s_at | KLF5 | 209211_at |
| | 204490_s_at | | 209212_s_at |
| | 209835_x_at | MYC | 202431_s_at |
| | 210916_s_at | NFKB2 | 207535_s_at |
| | 212014_x_at | | 209636_at |
| | 212063_at | | 211524_at |
| DLGAP5 | 203764_at | NOX1 | 206418_at |
| DTX1 | 227336_at | | 207217_s_at |
| EPHB3 | 1438_at | | 207380_x_at |
| | 204600_at | | 210808_s_at |
| FABP7 | 205029_s_at | NRARP | 226499_at |
| | 205030_at | PBX1 | 205253_at |
| | 216192_at | PIN1 | 202927_at |
| GFAP | 203540_at | PLXND1 | 1563657_at |
| | 229259_at | | 212235_at |
| GIMAP5 | 218805_at | | 38671_at |
| | 64064_at | PTCRA | 211252_x_at |
| HES1 | 203393_at | | 211837_s_at |
| | 203394_s_at | | 215492_x_at |
| | 203395_s_at | SOX9 | 202935_s_at |
| HES4 | 227347_x_at | | 202936_s_at |
| HES5 | 239230_at | TNC | 201645_at |
| HES7 | 224548_at | | 237169_at |
| HEY1 | 218839_at | | |
| | 44783_s_at | | |

Table 2: "18 target genes shortlist" of Notch target genes based on the evidence curated list of Notch target genes. (The associated probesets are the same as in Table 1.)

| Target gene |
|---|
| CD44 |
| DTX1 |
| EPHB3 |

(continued)

| Target gene |
|---|
| HES1 |
| HES4 |
| HES5 |
| HES7 |
| HEY1 |
| HEY2 |
| HEYL |
| MYC |
| NFKB2 |
| NOX1 |
| NRARP |
| PBX1 |
| PIN1 |
| PLXND1 |
| SOX9 |

Table 3: "12 target genes shortlist" of Notch target genes based on the evidence curated list of Notch target genes. (The associated probesets are the same as in Table 1.)

| Target gene |
|---|
| DTX1 |
| EPHB3 |
| HES1 |
| HES4 |
| HES5 |
| HEY2 |
| MYC |
| NFKB2 |
| NRARP |
| PIN1 |
| PLXND1 |
| SOX9 |

**Example 3: Training and using the mathematical model**

Before the mathematical model can be used to infer the activity of the cellular signaling pathway, herein, the Notch cellular signaling pathway, in a subject, the model must be appropriately trained.

If the mathematical pathway model is a probabilistic model, e.g., a Bayesian network model, based on conditional probabilities relating the activity level of the Notch TF element and expression levels of three or more target genes of the Notch cellular signaling pathway measured in the sample of the subject, the training may preferably be performed as described in detail in the published international patent application WO 2013/011479 A2 ("Assessment of cellular signaling pathway activity using probabilistic modeling of target gene expression").

If the mathematical pathway model is based on one or more linear combination(s) of expression levels of three or more target genes of the Notch cellular signaling pathway measured in the sample of the subject, the training may preferably be performed as described in detail in the published international patent application WO 2014/102668 A2 ("Assessment of cellular signaling pathway activity using linear combination(s) of target gene expressions").

Herein, an exemplary Bayesian network model as shown in Fig. 2 was used to model the transcriptional program of the Notch cellular signaling pathway in a simple manner. The model consists of three types of nodes: (a) a transcription factor (TF) element (with states "absent" and "present") in a first layer 1; (b) target genes $TG_1$, $TG_2$, $TG_n$ (with states "down" and "up") in a second layer 2, and; (c) measurement nodes linked to the expression levels of the target genes in a third layer 3. These can be microarray probesets $PS_{1,1}$, $PS_{1,2}$, $PS_{1,3}$, $PS_{2,1}$, $PS_{n,1}$, $PS_{n,m}$ (with states "low" and "high"), as preferably used herein, but could also be other gene expression measurements such as RNAseq or RT-qPCR.

A suitable implementation of the mathematical model, herein, the exemplary Bayesian network model, is based on microarray data. The model describes (i) how the expression levels of the target genes depend on the activation of the TF element, and (ii) how probeset intensities, in turn, depend on the expression levels of the respective target genes. For the latter, probeset intensities may be taken from fRMA pre-processed Affymetrix HG-U133Plus2.0 microarrays, which are widely available from the Gene Expression Omnibus (GEO, www.ncbi.nlm.nih.gov/geo) and ArrayExpress (www.ebi.ac.uk/arrayexpress).

As the exemplary Bayesian network model is a simplification of the biology of a cellular signaling pathway, herein, the Notch cellular signaling pathway, and as biological measurements are typically noisy, a probabilistic approach was opted for, i.e., the relationships between (i) the TF element and the target genes, and (ii) the target genes and their respective probesets, are described in probabilistic terms. Furthermore, it was assumed that the activity of the oncogenic cellular signaling pathway which drives tumor growth is not transiently and dynamically altered, but long term or even irreversibly altered. Therefore the exemplary Bayesian network model was developed for interpretation of a static cellular condition. For this reason complex dynamic cellular signaling pathway features were not incorporated into the model.

Once the exemplary Bayesian network model is built and calibrated (see below), the model can be used on microarray data of a new sample by entering the probeset measurements as observations in the third layer 3, and inferring backwards in the model what the probability must have been for the TF element to be "present". Here, "present" is considered to be the phenomenon that the TF element is bound to the DNA and is controlling transcription of the cellular signaling pathway's target genes, and "absent" the case that the TF element is not controlling transcription. This probability is hence the primary read-out that may be used to indicate activity of the cellular signaling pathway, herein, the Notch cellular signaling pathway, which can next be translated into the odds of the cellular signaling pathway being active by taking the ratio of the probability of it being active vs. it being passive (i.e., the odds are given by p/(1-p), where p is the predicted probability of the cellular signaling pathway being active).

In the exemplary Bayesian network model, the probabilistic relations have been made quantitative to allow for a quantitative probabilistic reasoning. In order to improve the generalization behavior across tissue types, the parameters describing the probabilistic relationships between (i) the TF element and the target genes have been carefully hand-picked. If the TF element is "absent", it is most likely that the target gene is "down", hence a probability of 0.95 is chosen for this, and a probability of 0.05 is chosen for the target gene being "up". The latter (non-zero) probability is to account for the (rare) possibility that the target gene is regulated by other factors or that it is accidentally observed as being "up" (e.g. because of measurement noise). If the TF element is "present", then with a probability of 0.70 the target gene is considered "up", and with a probability of 0.30 the target gene is considered "down". The latter values are chosen this way, because there can be several causes why a target gene is not highly expressed even though the TF element is present, e.g., because the gene's promoter region is methylated. In the case that a target gene is not up-regulated by the TF element, but down-regulated, the probabilities are chosen in a similar way, but reflecting the down-regulation upon presence of the TF element. The parameters describing the relationships between (ii) the target genes and their respective probesets have been calibrated on experimental data. For the latter, in this example, microarray data was used from patients samples which are known to have an active Notch cellular signaling pathway whereas normal, healthy samples from a different data set were used as passive Notch cellular signaling pathway samples, but this could also be performed using cell line experiments or other patient samples with known cellular signaling pathway activity status. The resulting conditional probability tables are given by:

*A: for upregulated target genes*

| | $PS_{i,j}$ = *low* | $PS_{i,j}$ = *high* |
|---|---|---|
| $TG_i$ = *down* | $\frac{AL_{i,j}+1}{AL_{i,j}+AH_{i,j}+2}$ | $\frac{AH_{i,j}+1}{AL_{i,j}+AH_{i,j}+2}$ |

(continued)

| | $PS_{i,j}$ = low | $PS_{i,j}$ = high |
|---|---|---|
| $TG_i$ = up | $\frac{PL_{i,j}+1}{PL_{i,j}+PH_{i,j}+2}$ | $\frac{PH_{i,j}+1}{PL_{i,j}+PH_{i,j}+2}$ |

*B: for downregulated target genes*

| | $PS_{i,j}$ = low | $PS_{i,j}$ = high |
|---|---|---|
| $TG_i$ = down | $\frac{PL_{i,j}+1}{PL_{i,j}+PH_{i,j}+2}$ | $\frac{PH_{i,j}+1}{PL_{i,j}+PH_{i,j}+2}$ |
| $TG_i$ = up | $\frac{AL_{i,j}+1}{AL_{i,j}+AH_{i,j}+2}$ | $\frac{AH_{i,j}+1}{AL_{i,j}+AH_{i,j}+2}$ |

In these tables, the variables $AL_{i,j}$, $AH_{i,j}$, $PL_{i,j}$, and $PH_{i,j}$ indicate the number of calibration samples with an "absent" (A) or "present" (P) transcription complex that have a "low" (L) or "high" (H) probeset intensity, respectively. Dummy counts have been added to avoid extreme probabilities of 0 and 1.

To discretize the observed probeset intensities, for each probeset $PS_{i,j}$ a threshold $t_{i,j}$ was used, below which the observation is called "low", and above which it is called "high". This threshold has been chosen to be the (weighted) median intensity of the probeset in the used calibration data set. Due to the noisiness of microarray data, a fuzzy method was used when comparing an observed probeset intensity to its threshold, by assuming a normal distribution with a standard deviation of 0.25 (on a log2 scale) around the reported intensity, and determining the probability mass below and above the threshold.

If instead of the exemplary Bayesian network described above, a (pseudo-)linear model as described in Example 1 above was employed, the weights indicating the sign and magnitude of the correlation between the nodes and a threshold to call whether a node is either "absent" or "present" would need to be determined before the model could be used to infer cellular signaling pathway activity in a test sample. One could use expert knowledge to fill in the weights and the threshold a priori, but typically the model would be trained using a representative set of training samples, of which preferably the ground truth is known, e.g., expression data of probesets in samples with a known "present" transcription factor complex (= active cellular signaling pathway) or "absent" transcription factor complex (= passive cellular signaling pathway).

Known in the field are a multitude of training algorithms (e.g., regression) that take into account the model topology and changes the model parameters, here, the weights and the threshold, such that the model output, here, a weighted linear score, is optimized. Alternatively, it is also possible to calculate the weights directly from the observed expression levels without the need of an optimization algorithm.

A first method, named "black and white"-method herein, boils down to a ternary system, in which each weight is an element of the set {-1, 0, 1}. If this is put in a biological context, the -1 and 1 correspond to target genes or probesets that are down- and up-regulated in case of cellular signaling pathway activity, respectively. In case a probeset or target gene cannot be statistically proven to be either up- or down-regulated, it receives a weight of 0. In one example, a left-sided and right-sided, two sample t-test of the expression levels of the active cellular signaling pathway samples versus the expression levels of the samples with a passive cellular signaling pathway can be used to determine whether a probe or gene is up- or down-regulated given the used training data. In cases where the average of the active samples is statistically larger than the passive samples, i.e., the p-value is below a certain threshold, e.g., 0.3, the target gene or probeset is determined to be up-regulated. Conversely, in cases where the average of the active samples is statistically lower than the passive samples, the target gene or probeset is determined to be down-regulated upon activation of the cellular signaling pathway. In case the lowest p-value (left- or right-sided) exceeds the aforementioned threshold, the weight of the target gene or probeset can be defined to be 0.

A second method, named "log odds"-weights herein, is based on the logarithm (e.g., base e) of the odds ratio. The odds ratio for each target gene or probeset is calculated based on the number of positive and negative training samples for which the probeset/target gene level is above and below a corresponding threshold, e.g., the (weighted) median of all training samples. A pseudo-count can be added to circumvent divisions by zero. A further refinement is to count the samples above/below the threshold in a somewhat more probabilistic manner, by assuming that the probeset/target gene levels are e.g. normally distributed around its observed value with a certain specified standard deviation (e.g., 0.25 on a 2-log scale), and counting the probability mass above and below the threshold. Herein, an odds ratio calculated in combination with a pseudo-count and using probability masses instead of deterministic measurement values is called a "soft" odds ratio.

Further details regarding the inferring of cellular signaling pathway activity using mathematical modeling of target gene expression can be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945.

Herein, we have used publically available data on the expression of patient samples from respectively a set of high grade papillary serous ovarian cancer patients (data sets GSE2109 and GSE9891, from the gene expression omnibus (GEO, www.ncbi.nlm. nih.gov/geo/, last accessed December 3rd, 2016) and a corresponding set of normal ovarian tissue samples (data sets GSE7307, GSE18520, GSE29450 and GSE36668). High grade serous ovarian cancer is known to have an active Notch cellular signaling pathway in the majority of cases while normal ovarian tissue samples have a passive Notch cellular signaling pathway. Before selecting calibration samples, a quality control was performed on the data sets to ensure that samples were reliable. For calibration purposes, the most active Notch ovarian cancer samples were chosen from the available sets, as determined by adding Affymetrix mRNA expression values for all target genes, for each individual sample and subsequently ranking the samples according to total value. The 20 highest ranking samples were assumed to be Notch active. From the 12 normal ovary samples that passed the quality control, 11 samples were chosen as Notch passive calibration samples (1 normal ovary sample was found to be Notch active), sample numbers: GSM176237, GSM729048, GSM462651, GSM729050, GSM729051, GSM175789, GSM462652, GSM176131, GSM176318, GSM898306, GSM898307. (Samples from data set GSE42259 were also considered as Notch passive calibration samples, but after a quality control none of these samples remained.) These were used to calibrate the model for Notch activity and passivity respectively. The calibrated model was evaluated on a number of public data sets from the GEO database, which contained a ground truth with respect to Notch activity, that is, cell lines in which Notch activity was either induced or inhibited (e.g. treated with a Notch inhibitor like gamma-secretase, or having the possibility to induce Notch3-intracellular). As an application example, the model was run on a data set of breast cancer samples for which survival data is known.

Fig. 9 shows calibration results of the Bayesian network model based on the 18 target genes shortlist from Table 2 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The model was able to separate clearly the inactive from the active calibration samples.

Fig. 10 shows calibration results of the Bayesian network model based on the evidence curated list of target genes (26 target genes list) from Table 1 and the methods as described herein using publically available expression data sets of 11 normal ovary (group 1) and 20 high grade papillary serous ovarian carcinoma (group 2) samples (subset of samples taken from data sets GSE2109, GSE9891, GSE7307, GSE18520, GSE29450, GSE36668). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Again, the model was able to separate clearly the inactive from the active calibration samples.

In the following, validation results of the trained exemplary Bayesian network models using the evidence curated list of target genes (26 target genes list) and the 18 target genes shortlist, respectively, are shown in Figs. 11 to 18.

Fig. 11 shows Notch cellular signaling pathway activity predictions of the trained exemplary Bayesian network model using the 18 target genes shortlist from Table 2 on three independent cultures of the MOLT4 cell line from data set GSE6495. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The MOLT4 cell line is known to have high Notch signaling, which the model correctly predicted (group 1). Cells were treated for 48 hours with 5 μM DAPT, a gamma-secretase inhibitor (GSI) (group 2). GSIs are known to inhibit Notch signaling and the model correctly detected a decrease in Notch activity in this group (see Dohda T. et al., "Notch signaling induces SKP2 expression and promotes reduction of p27Kip1 in T-cell acute lymphoblastic leukemia cell", Experimental Cell Research, Vol. 313, No. 14, August 2007, pages 3141 to 3152).

Fig. 12 shows Notch cellular signaling pathway activity predictions of the trained exemplary Bayesian network model using the evidence curated list of target genes (26 target genes list) from Table 1 on three independent cultures of the MOLT4 cell line from data set GSE6495. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The MOLT4 cell line is known to have high Notch signaling, which the model correctly predicted (group 1). Cells were treated for 48 hours with 5 $\mu$M DAPT, a gamma-secretase inhibitor (GSI) (group 2). GSIs are known to inhibit Notch signaling and the model correctly detected a decrease in Notch activity in this group (see Dohda T. et al., "Notch signaling induces SKP2 expression and promotes reduction of p27Kip1 in T-cell acute lymphoblastic leukemia cell", Experimental Cell Research, Vol. 313, No. 14, August 2007, pages 3141 to 3152).

Fig. 13 shows Notch cellular signaling pathway activity predictions of the trained exemplary Bayesian network model using the 18 target genes shortlist from Table 2 on IMR32 cells that were transfected with an inducible Notch3-intracellular construct. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Two independent single-cell derived clones (c6, c8) are shown which drive Notch3-intracellular expression in the presence of 50 ng/mL doxycycline. At t=0 hr, for both clones the trained exemplary Bayesian network model using the 18 target genes shortlist from Table 2 detects low Notch activity. After induction of Notch3-intracellular, we correctly observe that Notch activity goes up in both clones and stabilizes at t = 24 hrs (data set GSE16477, van Nes J. et al., "A NOTCH3 Transcriptional Module Induces Cell Motility in Neuroblastoma", Clinical Cancer Research, Vol. 19, No. 13, July 2013, pages 3485 to 3494).

Fig. 14 shows Notch cellular signaling pathway activity predictions of the trained exemplary Bayesian network model using the 18 target genes shortlist from Table 2 on CD34+CD45RA-Lin-HPCs that were cultured for 72 hrs with graded doses of plastic-immobilized Notch ligand Deltalext-IgG (data set GSE29524). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The trained exemplary Bayesian network model using the 18 target genes shortlist from Table 2 correctly predicts higher Notch activity in the cells cultured on Deltalext-IgG (group 2) compared to the control (group 1).

Fig. 15 shows Notch cellular signaling pathway activity predictions of the trained exemplary Bayesian network model using the 18 target genes shortlist from Table 2 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the 18 target genes shortlist from Table 2 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36,2011, pages 14908 to 14913).

Fig. 16 shows Notch cellular signaling pathway activity predictions of the trained exemplary Bayesian network model using the evidence curated list of target genes (26 target gene list) from Table 1 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the evidence curated list of target genes (26 target gene list) from Table 1 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36,2011, pages 14908 to 14913).

Fig. 17 shows Notch cellular signaling pathway activity predictions of the trained exemplary Bayesian network model using the 18 target genes shortlist from Table 2 on HUVEC cells that were transfected with COUP-TFII siRNA (data set GSE33301). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. COUP-TFII is known to repress Notch signaling (see You L.R. et al., "Suppression of Notch signaling by the COUP-TFII transcription factor regulates vein identity", Vol. 435, No. 7038, May 2005, pages 98 to 104). The trained exemplary Bayesian network model using the 18 target genes shortlist from Table 2 correctly detects higher Notch activity in COUP-TFII siRNA transfected cells (group 2) compared to control cells (group 1) (see Chen X. et al., "COUP-TFII is a major regulator of cell cycle and Notch signaling pathways", Molecular Endocrinology, Vol. 26, No. 8, August 2012, pages 1268 to 1277).

Fig. 18 shows Notch cellular signaling pathway activity predictions of the trained exemplary Bayesian network model using the 18 target genes shortlist on breast cancer subgroups in samples from GSE6532, GSE9195, GSE12276, GSE20685, GSE21653 and EMTAB365. In the diagram, the vertical axis indicates the odds that the TF element is "present" resp. "absent", which corresponds to the Notch cellular signaling pathway being active resp. passive. It is observed that Notch activity is high in all breast cancer samples in those data sets. Results of doing a one-way ANOVA followed by a Games-Howell post-hoc test show that almost all groups have significant differences except for NormL vs. Basal and LumA vs. HER2, see Table 4. (subgroups: Basal, HER2, LumA = Luminal A, LumB = Luminal B, NormL = Normal-like)

Table 4: Results of Games-Howell post-hoc test comparing different subgroups of breast cancer samples as shown in Fig. 18. p-values < 0.05 are considered to be significant.

| Comparison | p adj |
|---|---|
| HER2-Basal | 2.2e-04 |
| LumA-Basal | 7.0e-08 |
| LumB-Basal | 9.2e-10 |
| NormL-Basal | 1 |
| LumA-HER2 | 1 |
| LumB-HER2 | 1.5e-03 |
| NormL-HER2 | 5.6e-03 |
| LumB-LumA | 1.5e-03 |
| NormL-LumA | 2.6e-04 |
| NormL-LumB | 3.2e-09 |

Table 5 shows results of Cox regression on Notch activity for the trained exemplary Bayesian network model using the 18 target genes shortlist on data sets as used in Fig. 18. For all samples together and more specifically for Luminal A end Luminal B there is a significantly worse prognosis with increasing Notch activity predicted by our model. This is supported by a recent publication in which it was found that patients testing positive for Notch1 had shorter disease-free survival (see Zhong Y. et al., "NOTCH1 is a Poor Prognostic Factor for Breast Cancer and Is Associated With Breast Cancer Stem Cells", Oncotargets and Therapy, Vol. 9, November 2016, pages 6865 to 6871).

Table 5: Results of Cox regression on Notch activity for the trained exemplary Bayesian network model using the 18 target genes shortlist from Table 2 on data sets as used in Fig. 18.

| | Cox's coef | HR | se(Cox's coef) | z | p |
|---|---|---|---|---|---|
| **All** | 0.0593 | 1.061093 | 0.015547 | 3.814204 | 0.000137 |
| **Basal** | -0.00439 | 0.995624 | 0.036854 | -0.11899 | 0.905283 |
| **HER2** | 0.085358 | 1.089107 | 0.04685 | 1.821967 | 0.06846 |
| **LumA** | 0.075129 | 1.078023 | 0.036091 | 2.081647 | 0.037375 |
| **LumB** | 0.076441 | 1.079439 | 0.024199 | 3.158812 | 0.001584 |
| **NormL** | 0.080338 | 1.083653 | 0.054621 | 1.470822 | 0.141339 |

Fig. 19 shows Notch cellular signaling pathway activity predictions of the trained exemplary Bayesian network model using the 12 target genes shortlist from Table 3 on CD34+CD45RA-Lin-HPCs that were cultured for 72 hrs with graded doses of plastic-immobilized Notch ligand Delta1 ext-IgG (data set GSE29524). In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. The trained exemplary Bayesian network model using the 12 target genes shortlist from Table 3 correctly predicts higher Notch activity in the cells cultured on Deltalext-IgG (group 2) compared to the control (group 1).

Fig. 20 shows Notch cellular signaling pathway activity predictions of the trained exemplary Bayesian network model using the 12 target genes shortlist from Table 3 on CUTLL1 cells, which are known to have high Notch activity. In the diagram, the vertical axis indicates the odds that the TF element is "present" resp. "absent", which corresponds to the Notch cellular signaling pathway being active resp. passive, wherein values above the horizontal axis correspond to the TF element being more likely "present"/active and values below the horizontal axis indicate that the odds that the TF element is "absent"/passive are larger than the odds that it is "present"/active. Treatment with a gamma-secretase inhibitor (GSI) inhibits Notch signaling. In data set GSE29544, it was observed that Notch activity is high 2 hours after a GSI washout. In this figure data from untreated CUTLL1 cells and CUTLL1 cells after GSI washout are pooled, since in both cases Notch activity is expected to be high. Six groups can be distinguished: 1) Untreated CUTLL1 cells and CUTLL1 cells after GSI washout. Here, the trained exemplary Bayesian network model using the 18 target genes shortlist correctly predicts high Notch activity in this group. 2) GSI treated CUTLL1 cells for which the model correctly predicts low Notch activity. 3+4) CUTLL1 cells treated with an empty MigRI retrovirus, which is not expected to affect Notch signaling. Here, the trained exemplary Bayesian network model using the 12 target genes shortlist from Table 3 correctly predicts high Notch activity for cells after GSI washout (group 3) and GSI treated cells (group 4). 5+6) CUTLL cells transduced with MigRI-dominant negative MAML1 virus. DNMAML1 is a Notch antagonist and Notch signaling is expected to be low in these cells. The model correctly predicts low Notch activity for both the cells after GSI washout (group 5) as for GSI treated cells (group 6) (see Wang H. et al., "Genome-wide analysis reveals conserved and divergent features of Notch1/RBPJ binding in human and murine T-lymphoblastic leukemia cells", Proceedings of the National Academy of Sciences of the USA, Vol. 108, No. 36, 2011, pages 14908 to 14913).

Fig. 21 shows the correlation between the trained exemplary Bayesian network mode using the evidence curated list of target genes (26 target genes list) from Table 1 and the 12 target genes shortlist from Table 3, respectively. In the diagram, the horizontal axis indicates the odds (on a log2 scale) that the TF element is "present" resp. "absent", which corresponds to the Notch cellular signaling pathway being active resp. passive, as predicted by the trained exemplary Bayesian network model using the evidence curated list of target genes (26 target genes list) from Table 1. The vertical axis indicates the same information, as predicted by the trained exemplary Bayesian network model using the 12 target gene shortlist from Table 3 (data sets GSE5682, GSE5716, GSE6495, GSE9339, GSE14995, GSE15947, GSE16477, GSE16906, GSE18198, GSE20011, GSE20285, GSE20667, GSE24199, GSE27424, GSE29524, GSE29544, GSE29850, GSE29959, GSE32375, GSE33301, GSE33562, GSE34602, GSE35340, GSE36176, GSE37645, GSE39223, GSE42259, GSE46909, GSE49673, GSE53537, GSE54378, GSE57022, GSE61827, GSE74996, GSE81156, GSE82298). The two models are significantly correlated with a p-value of 2.2e-16 and a correlation coefficient of 0.929.

Figs. 22 and 23 show additional comparisons of Notch cellular signaling pathway activity predictions from a trained exemplary Bayesian network mode using (i) a list of 7 Notch target genes (DTX1, HES1, HES4, HES5, HEY2, MYC, and NRARP) and a list of 10 Notch target genes (the 7 Notch target genes plus EPHB3, SOX9, and NFKB2), and (ii) a list of 8 Notch target genes (DTX1, HES1, HES4, HES5, HEY2, MYC, NRARP, and PTCRA) and a list of 12 Notch target genes (the 8 Notch target genes plus HEYL, HEY1, PLXND1, and GFAP). The 7 Notch target genes are included in each of the lists of target genes from Tables 1 to 3 and the 8 Notch target genes include an additional target gene (PTCRA) that is only included in the evidence curated list of target genes (26 target genes list) from Table 1. The 3 additional target genes of the list of 10 Notch target genes were taken from the 12 target genes shortlist from Table 3 and the 4 additional target genes of the list of 12 Notch target genes, which differ from the 3 additional target genes, were taken from the evidence curated list of target genes (26 target genes list) from Table 1. The comparisons exemplarily show that the Notch cellular signaling pathway activity predictions from the trained exemplary Bayesian network mode using a list of 7 Notch target genes which is a subset of each of the lists of target genes from Tables 1 to 3, and a list of 8 Notch target genes, which is a subset of the evidence curated list of target genes (26 target genes list) from Table 1, can be further improved by adding additional target genes from the respective lists. In detail:

Fig. 22 shows a comparison of the Notch cellular signaling pathway activity predictions using the list of 7 Notch target genes vs. the list of 10 Notch target genes. The models were run on samples from IMR32 cells that were transfected with an inducible Notch3-intracellular construct. In the diagram, the horizontal axis indicates time in hours and the vertical axis indicates the relative Notch cellular signaling pathway activity (on a log2odds scale). Both models correctly show the expected increase in Notch activity after induction of the Notch3-intracellular construct. The 10-target gene model (stippled line), however, shows a bigger increase in activity compared to the 7-target gene model (solid line). The Notch activity has been set to 0 at t=0 hours, to make comparison easier (data set GSE16477, see also van Nes J. et al., "A NOTCH3 Transcriptional Module Induces Cell Motility in Neuroblastoma", Clinical Cancer Research, Vol. 19, No. 13, July 2013, pages 3485 to 3494).

Fig. 23 shows a comparison of the Notch cellular signaling pathway activity predictions using the list of 8 Notch target genes vs. the list of 12 Notch target genes. The models were run on samples from endometrial stromal cells that were infected by a Jag1 retrovirus (data set GSE16906). Jag1 is a Notch ligand which induces cleavage of the Notch receptor upon binding, thereby ultimately inducing Notch target gene transcription. The 12-target gene model (right side of the graph) shows a better separation of the Notch activity (given on the vertical axis as log2odds) between control ("C" in the figure) and Jag1 infected cells ("Jag1 INF" in the figure) compared to the 8-target gene model (left side of the graph) (see also Mikhailik A. et al. "Notch ligand-dependent gene expression in human endometrial stromal cells", Biochemical and Biophysical Research Communications, Vol. 388, No. 3, October 2009, pages 479 to 482).

Instead of applying the calibrated mathematical model, e.g., the exemplary Bayesian network model, on mRNA input data coming from microarrays or RNA sequencing, it may be beneficial in clinical applications to develop dedicated assays to perform the sample measurements, for instance on an integrated platform using qPCR to determine mRNA levels of target genes. The RNA/DNA sequences of the disclosed target genes can then be used to determine which primers and probes to select on such a platform.

Validation of such a dedicated assay can be done by using the microarray-based mathematical model as a reference model, and verifying whether the developed assay gives similar results on a set of validation samples. Next to a dedicated assay, this can also be done to build and calibrate similar mathematical models using RNA sequencing data as input measurements.

The set of target genes which are found to best indicate specific cellular signaling pathway activity, e.g., Tables 1 to 3, based on microarray/RNA sequencing based investigation using the calibrated mathematical model, e.g., the exemplary Bayesian network model, can be translated into a multiplex quantitative PCR assay to be performed on a sample of the subject and/or a computer to interpret the expression measurements and/or to infer the activity of the Notch cellular signaling pathway. To develop such a test (e.g., FDA-approved or a CLIA waived test in a central service lab or a laboratory developed test for research use only) for cellular signaling pathway activity, development of a standardized test kit is required, which needs to be clinically validated in clinical trials to obtain regulatory approval.

The present invention relates to a computer-implemented method for inferring activity of a Notch cellular signaling pathway in a subject performed by a digital processing device, wherein the inferring is based on expression levels of three or more target genes of the Notch cellular signaling pathway measured in a sample of the subject. The present invention further relates to an apparatus for inferring activity of a Notch cellular signaling pathway in a subject comprising a digital processor configured to perform the method, to a non-transitory storage medium for inferring activity of a Notch cellular signaling pathway in a subject storing instructions that are executable by a digital processing device to perform the method, and to a computer program for inferring activity of a Notch cellular signaling pathway in a subject comprising program code means for causing a digital processing device to perform the method, when the computer program is run on the digital processing device.

The method may be used, for instance, in diagnosing an (abnormal) activity of the Notch cellular signaling pathway, in prognosis based on the inferred activity of the Notch cellular signaling pathway, in the enrollment of a subject in a clinical trial based on the inferred activity of the Notch cellular signaling pathway, in the selection of subsequent test(s) to be performed, in the selection of companion diagnostics tests, in clinical decision support systems, or the like. In this regard, reference is made to the published international patent application WO 2013/011479 A2 ("Assessment of cellular signaling pathway activity using probabilistic modeling of target gene expression"), to the published international patent application WO 2014/102668 A2 ("Assessment of cellular signaling pathway activity using linear combination(s) of target gene expressions"), and to Verhaegh W. et al., "Selection of personalized patient therapy through the use of knowledge-based computational models that identify tumor-driving signal transduction pathways", Cancer Research, Vol. 74, No. 11, 2014, pages 2936 to 2945, which describe these applications in more detail.

**Example 4: Further information for illustrating the present invention**

(1) Measuring Levels of gene expression

Data derived from the unique set of target genes described herein is further utilized to infer an activity of the Notch cellular signaling pathway using the methods described herein.

Methods for analyzing gene expression levels in extracted samples are generally known. For example, methods such as Northern blotting, the use of PCR, nested PCR, quantitative real-time PCR (qPCR), RNA-seq, or microarrays can all be used to derive gene expression level data. All methods known in the art for analyzing gene expression of the target genes are contemplated herein.

Methods of determining the expression product of a gene using PCR based methods may be of particular use. In order to quantify the level of gene expression using PCR, the amount of each PCR product of interest is typically estimated using conventional quantitative real-time PCR (qPCR) to measure the accumulation of PCR products in real time after each cycle of amplification. This typically utilizes a detectible reporter such as an intercalating dye, minor groove binding dye, or fluorogenic probe whereby the application of light excites the reporter to fluoresce and the resulting fluorescence is typically detected using a CCD camera or photomultiplier detection system, such as that disclosed in U.S. Pat. No. 6,713,297 which is hereby incorporated by reference.

In some embodiments, the probes used in the detection of PCR products in the quantitative real-time PCR (qPCR) assay can include a fluorescent marker. Numerous fluorescent markers are commercially available. For example, Molecular Probes, Inc. (Eugene, Oreg.) sells a wide variety of fluorescent dyes. Non-limiting examples include Cy5, Cy3, TAMRA, R6G, R110, ROX, JOE, FAM, Texas Red™, and Oregon Green™. Additional fluorescent markers can include IDT ZEN Double-Quenched Probes with traditional 5' hydrolysis probes in qPCR assays. These probes can contain, for example, a 5' FAM dye with either a 3' TAMRA Quencher, a 3' Black Hole Quencher (BHQ, Biosearch Technologies), or an internal ZEN Quencher and 3' Iowa Black Fluorescent Quencher (IBFQ).

Fluorescent dyes useful according to the invention can be attached to oligonucleotide primers using methods well known in the art. For example, one common way to add a fluorescent label to an oligonucleotide is to react an N-Hydroxysuccinimide (NHS) ester of the dye with a reactive amino group on the target. Nucleotides can be modified to carry a reactive amino group by, for example, inclusion of an allyl amine group on the nucleobase. Labeling via allyl amine is described, for example, in U.S. Pat. Nos. 5,476,928 and 5,958,691, which are incorporated herein by reference. Other means of fluorescently labeling nucleotides, oligonucleotides and polynucleotides are well known to those of skill in the art.

Other fluorogenic approaches include the use of generic detection systems such as SYBR-green dye, which fluoresces when intercalated with the amplified DNA from any gene expression product as disclosed in U.S. Pat. Nos. 5,436,134 and 5,658,751 which are hereby incorporated by reference.

Another useful method for determining target gene expression levels includes RNA-seq, a powerful analytical tool used for transcriptome analyses, including gene expression level difference between different physiological conditions, or changes that occur during development or over the course of disease progression.

Another approach to determine gene expression levels includes the use of microarrays for example RNA and DNA microarray, which are well known in the art. Microarrays can be used to quantify the expression of a large number of genes simultaneously.

(2) Generalized workflow for determining the activity of Notch cellular signaling

A flowchart exemplarily illustrating a process for inferring the activity of Notch cellular signaling from a sample isolated from a subject is shown in Fig. 3. First, the mRNA from a sample is isolated (11). Second, the mRNA expression levels of a unique set of at least three or more Notch target genes, as described herein, are measured (12) using methods for measuring gene expression that are known in the art. Next, an activity level of a Notch transcription factor (TF) element (13) is determined using a calibrated mathematical pathway model (14) relating the expression levels of the three or more Notch target genes to the activity level of the Notch TF element. Finally, the activity of the Notch cellular signaling pathway in the subject is inferred (15) based on the determined activity level of the Notch TF element in the sample of the subject. For example, the Notch cellular signaling pathway is determined to be active if the activity is above a certain threshold, and can be categorized as passive if the activity falls below a certain threshold.

(3) Calibrated mathematical pathway model

As contemplated herein, the expression levels of the unique set of three or more Notch target genes described herein are used to determine an activity level of a Notch TF element using a calibrated mathematical pathway model as further described herein. The calibrated mathematical pathway model relates the expression levels of the three or more Notch target genes to the activity level of the Notch TF element.

As contemplated herein, the calibrated mathematical pathway model is based on the application of a mathematical pathway model. For example, the calibrated mathematical pathway model can be based on a probabilistic model, for example, a Bayesian network model, or a linear or pseudo-linear model.

In an embodiment, the calibrated mathematical pathway model is a probabilistic model incorporating conditional probabilistic relationships relating the Notch TF element and the expression levels of the three or more Notch target genes. In an embodiment, the probabilistic model is a Bayesian network model.

In an alternative embodiment, the calibrated pathway mathematical model can be a linear or pseudo-linear model. In an embodiment, the linear or pseudo-linear model is a linear or pseudo-linear combination model as further described herein.

A flowchart exemplarily illustrating a process for generating a calibrated mathematical pathway model is shown in Fig. 4. As an initial step, the training data for the mRNA expression levels is collected and normalized. The data can be collected using, for example, microarray probeset intensities (101), real-time PCR Cq values (102), raw RNAseq reads (103), or alternative measurement modalities (104) known in the art. The raw expression level data can then be normalized for each method, respectively, by normalization using a normalization algorithm, for example, frozen robust multiarray analysis (fRMA) or MAS5.0 (111), normalization to average Cq of reference genes (112), normalization of reads into reads/fragments per kilobase of transcript per million mapped reads (RPKM/FPKM) (113), or normalization w.r.t. reference genes/proteins (114). This normalization procedure leads to a normalized probeset intensity (121), normalized Cq values (122), normalized RPKM/FPKM (123), or normalized measurement (124) for each method, respectively, which indicate target gene expression levels within the training samples.

Once the training data has been normalized, a training sample ID or IDs (131) is obtained and the training data of these specific samples is obtained from one of the methods for determining gene expression (132). The final gene expression results from the training sample are output as training data (133). All of the data from various training samples are incorporated to calibrate the model (including for example, thresholds, CPTs, for example in the case of the probabilistic or Bayesian network, weights, for example, in the case of the linear or pseudo-linear model, etc) (144). In addition, the pathway's target genes and measurement nodes (141) are used to generate the model structure for example, as described in Fig. 2 (142). The resulting model structure (143) of the pathway is then incorporated with the training data (133) to calibrate the model (144), wherein the gene expression levels of the target genes is indicative of the transcription factor element activity. As a result of the TF element determination in the training samples, a calibrated pathway model (145) is generated, which assigns the Notch cellular signaling pathway activity for a subsequently examined sample of interest, for example from a subject with a cancer, based on the target gene expression levels in the training samples.

(4) TF element determination

A flowchart exemplarily illustrating a process for determining an activity level of a TF element is shown in Fig. 5. The expression level data (test data) (163) from a sample extracted from a subject is input into the calibrated mathematical pathway model (145). The mathematical pathway model may be a probabilistic model, for example, a Bayesian network model, a linear model, or a pseudo-linear model.

The mathematical pathway model may be a probabilistic model, for example, a Bayesian network model, based on conditional probabilities relating the Notch TF element and expression levels of the three or more target genes of the Notch cellular signaling pathway measured in the sample of the subject, or the mathematical model may be based on one or more linear combination(s) of expression levels of the three or more target genes of the Notch cellular signaling pathway measured in the sample of the subject. In particular, the determining of the activity of the Notch cellular signaling pathway may be performed as disclosed in the published international patent application WO 2013/011479 A2 ("Assessment of cellular signaling pathway activity using probabilistic modeling of target gene expression"), the contents of which are herewith incorporated in their entirety. Briefly, the data is entered into a Bayesian network (BN) inference engine call (for example, a BNT toolbox) (154). This leads to a set of values for the calculated marginal BN probabilities of all the nodes in the BN (155). From these probabilities, the transcription factor (TF) node's probability (156) is determined and establishes the TF element's activity level (157).

Alternatively, the mathematical model may be a linear model. For example, a linear model can be used as described in the published international patent application WO 2014/102668 A2 ("Assessment of cellular signaling pathway activity using linear combination(s) of target gene expressions"), the contents of which are herewith incorporated in their entirety. Further details regarding the calculating/determining of cellular signaling pathway activity using mathematical modeling of target gene expression can also be found in Verhaegh W. et al., "Selection of personalized patient therapy through the use of knowledge-based computational models that identify tumor-driving signal transduction pathways", Cancer Research, Vol. 74, No. 11, 2014, pages 2936 to 2945. Briefly, the data is entered into a calculated weighted linear combination score (w/c) (151). This leads to a set of values for the calculated weighted linear combination score (152). From these weighted linear combination scores, the transcription factor (TF) node's weighted linear combination score (153) is determined and establishes the TF's element activity level (157).

(5) Procedure for discretized observables

A flowchart exemplarily illustrating a process for inferring activity of a Notch cellular signaling pathway in a subject as a discretized observable is shown in Fig. 6. First, the test sample is extracted and given a test sample ID (161). Next, the test data for the mRNA expression levels is collected and normalized (162). The test data can be collected using the same methods as discussed for the training samples in Fig. 5, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA or MAS5.0 (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in a thresholding step (164) based on the calibrated mathematical pathway model (145), resulting in the thresholded test data (165). In using discrete observables, in one non-limiting example, every expression above a certain threshold is, for example, given a value of 1 and values below the threshold are given a value of 0, or in an alternative embodiment, the probability mass above the threshold as described herein is used as a thresholded value. Based on the calibrated mathematical pathway model, this value represents the TF element's activity level (157), which is then used to calculate the cellular signaling pathway's activity (171). The final output gives the cellular signaling pathway's activity (172) in the subject.

(6) Procedure for continuous observables

A flowchart exemplarily illustrating a process for inferring activity of a Notch cellular signaling pathway in a subject as a continuous observable is shown in Fig. 7. First, the test sample is extracted and given a test sample ID (161). Next, the test data for the mRNA expression levels is collected and normalized (162). The test data can be collected using the same methods as discussed for the training samples in Figure 5, using microarray probeset intensities (101), real-time PCR Cq values (102), raw RNAseq reads (103), or an alternative measurement modalities (104). The raw expression level data can then be normalized for each method, respectively, by normalization using an algorithm, for example fRMA (111), normalization to average Cq of reference genes (112), normalization of reads into RPKM/FPKM (113), and normalization w.r.t. reference genes/proteins (114). This normalization procedure leads to a normalized probeset intensity (121), normalized Cq values (122), normalized RPKM/FPKM (123), or normalized measurement (124) for each method, respectively.

Once the test data has been normalized, the resulting test data (163) is analyzed in the calibrated mathematical pathway model (145). In using continuous observables, as one non-limiting example, the expression levels are converted to values between 0 and 1 using a sigmoid function as described in further detail herein. The TF element determination as described herein is used to interpret the test data in combination with the calibrated mathematical pathway model, the resulting value represents the TF element's activity level (157), which is then used to calculate the cellular signaling pathway's activity (171). The final output gives the cellular signaling pathway's activity (172) in the subject.

(7) Target gene expression level determination procedure

A flowchart exemplary illustrating a process for deriving target gene expression levels from a sample extracted from a subject is shown in Fig. 8. In an exemplary embodiment, samples are received and registered in a laboratory. Samples can include, for example, Formalin-Fixed, Paraffin-Embedded (FFPE) samples (181) or fresh frozen (FF) samples (180). FF samples can be directly lysed (183). For FFPE samples, the paraffin can be removed with a heated incubation step upon addition of Proteinase K (182). Cells are then lysed (183), which destroys the cell and nuclear membranes which makes the nucleic acid (NA) available for further processing. The nucleic acid is bound to a solid phase (184) which could for example, be beads or a filter. The nucleic acid is then washed with washing buffers to remove all the cell debris which is present after lysis (185). The clean nucleic acid is then detached from the solid phase with an elution buffer (186). The DNA is removed by DNAse treatment to ensure that only RNA is present in the sample (187). The nucleic acid sample can then be directly used in the RT-qPCR sample mix (188). The RT-qPCR sample mixes contains the RNA sample, the RT enzyme to prepare cDNA from the RNA sample and a PCR enzyme to amplify the cDNA, a buffer solution to ensure functioning of the enzymes and can potentially contain molecular grade water to set a fixed volume of concentration. The sample mix can then be added to a multiwell plate (i.e., 96 well or 384 well plate) which contains dried RT-qPCR assays (189). The RT-qPCR can then be run in a PCR machine according to a specified protocol (190). An example PCR protocol includes i) 30 minutes at 50°C; ii) 5 minutes at 95°C; iii) 15 seconds at 95°C; iv) 45 seconds at 60°C; v) 50 cycles repeating steps iii and iv. The Cq values are then determined with the raw data by using the second derivative method (191). The Cq values are exported for analysis (192).

(8) Notch Mediated diseases and disorders and methods of treatment

As contemplated herein, the methods and apparatuses of the present invention can be utilized to assess Notch cellular signaling pathway activity in a subject, for example, a subject suspected of having, or having, a disease or disorder wherein the status of the Notch signaling pathway is probative, either wholly or partially, of disease presence or progression. In an embodiment, provided herein is a method of treating a subject comprising receiving information regarding the activity status of a Notch cellular signaling pathway derived from a sample extracted from the subject using the methods described herein and administering to the subject a Notch inhibitor if the information regarding the activity of the Notch cellular signaling pathway is indicative of an active Notch signaling pathway. In a particular embodiment, the Notch cellular signaling pathway activity indication is set at a cutoff value of odds of the Notch cellular signaling pathway being active of 10:1, 5:1, 4:1, 2:1, 1:1, 1:2, 1:4, 1:5, 1:10.

Notch inhibitors that may be used in the present invention are well known. Examples of Notch inhibitors include, but are not limited to, DAPT, PF-03084014, MK-0752, RO-4929097, LY450139, BMS-708163, LY3039478, IMR-1, Dibenzazepine, LY411575, FLI-06.

The Notch pathway plays a role in a large number of diseases, and notably in different types of neoplasms, e.g., carcinomas, sarcomas and hematological malignancies, immune-mediated diseases, degenerative diseases, inflammatory diseases, infectious diseases. These can be categorized according to the embryonic lineage-derived organ or tissue in which they mainly occur, for example, brain, breast, skin, esophagus, gastrointestinal tract, blood (hematological), ovarian, etc.

In a particular embodiment, the subject is suffering, or suspected to be suffering from, a breast cancer, lung cancer, a colon cancer, pancreatic cancer, brain cancer, hematological cancer, ovarian cancer. In a particular embodiment, the subject is suffering from, or suspected to be suffering from, a breast cancer.

In another particular embodiment, the subject is suffering from, or suspected to be suffering from, a brain cancer or more preferred a neuroblastoma cancer. In another particular embodiment, the subject is suffering form, or suspected to be suffering from, a hematological cancer or more preferred a T-cell lymphoblastic leukemia.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the risk score performed by one or several units or devices can be performed by any other number of units or devices.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

**Example 5: Sequence Listings Used in Application**

SEQUENCE LISTING:

| Seq. No. | Gene: |
|---|---|
| Seq. 1 | CD28 |
| Seq. 2 | CD44 |
| Seq. 3 | DLGAP5 |
| Seq. 4 | DTX1 |
| Seq. 5 | EPHB3 |
| Seq. 6 | FABP7 |
| Seq. 7 | GFAP |
| Seq. 8 | GIMAP5 |
| Seq. 9 | HES1 |
| Seq. 10 | HES4 |
| Seq. 11 | HES5 |
| Seq. 12 | HES7 |
| Seq. 13 | HEY1 |
| Seq. 14 | HEY2 |
| Seq. 15 | HEYL |
| Seq. 16 | KLF5 |
| Seq. 17 | MYC |
| Seq. 18 | NFKB2 |
| Seq. 19 | NOX1 |
| Seq. 20 | NRARP |
| Seq. 21 | PBX1 |
| Seq. 22 | PIN1 |
| Seq. 23 | PLXND1 |
| Seq. 24 | PTCRA |
| Seq. 25 | SOX9 |
| Seq. 26 | TNC |

## Claims

1. A computer-implemented method for inferring activity of a Notch cellular signaling pathway in a subject performed by a digital processing device, wherein the inferring comprises:

 receiving expression levels of three or more target gene(s) of the Notch cellular signaling pathway measured in a sample of the subject,
 determining an activity level of a Notch transcription factor (TF) element in the sample of the subject, the Notch TF element controlling transcription of the three or more Notch target gene(s), the determining being based on evaluating a calibrated mathematical pathway model relating the expression levels of the three or more Notch target gene(s) to the activity level of the Notch TF element, and
 inferring the activity of the Notch cellular signaling pathway in the subject based on the determined activity level of the Notch TF element in the sample of the subject,
 wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

2. The method of claim 1, wherein the three or more Notch target gene(s) are selected from the group consisting of: CD44, DTX1, EPHB3, HES1, HES4, HES5, HES7, HEY1, HEY2, HEYL, MYC, NFKB2, NOX1, NRARP, PBX1, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: CD44, EPHB3, HES7, HEY1, HEYL, NFKB2, NOX1, PBX1, PIN1, PLXND1, and SOX9.

3. The method of claim 1 or 2, wherein the three or more Notch target gene(s) are selected from the group consisting of: DTX1, EPHB3, HES1, HES4, HES5, HEY2, MYC, NFKB2, NRARP, PIN1, PLXND1, and SOX9, preferably, wherein two or more Notch target gene(s) are selected from the group consisting of: DTX1, HES1, HES4, HES5, HEY2, MYC, and NRARP, and one or more Notch target gene(s) are selected from the group consisting of: EPHB3, NFKB2, PIN1, PLXND1, and SOX9.

4. The method of any of claims 1 to 3, further comprising:

 determining whether the Notch cellular signaling pathway is operating abnormally in the subject based on the inferred activity of the Notch cellular signaling pathway in the subject.

5. The method of claim 4, further comprising:

 recommending prescribing a drug for the subject that corrects for abnormal operation of the Notch cellular signaling pathway,
 wherein the recommending is performed if the Notch cellular signaling pathway is determined to be operating abnormally in the subject based on the inferred activity of the Notch cellular signaling pathway.

6. The method of claim 4 or 5, wherein the abnormal operation of the Notch cellular signaling pathway is an operation in which the Notch cellular signaling pathway operates as a tumor promoter in the subject.

7. The method of any of claims 1 to 6, wherein the method is used in at least one of the following activities:

diagnosis based on the inferred activity of the Notch cellular signaling pathway in the subject;
prognosis based on the inferred activity of the Notch cellular signaling pathway in the subject;
drug prescription based on the inferred activity of the Notch cellular signaling pathway in the subject;
prediction of drug efficacy based on the inferred activity of the Notch cellular signaling pathway in the subject;
prediction of adverse effects based on the inferred activity of the Notch cellular signaling pathway in the subject;
monitoring of drug efficacy;
drug development;
assay development;
pathway research;
cancer staging;
enrollment of the subject in a clinical trial based on the inferred activity of the Notch cellular signaling pathway in the subject;
selection of subsequent test to be performed; and
selection of companion diagnostics tests.

8. The method of any of claims 1 to 7, wherein the calibrated mathematical pathway model is a probabilistic model, preferably a Bayesian network model, based on conditional probabilities relating the activity level of the Notch TF element and the expression levels of the three or more Notch target genes, or wherein the mathematical pathway model is based on one or more linear combination(s) of the expression levels of the three or more Notch target genes.

9. An apparatus for inferring activity of a Notch cellular signaling pathway in a subject comprising a digital processor configured to perform the method of any of claims 1 to 8.

10. A non-transitory storage medium for inferring activity of a Notch cellular signaling pathway in a subject storing instructions that are executable by a digital processing device to perform the method of any of claims 1 to 8.

11. A computer program for inferring activity of a Notch cellular signaling pathway in a subject comprising program code means for causing a digital processing device to perform the method of any of claims 1 to 8, when the computer program is run on the digital processing device.

12. A kit for measuring expression levels of three or more target gene(s) of the

polymerase chain reaction primers directed to the three or more Notch target gene(s),
probes directed to the three or more Notch target gene(s),
wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

13. A kit for inferring activity of a Notch cellular signaling pathway in a subject, comprising:

the kit of claim 12, and
the apparatus of claim 9, the non-transitory storage medium of claim 10, or the computer program of claim 11.

14. A kit for inferring activity of a Notch cellular signaling pathway in a subject, comprising:

one or more components for determining expression levels of three or more target gene(s) of the Notch cellular signaling pathway in a sample of the subject,
wherein the one or more components are preferably selected from the group consisting of: a DNA array chip, an oligonucleotide array chip, a protein array chip, an antibody, a plurality of probes, for example, labeled probes, a set of RNA reverse-transcriptase sequencing components, and/or RNA or DNA, including cDNA, amplification primers,
wherein the three or more Notch target gene(s) are selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target gene(s) are selected from the group consisting of: DTX1, HES1, HES4, HES5, HEY2, MYC, NRARP, and PTCRA, and one or more Notch target gene(s) are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC.

**15.** Use of the kit of any of claims 12 to 14 in performing the method of any of claims 1 to 8.

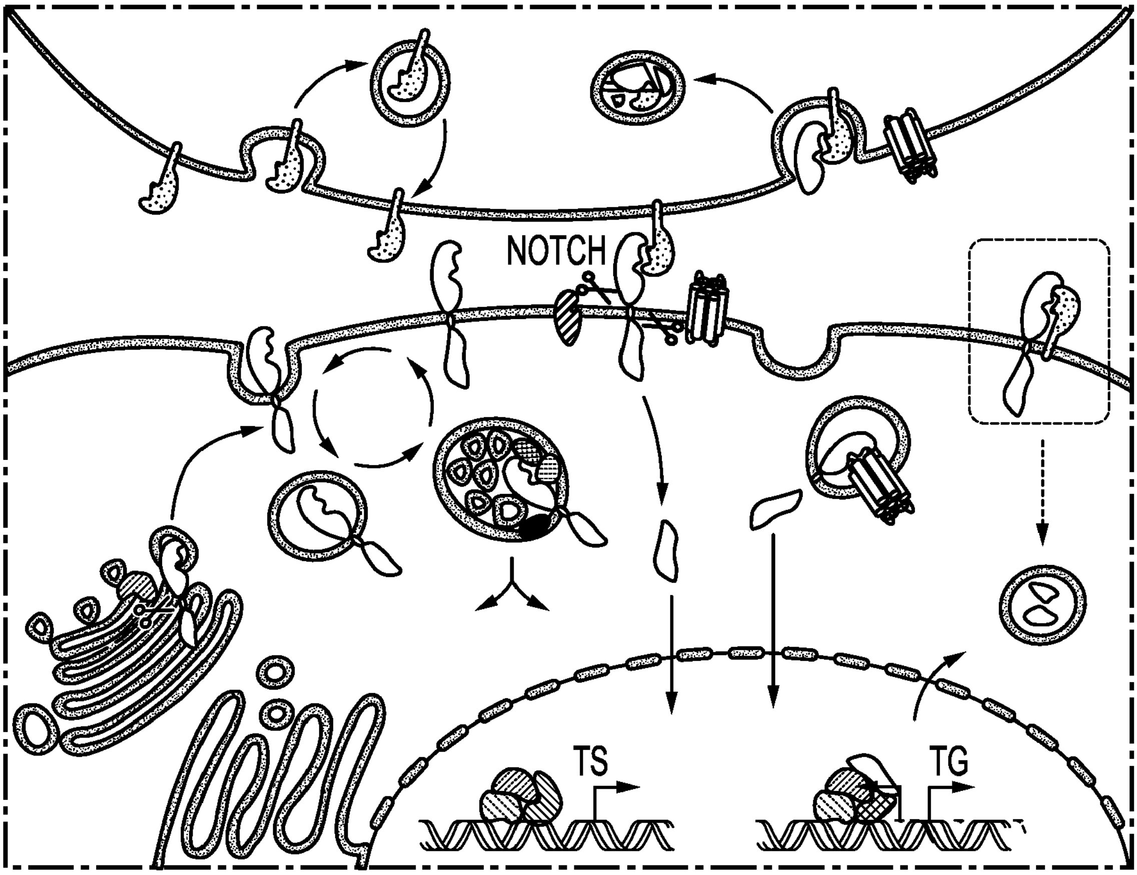


FIG. 1

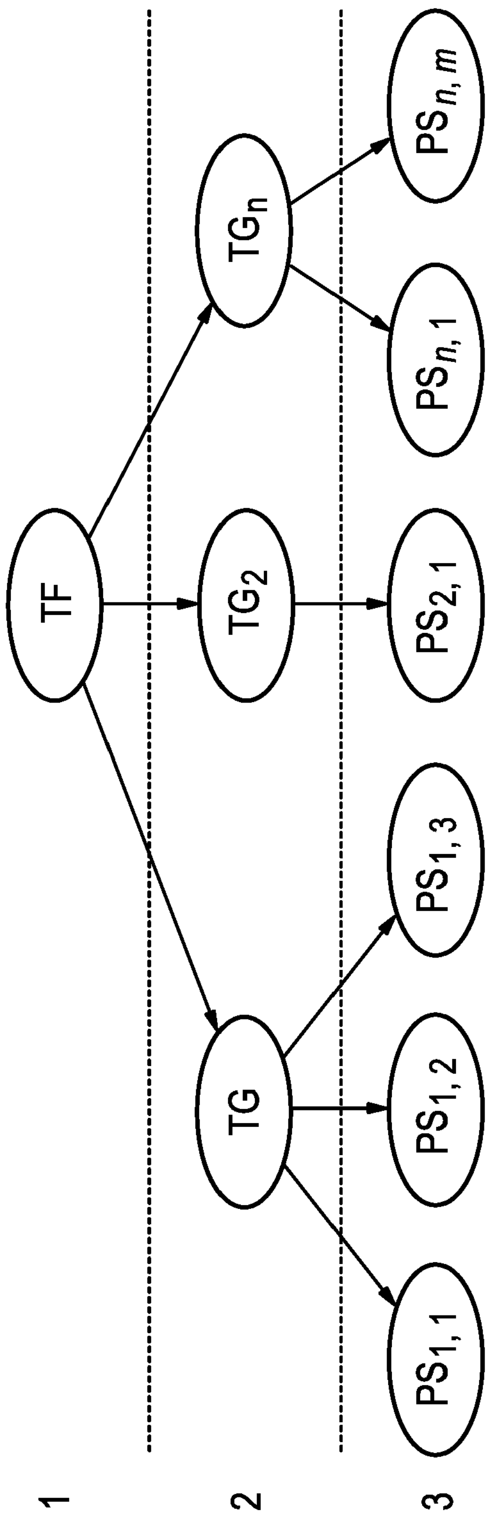


FIG. 2

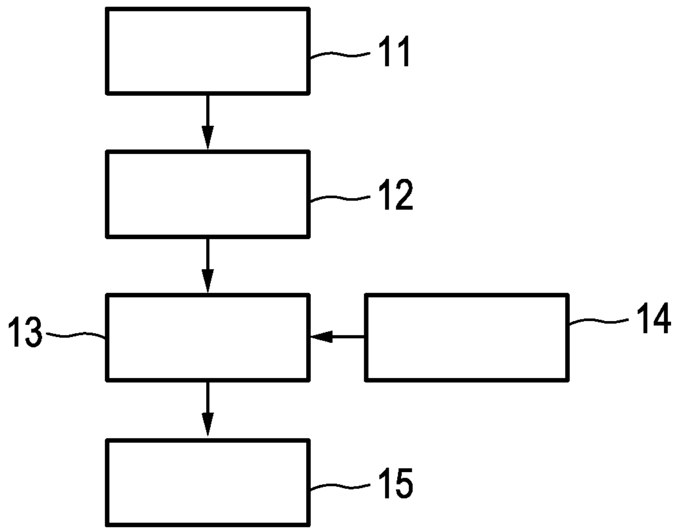


FIG. 3

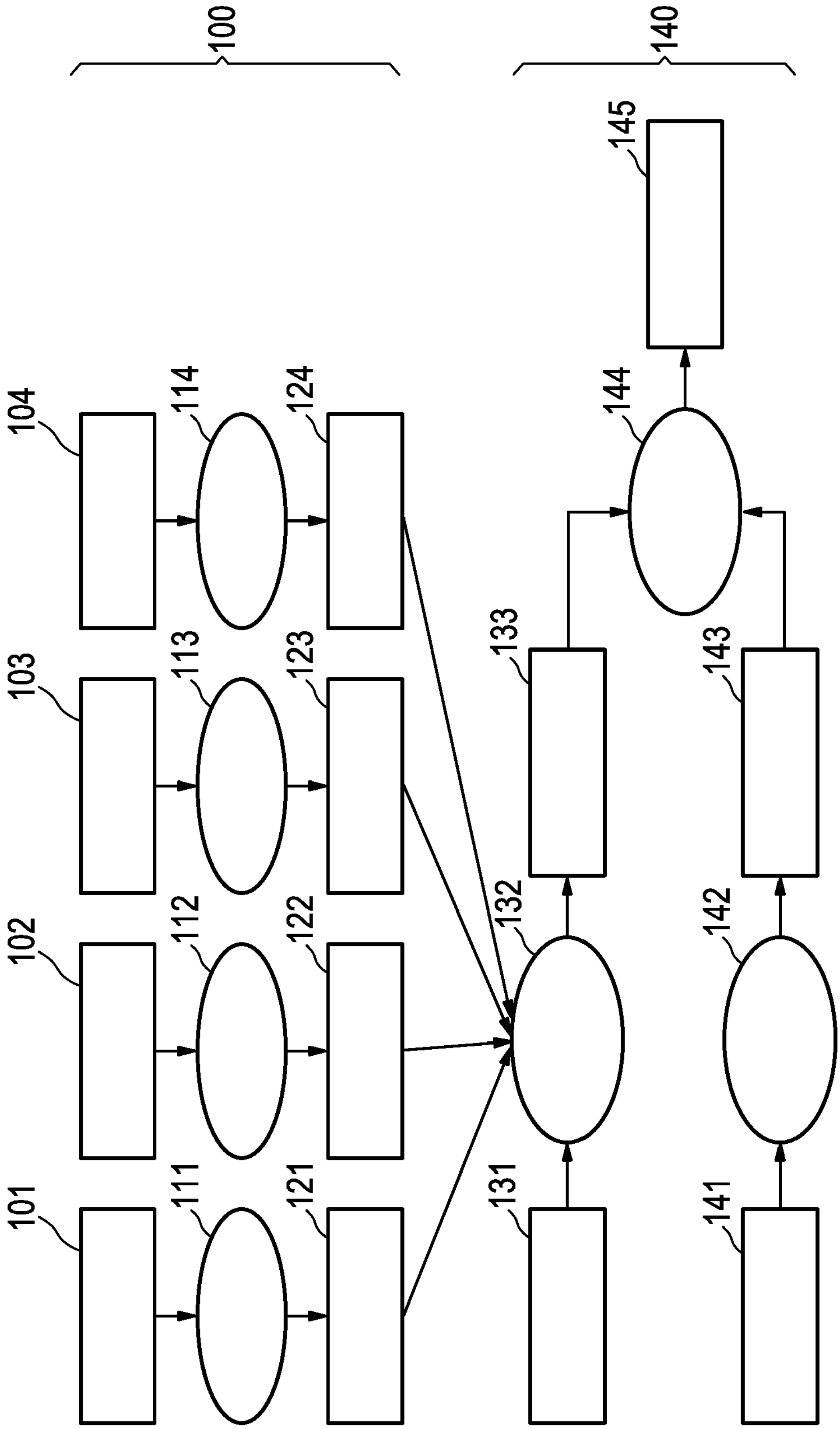
100
140
101
102
103
104
111
112
113
114
121
122
123
124
131
132
133
141
142
143
144
145

FIG. 4

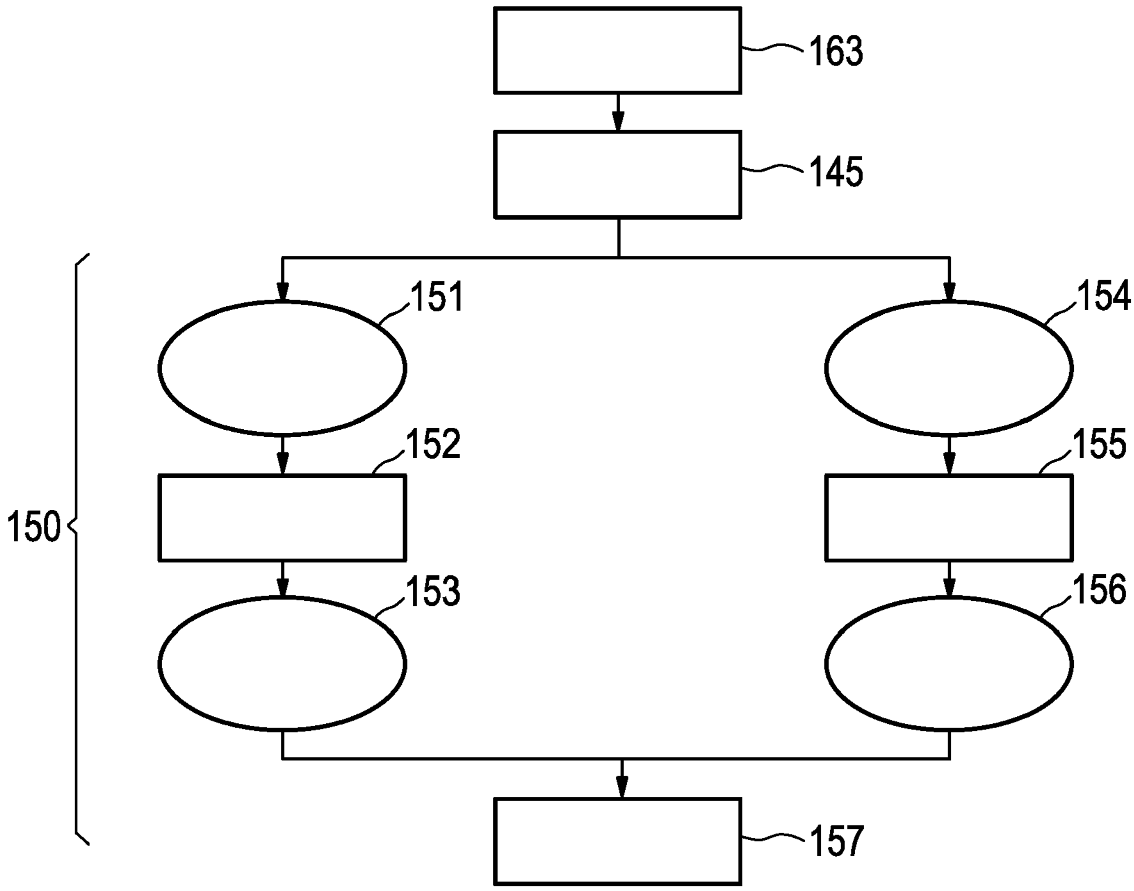


FIG. 5

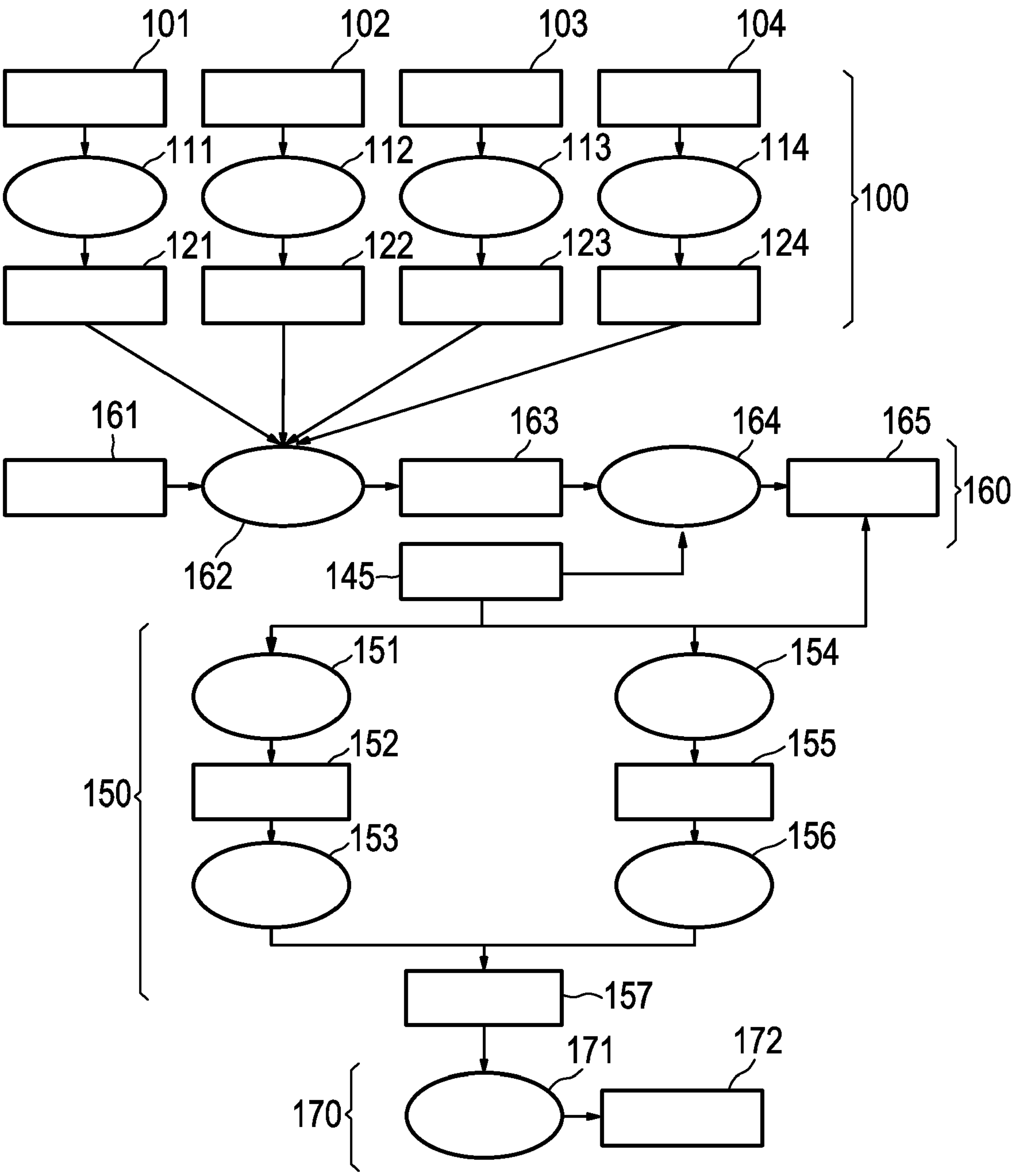
101
102
103
104
111
112
113
114
121
122
123
124
100
161
162
163
164
165
160
145
151
152
153
154
155
156
150
157
171
172
170

FIG. 6

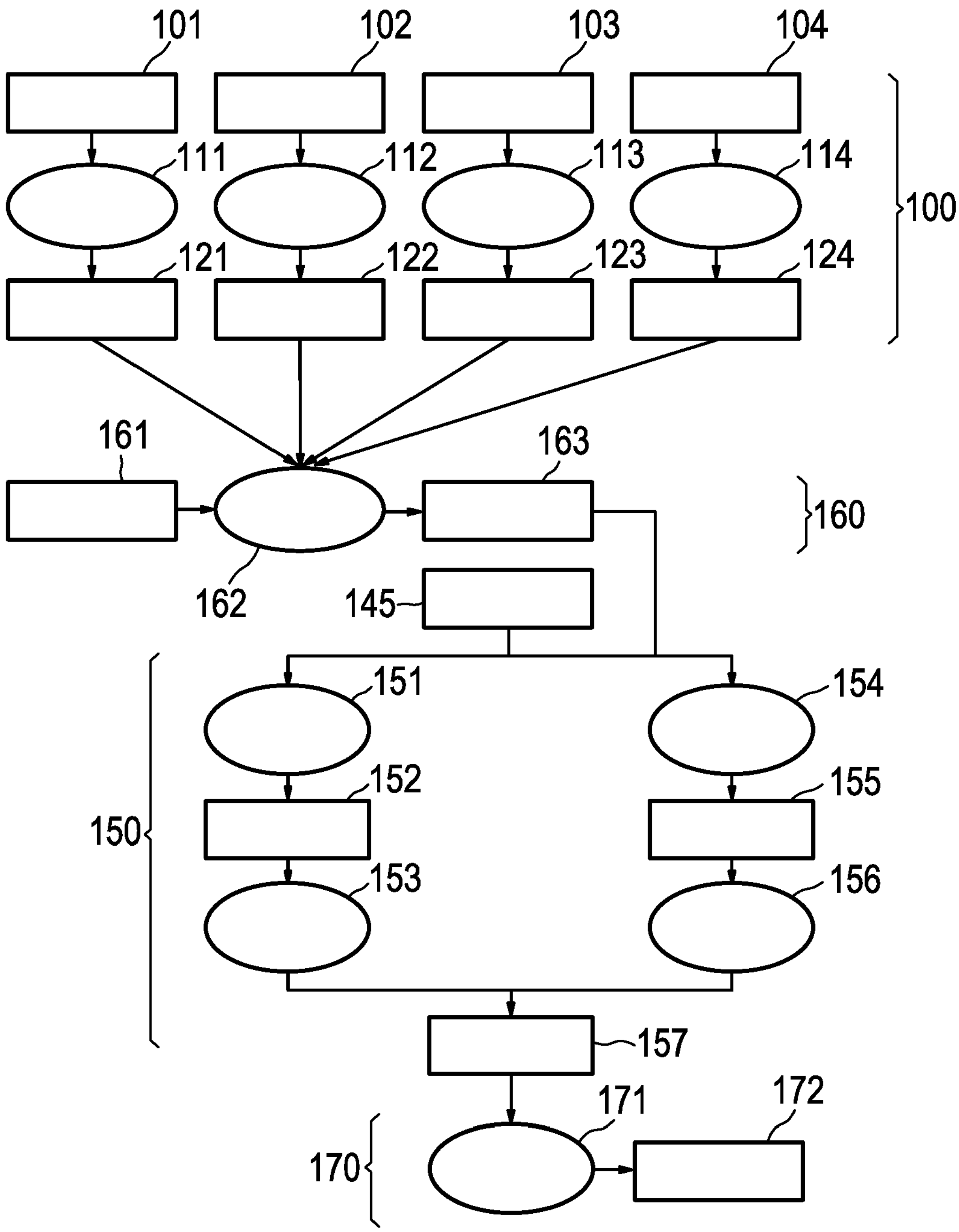
101
102
103
104
111
112
113
114
121
122
123
124
100
161
162
163
160
145
151
152
153
154
155
156
150
157
171
172
170

FIG. 7

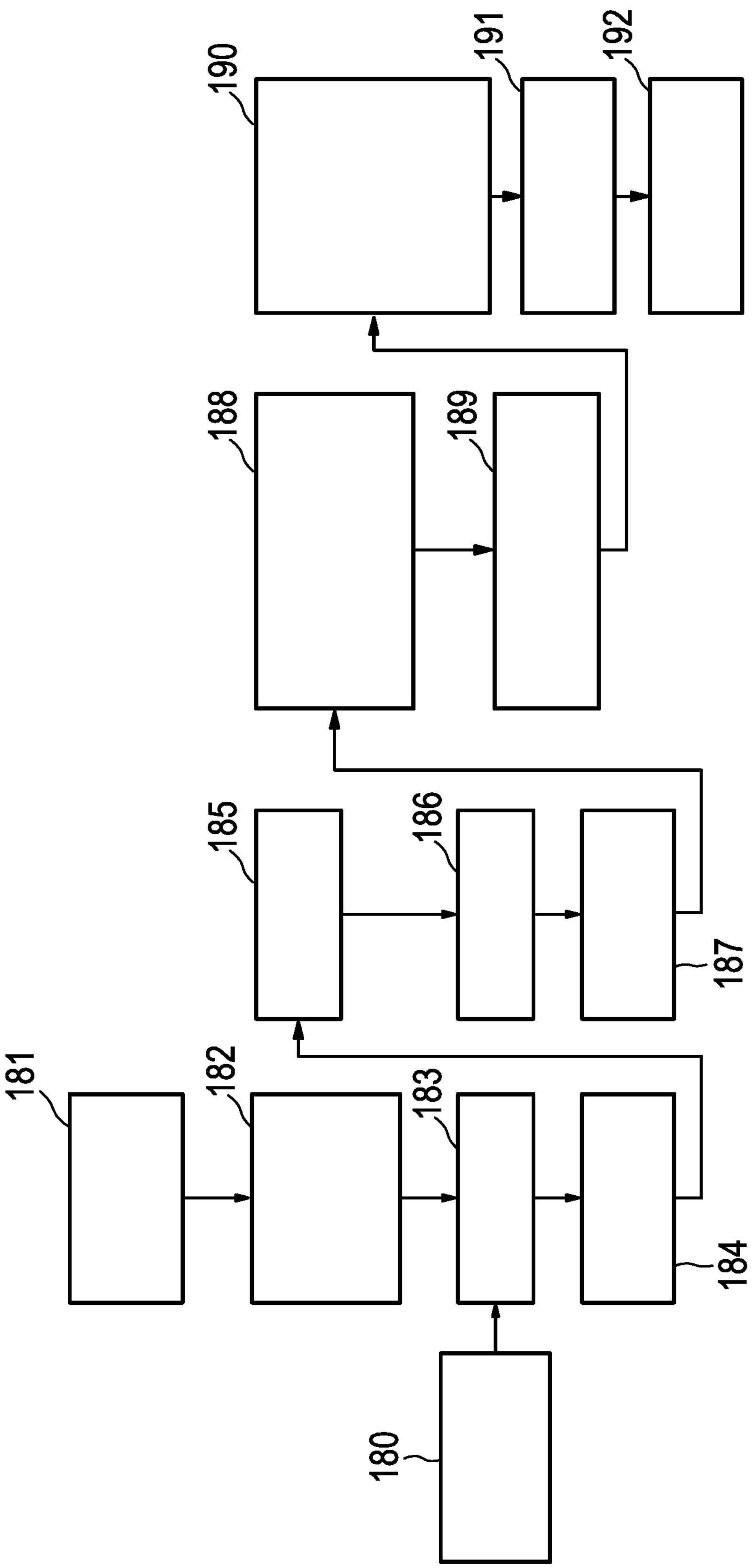


FIG. 8

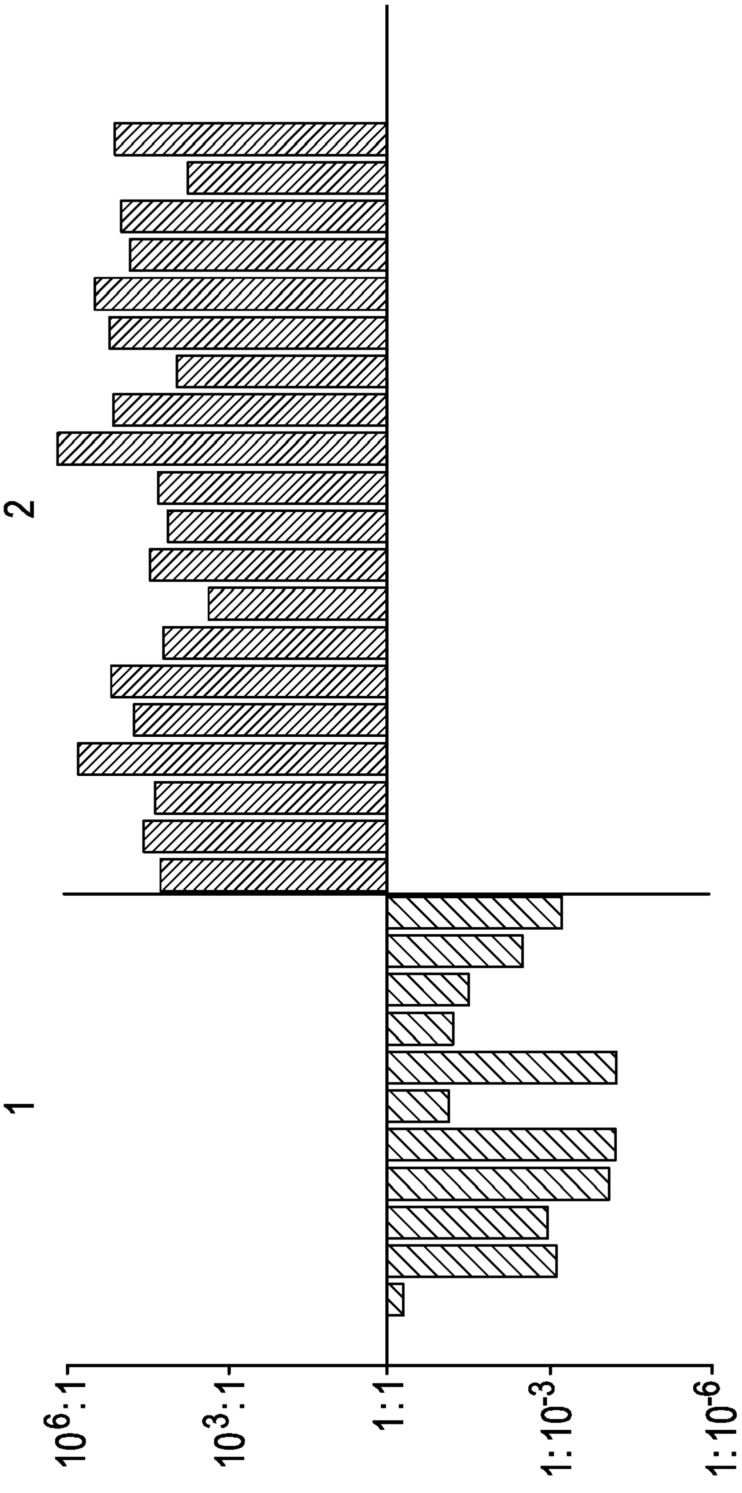


FIG. 9

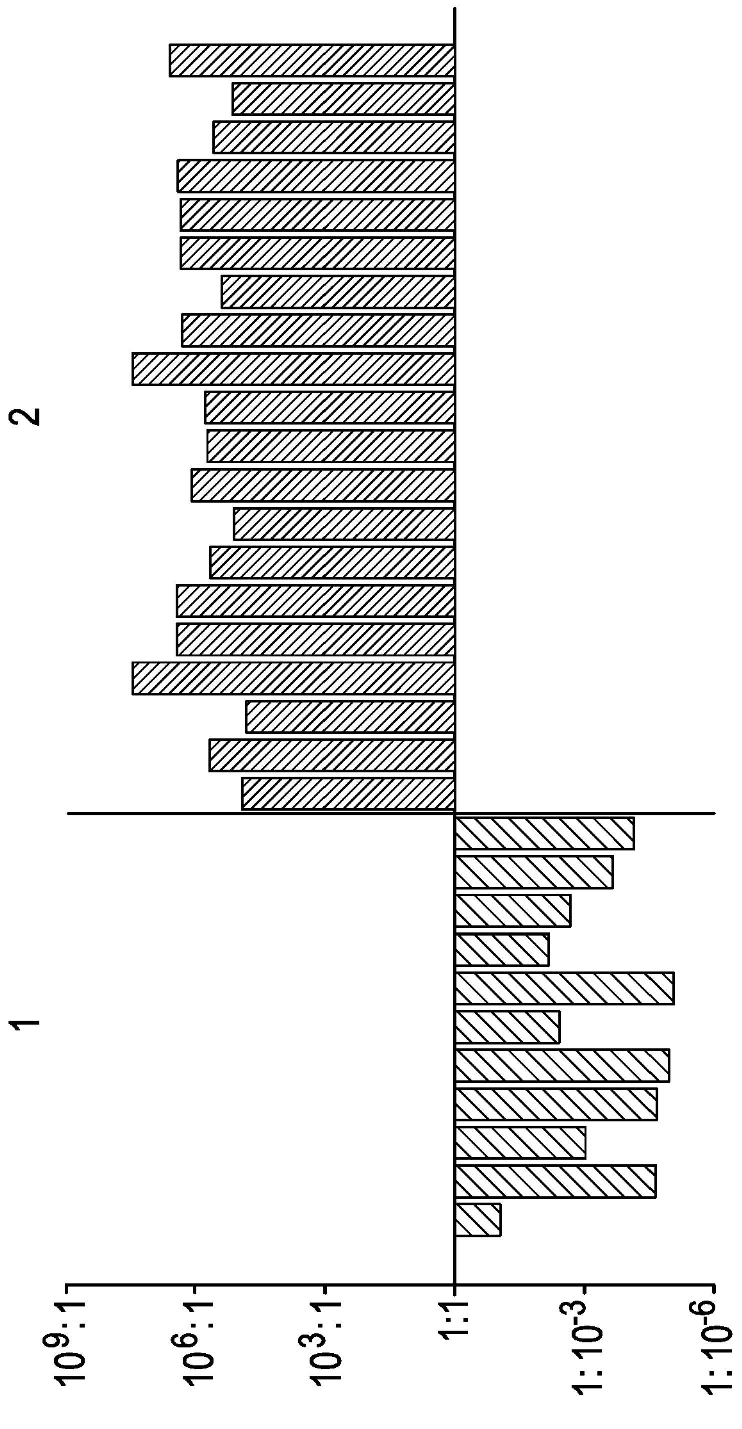


FIG. 10

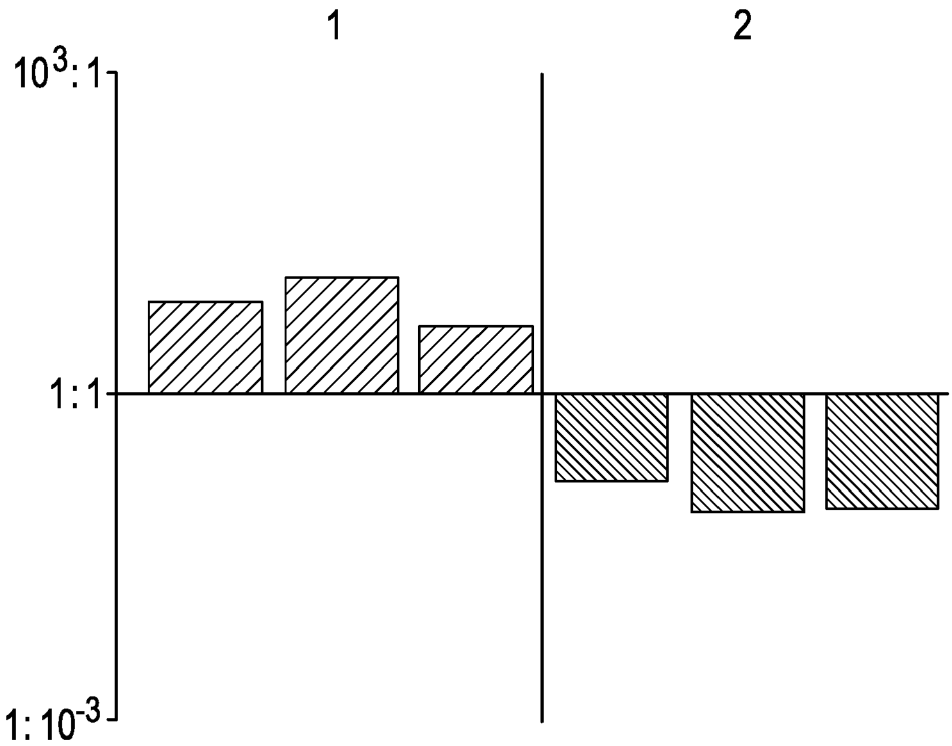


FIG. 11

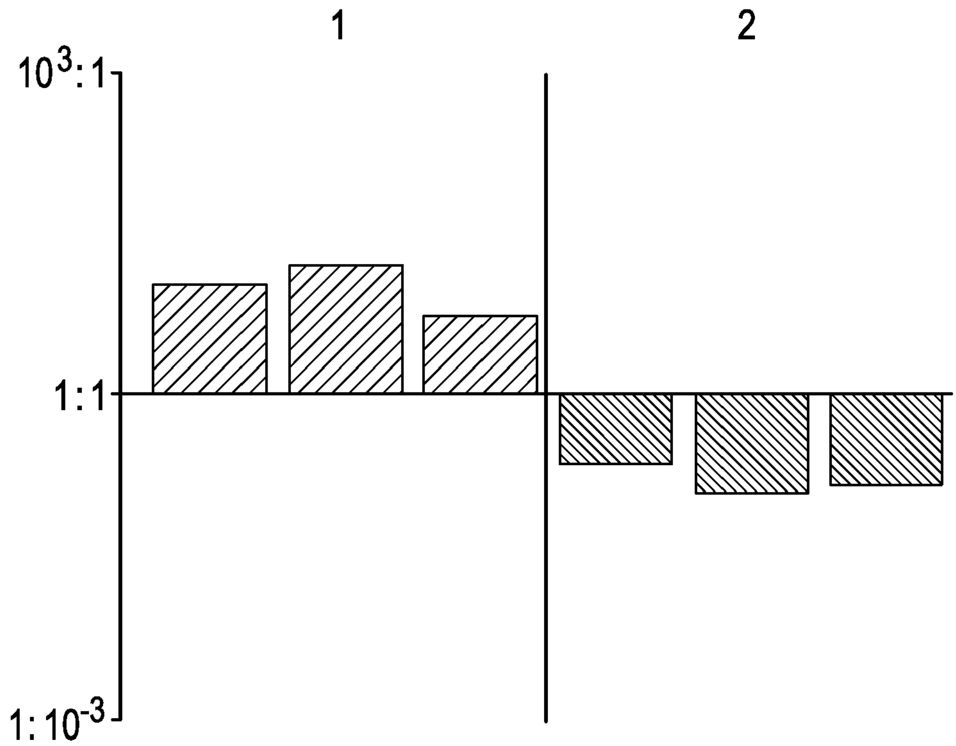


FIG. 12

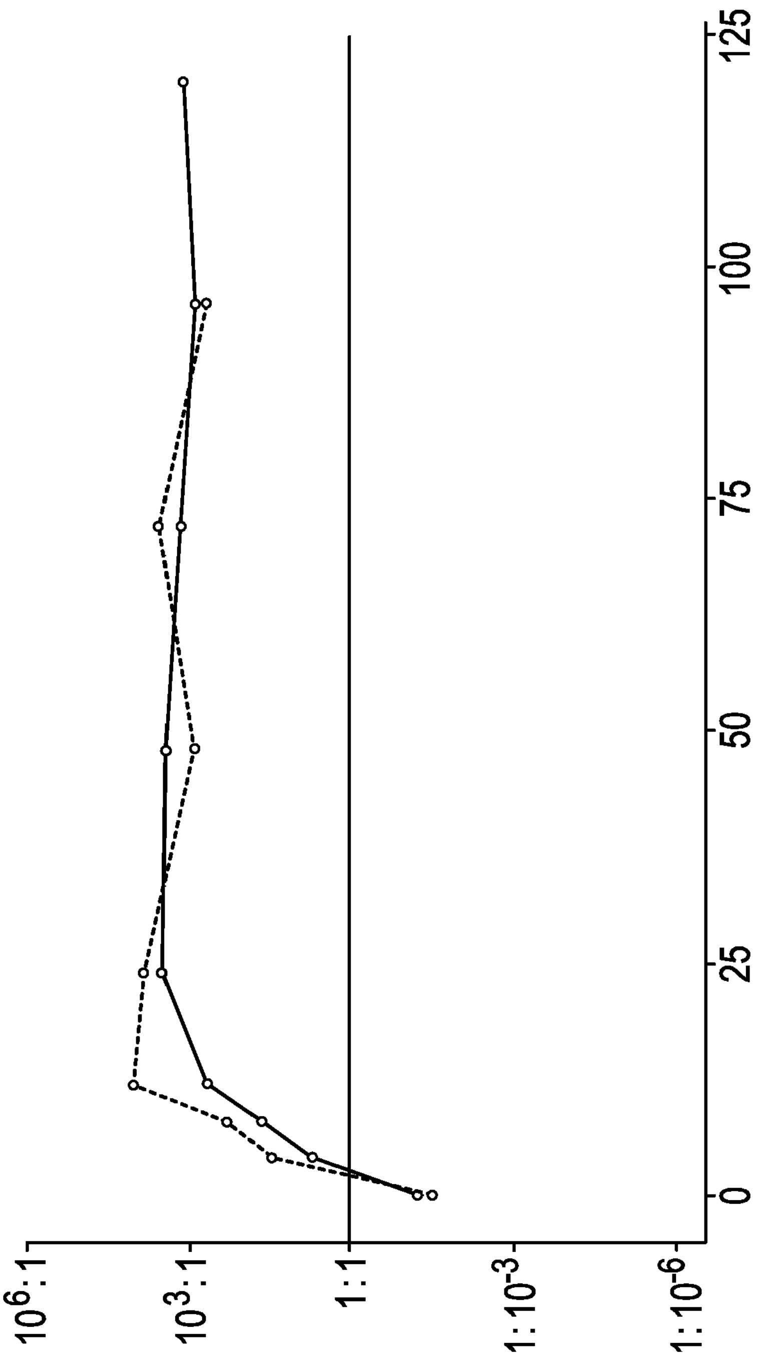


FIG. 13

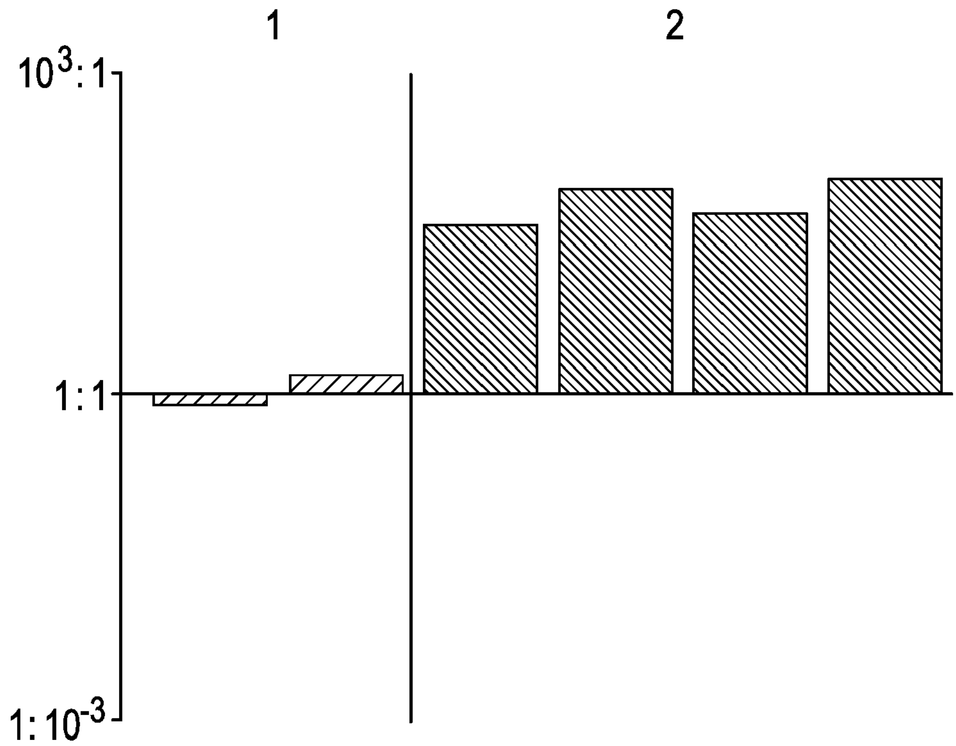


FIG. 14

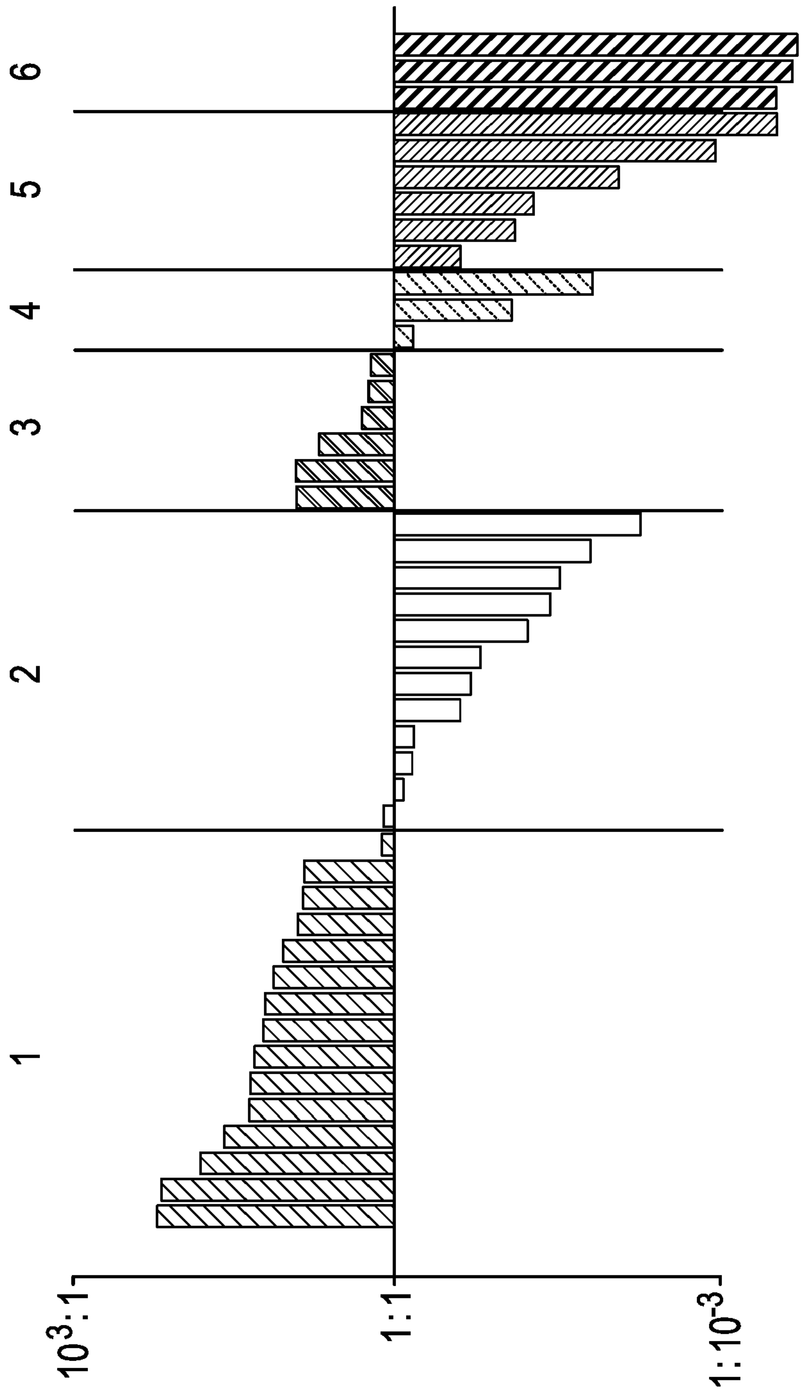


FIG. 15

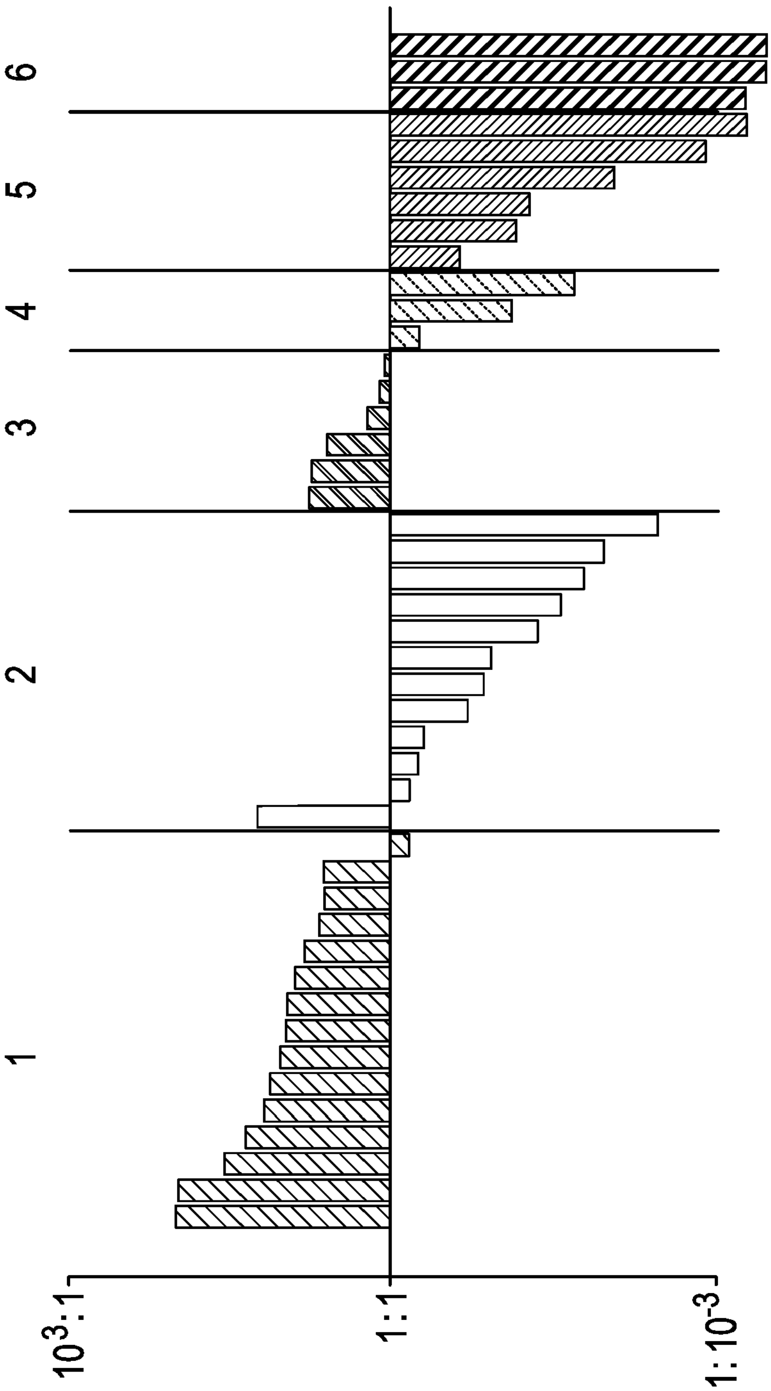


FIG. 16

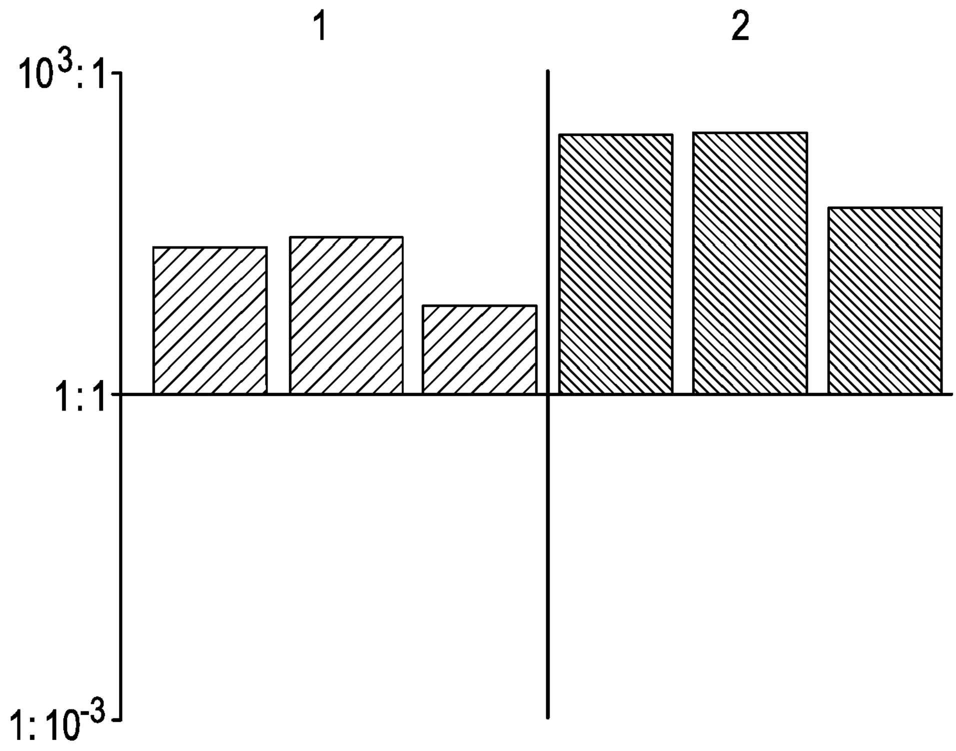


FIG. 17

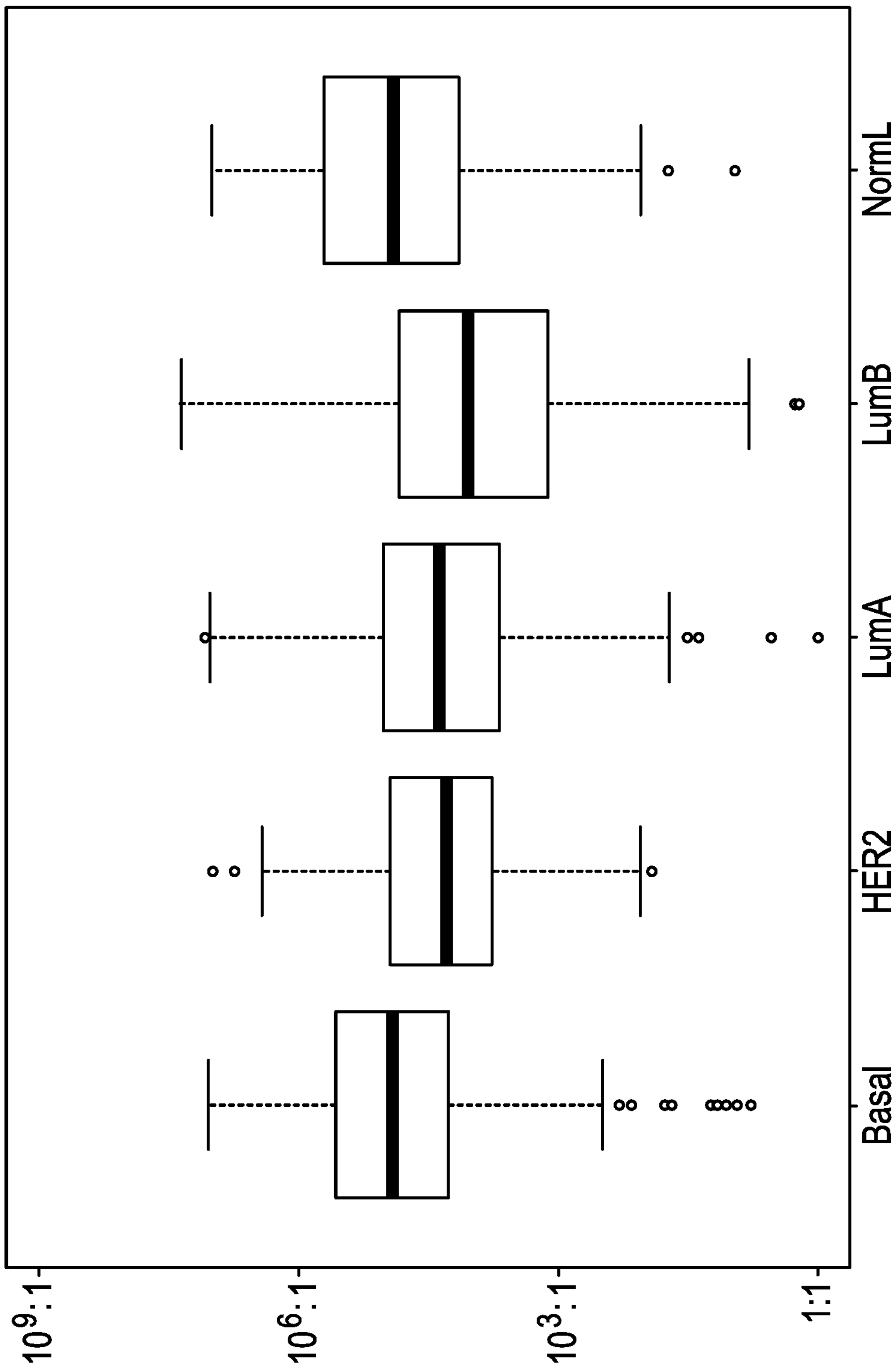


FIG. 18

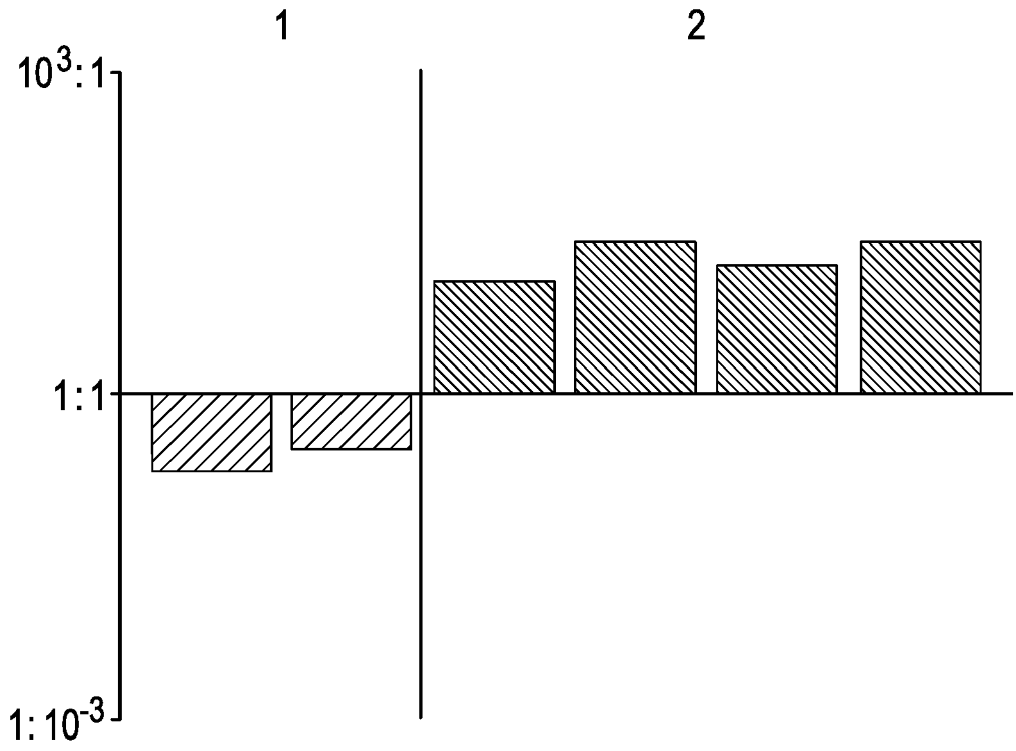


FIG. 19

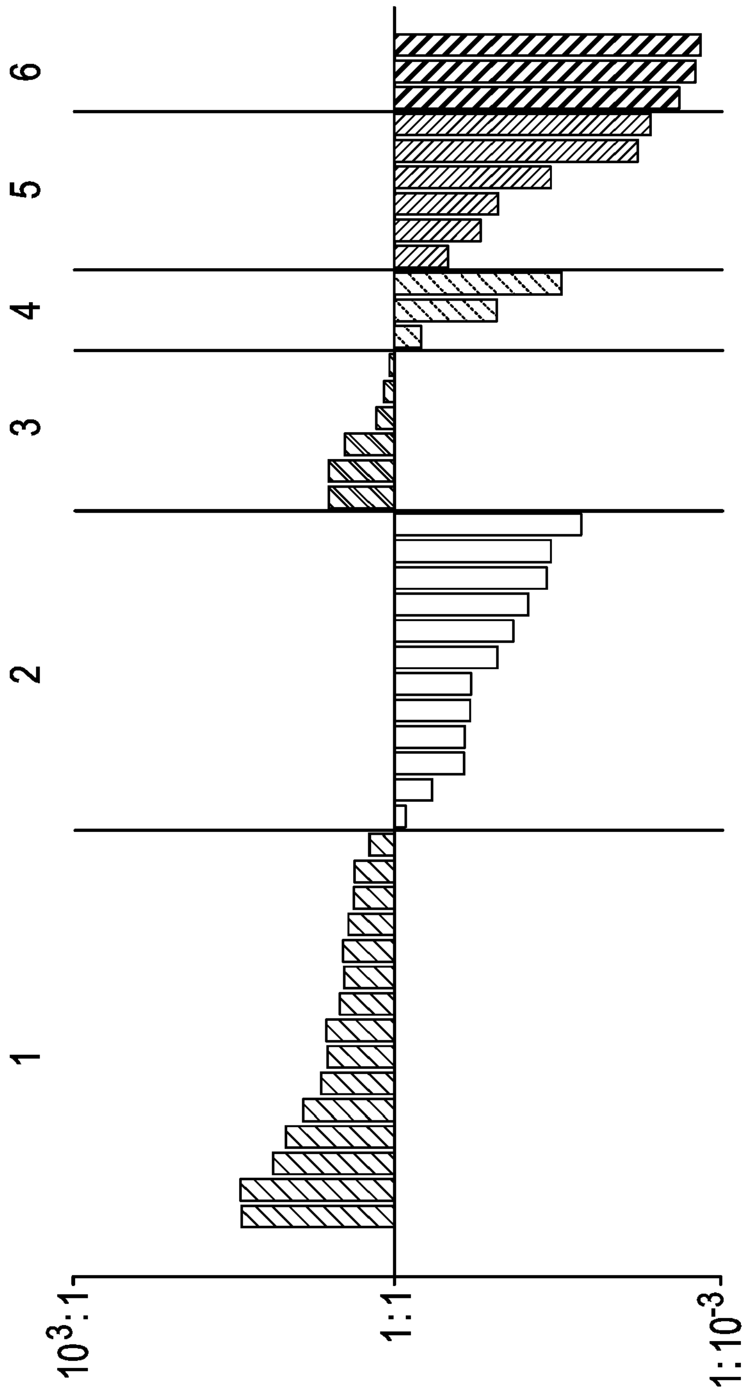


FIG. 20

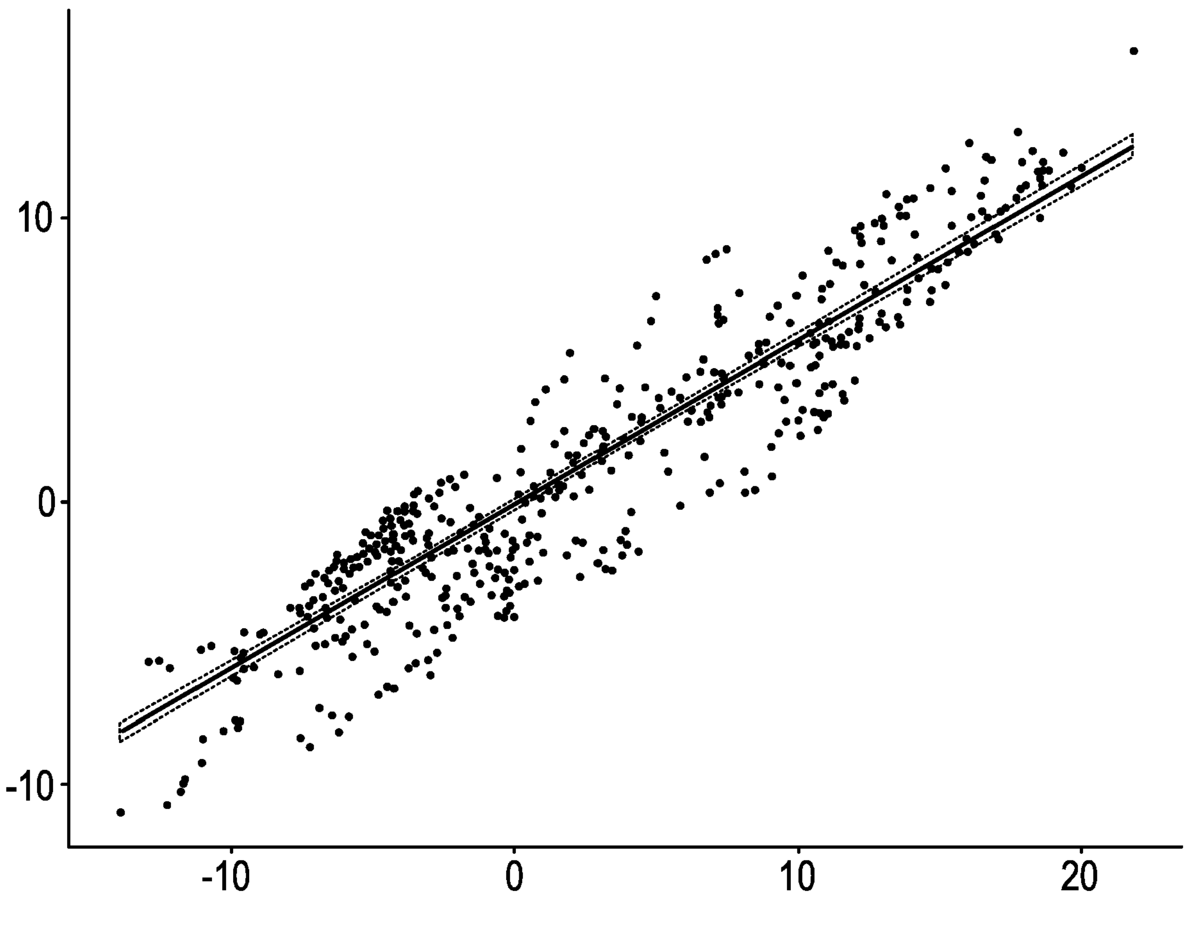


FIG. 21

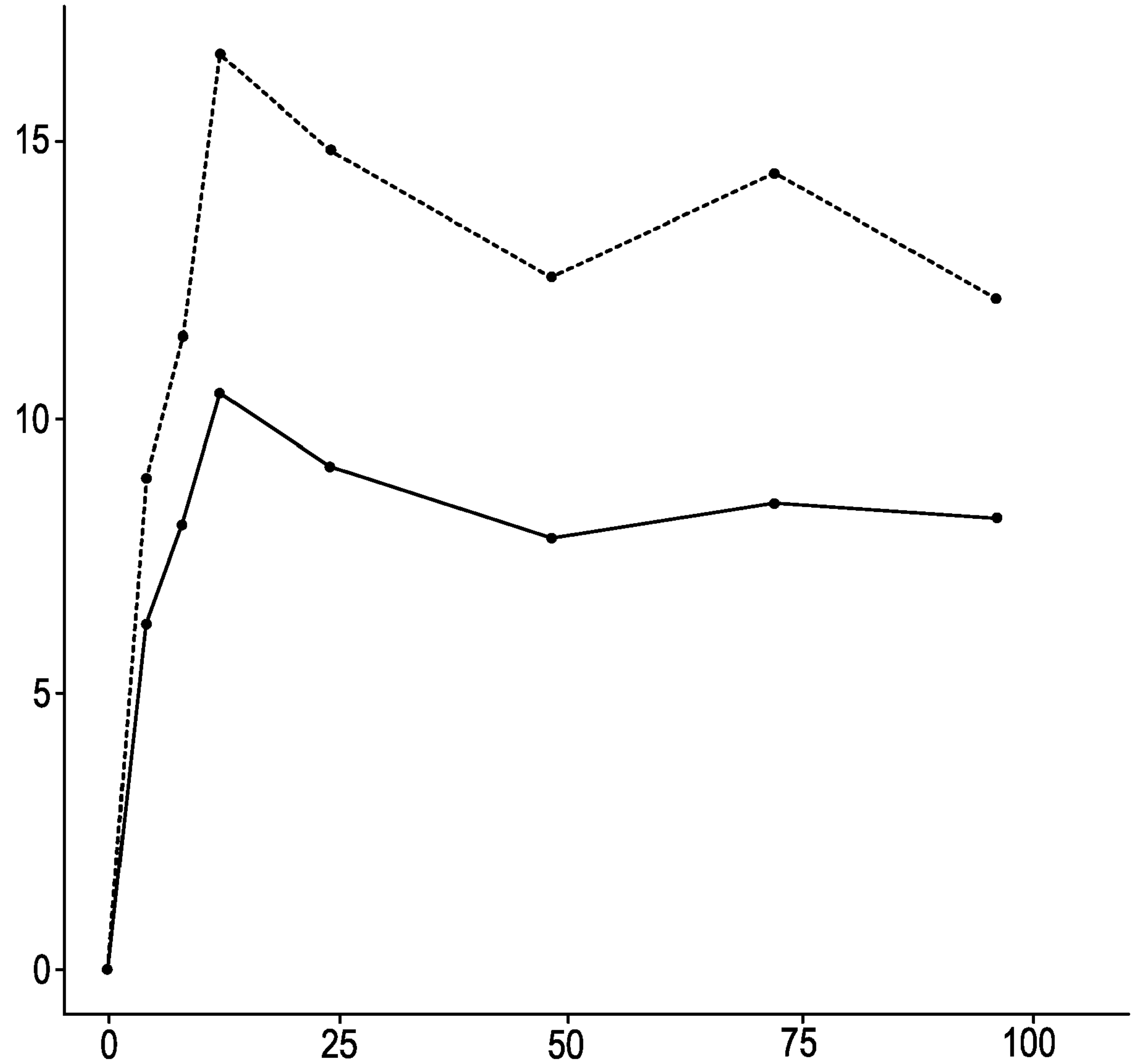


FIG. 22

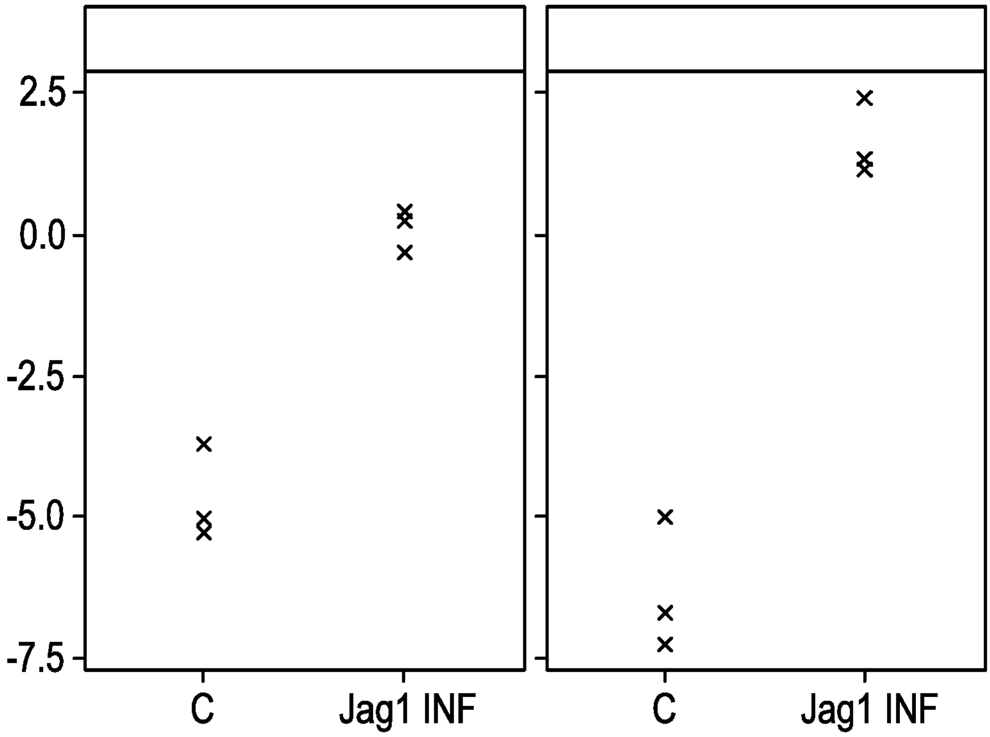


FIG. 23

Europäisches Patentamt
European Patent Office
Office européen des brevets

# EUROPEAN SEARCH REPORT

Application Number
EP 17 19 4288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | US 2016/117439 A1 (VAN OOIJEN HENDRIK JAN [NL] ET AL) 28 April 2016 (2016-04-28)<br>* the whole document *<br>* para. 68, 214, 218 *<br>* para. 17, 21, 32, 35 *<br>----- | 12,14<br><br>1-11,13,15 | INV.<br>G06F19/12<br>C12Q1/6844<br>C12Q1/6886 |
| Y,D | WO 2013/011479 A2 (KONINKL PHILIPS ELECTRONICS NV [NL]; VERHAEGH WILHELMUS FRANCISCUS JOH)<br>24 January 2013 (2013-01-24)<br>* the whole document *<br>* p. 3-4 *<br>----- | 1-15 | |
| Y | WO 2016/062891 A1 (KONINKL PHILIPS NV [NL]) 28 April 2016 (2016-04-28)<br>* the whole document *<br>* . p. 3-4 *<br>----- | 1-15 | |
| Y | S. S. RAO ET AL: "Inhibition of NOTCH Signaling by Gamma Secretase Inhibitor Engages the RB Pathway and Elicits Cell Cycle Exit in T-Cell Acute Lymphoblastic Leukemia Cells",<br>CANCER RESEARCH,<br>vol. 69, no. 7, 24 March 2009 (2009-03-24), pages 3060-3068, XP055075643,<br>ISSN: 0008-5472, DOI: 10.1158/0008-5472.CAN-08-4295<br>* the whole document *<br>* Fig. 1; p. 3063, col. 1 *<br>----- | 1-15 | TECHNICAL FIELDS SEARCHED (IPC)<br>C12Q<br>C12N<br>G06F |
| Y | US 7 544 476 B1 (O'HAGAN RONAN [US] ET AL) 9 June 2009 (2009-06-09)<br>* the whole document *<br>* col. 5, ll. 3-8 *<br>-----<br>-/-- | 1-15 | |

The present search report has been drawn up for all claims

| Place of search | Date of completion of the search | Examiner |
|---|---|---|
| Munich | 16 January 2018 | Sauer, Tincuta |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 17 19 4288

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KR 2016 0086775 A (UNIV SUNGKYUNKWAN RES & BUS [KR]) 20 July 2016 (2016-07-20)<br>* the whole document *<br>* Example 2 *<br>----- | 1-15 | |
| Y | US 2016/266095 A1 (RADTKE FREDDY [CH] ET AL) 15 September 2016 (2016-09-15)<br>* the whole document *<br>* para. 11, 12, 20 *<br>----- | 1-15 | |

TECHNICAL FIELDS SEARCHED (IPC)

The present search report has been drawn up for all claims

2

| Place of search | Date of completion of the search | Examiner |
|---|---|---|
| Munich | 16 January 2018 | Sauer, Tincuta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 17 19 4288

This annex lists the patent family members relating to the patent documents cited in the above-mentioned European search report. The members are as contained in the European Patent Office EDP file on
The European Patent Office is in no way liable for these particulars which are merely given for the purpose of information.

16-01-2018

| Patent document cited in search report | Publication date | Patent family member(s) | Publication date |
|---|---|---|---|
| US 2016117439 A1 | 28-04-2016 | AU 2015334842 A1 | 15-06-2017 |
| | | CA 2965408 A1 | 28-04-2016 |
| | | EP 3210144 A1 | 30-08-2017 |
| | | US 2016117439 A1 | 28-04-2016 |
| | | WO 2016062893 A1 | 28-04-2016 |
| WO 2013011479 A2 | 24-01-2013 | BR 112014000965 A2 | 13-06-2017 |
| | | CN 103649337 A | 19-03-2014 |
| | | DK 2734643 T3 | 20-03-2017 |
| | | EP 2549399 A1 | 23-01-2013 |
| | | EP 2734643 A2 | 28-05-2014 |
| | | EP 3173489 A1 | 31-05-2017 |
| | | ES 2617195 T3 | 15-06-2017 |
| | | JP 6204354 B2 | 27-09-2017 |
| | | JP 2014520566 A | 25-08-2014 |
| | | JP 2017205129 A | 24-11-2017 |
| | | KR 20140046464 A | 18-04-2014 |
| | | RU 2014106026 A | 27-08-2015 |
| | | US 2014156200 A1 | 05-06-2014 |
| | | WO 2013011479 A2 | 24-01-2013 |
| WO 2016062891 A1 | 28-04-2016 | AU 2015334840 A1 | 15-06-2017 |
| | | CA 2965442 A1 | 28-04-2016 |
| | | CN 107077536 A | 18-08-2017 |
| | | EP 3210142 A1 | 30-08-2017 |
| | | US 2016113572 A1 | 28-04-2016 |
| | | WO 2016062891 A1 | 28-04-2016 |
| US 7544476 B1 | 09-06-2009 | AU 2009269081 A1 | 14-01-2010 |
| | | CA 2730215 A1 | 14-01-2010 |
| | | EP 2313526 A1 | 27-04-2011 |
| | | JP 2011527575 A | 04-11-2011 |
| | | US 7544476 B1 | 09-06-2009 |
| | | WO 2010005644 A1 | 14-01-2010 |
| KR 20160086775 A | 20-07-2016 | NONE | |
| US 2016266095 A1 | 15-09-2016 | AU 2012356033 A1 | 07-08-2014 |
| | | CA 2859740 A1 | 27-06-2013 |
| | | CN 104136027 A | 05-11-2014 |
| | | CN 107434779 A | 05-12-2017 |
| | | EP 2606884 A1 | 26-06-2013 |
| | | EP 2793884 A1 | 29-10-2014 |
| | | HK 1203396 A1 | 30-10-2015 |
| | | JP 2015506345 A | 02-03-2015 |
| | | NZ 627559 A | 24-06-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 17 19 4288

This annex lists the patent family members relating to the patent documents cited in the above-mentioned European search report. The members are as contained in the European Patent Office EDP file on
The European Patent Office is in no way liable for these particulars which are merely given for the purpose of information.

16-01-2018

| Patent document cited in search report | Publication date | Patent family member(s) | Publication date |
|---|---|---|---|
| | | RU 2014127753 A | 10-02-2016 |
| | | SG 10201605984R A | 29-09-2016 |
| | | SG 11201403418V A | 30-07-2014 |
| | | US 2014329867 A1 | 06-11-2014 |
| | | US 2016266095 A1 | 15-09-2016 |
| | | WO 2013093885 A1 | 27-06-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013011479 A2
- WO 2014102668 A2
- US 6713297 B
- US 5476928 A
- US 5958691 A
- US 5436134 A
- US 5658751 A


### Non-patent literature cited in the description


- **ASTER J.C. et al.** The varied roles of Notch in cancer. *Annual Review of Pathology,* December 2016, vol. 12 (1), 245-275
- **GURUHARSHA K.G. et al.** The Notch signaling system: recent insights into the complexity of a conserved pathway. *Nature Reviews Genetics,* September 2012, vol. 13, 654-666
- **ESPINOZA I. ; MIELE L.** Notch inhibitors for cancer treatment. *Pharmacology & Therapeutics,* August 2013, vol. 139 (2), 95-110
- **VERHAEGH W. et al.** Selection of personalized patient therapy through the use of knowledge-based computational models that identify tumor-driving signal transduction pathways. *Cancer Research,* 2014, vol. 74 (11), 2936-2945
- **GURUHARSHA K.G. et al.** The Notch signaling system: recent insights into the complexity of a conserved pathway. *Nature Reviews Genetics,* 13 September 2012, 654-666
- **MEIER-STIEGEN F. et al.** Activated Notch1 target genes during embryonic cell differentiation depend on the cellular context and include lineage determinants and inhibitors. *PLoS One,* July 2010, vol. 5 (7
- **DOHDA T. et al.** Notch signaling induces SKP2 expression and promotes reduction of p27Kip1 in T-cell acute lymphoblastic leukemia cell. *Experimental Cell Research,* August 2007, vol. 313 (14), 3141-3152
- **VAN NES J. et al.** A NOTCH3 Transcriptional Module Induces Cell Motility in Neuroblastoma. *Clinical Cancer Research,* July 2013, vol. 19 (13), 3485-3494
- **WANG H. et al.** Genome-wide analysis reveals conserved and divergent features of Notch1/RBPJ binding in human and murine T-lymphoblastic leukemia cells. *Proceedings of the National Academy of Sciences of the USA,* 2011, vol. 108 (36), 14908-14913
- **YOU L.R. et al.** *Suppression of Notch signaling by the COUP-TFII transcription factor regulates vein identity,* May 2005, vol. 435 (7038), 98-104
- **CHEN X. et al.** COUP-TFII is a major regulator of cell cycle and Notch signaling pathways. *Molecular Endocrinology,* August 2012, vol. 26 (8), 1268-1277
- **ZHONG Y. et al.** NOTCH1 is a Poor Prognostic Factor for Breast Cancer and Is Associated With Breast Cancer Stem Cells. *Oncotargets and Therapy,* November 2016, vol. 9, 6865-6871
- **MIKHAILIK A. et al.** Notch ligand-dependent gene expression in human endometrial stromal cells. *Biochemical and Biophysical Research Communications,* October 2009, vol. 388 (3), 479-482